# EUROPÄISCHE PATENTSCHRIFT

(11) **EP 2 254 871 B1**
(45) Veröffentlichungstag und Bekanntmachung des Hinweises auf die Patenterteilung: **09.11.2016**
(21) Anmeldenummer: 09705287.2
(22) Anmeldetag: 30.01.2009
(51) Int. Cl.: C07D 233/58, C07D 233/60, C07D 249/08, B01D 15/38

(54) **SALZE MIT ARYL-ALKYL-SUBSTITUIERTEN IMIDAZOLIUM-UND TRIAZOLIUMKATIONEN ALS IONISCHE FLÜSSIGKEITEN**
SALTS COMPRISING ARYL-ALKYL-SUBSTITUTED IMIDAZOLIUM AND TRIAZOLIUM CATIONS AS IONIC FLUIDS
SELS CONTENANT DES CATIONS D'IMIDAZOLIUM ET DE TRIAZOLIUM À SUBSTITUTION ARYLE-ALKYLE SERVANT DE LIQUIDES IONIQUES

(30) Priorität: 30.01.2008 DE 102008007675
(43) Veröffentlichungstag der Anmeldung: 01.12.2010
(73) Patentinhaber: Technische Universität Dresden, 01069 Dresden (DE)
(72) Erfinder: STRASSNER, Thomas, 01156 Dresden (DE); AHRENS, Sebastian, 67059 Ludwigshafen (DE)
(74) Vertreter: Kailuweit & Uhlemann Patentanwälte Partnerschaft mbB
(86) Internationale Anmeldenummer: PCT/DE2009/000149
(87) Internationale Veröffentlichungsnummer: WO 2009/095012

(56) Entgegenhaltungen:
- EP-A- 1 201 657
- EP-A- 1 512 460
- WO-A-03/074494
- WO-A-2004/054991
- WO-A-2004/096776
- WO-A-2008/095069
- DE-A1-102004 060 247
- DATABASE WPI Week 200872 Thomson Scientific, London, GB; AN M15522 XP002529232 -& CN 101 182 308 A (UNIV. ZHEJIANG POLYTECHNIC) 21. Mai 2008 (2008-05-21)
- DATABASE WPI Week 200902 Thomson Scientific, London, GB; AN A35933 XP002529233 -& CN 101 215 262 A (CHINA TEXTILE ACAD) 9. Juli 2008 (2008-07-09)
- DATABASE CAPLUS [Online] CHEMICAL ABSTRACTS SERVICE, COLUMBUS, OHIO, US; 2. Oktober 2007 (2007-10-02), XP002529231 gefunden im STN Database accession no. 2007:1103827 & X. LIU ET AL.: "Synthesis of 1-(aryl)imidazoles and (aryl)imidazolium salts and their activity as agrochemical fungicides" YUNNAN MINZU DAXUE XUEBAO, ZIRAN KEXUEBAN, Bd. 16, Nr. 3, 2007, Seiten 228-232,
- R. E. BALTUS ET AL.: "Low-Pressure Solubility of Carbon Dioxide in Room-Temperature Ionic Liquids Measured with a Quartz Crystal Microbalance" J. PHYS. CHEM. B, Bd. 108, Nr. 2, 12. September 2003 (2003-09-12), Seiten 721-727, XP002529221
- X. HU ET AL.: "Dioxygen Activation by a Low-Valent Cobalt Complex Employing a Flexible Tripodal N-Heterocyclic Carbene Ligand" J. AM. CHEM. SOC., Bd. 126, Nr. 41, 28. September 2004 (2004-09-28), Seiten 13464-13473, XP002529222
- S. PRÜHS ET AL.: "Preparation, Reactivity, and Structural Peculiarities of Hydroxyalkyl-Functionalized "Second-Generation" Ruthenium Carbene Complexes" ORGANOMETALLICS, Bd. 23, Nr. 2, 16. Dezember 2003 (2003-12-16), Seiten 280-287, XP002529223
- A. T. NORMAND ET AL.: "Catalytic Annulation of Heterocycles via a Novel Redox Process Involving the Imidazolium Salt N-Heterocyclic Carbene Couple" ORGANOMETALLICS, Bd. 27, Nr. 3, 3. Juni 2008 (2008-06-03), Seiten 3153-3160, XP002529224
- P. H. J. KOUWER, T. M. SWAGER: "Synthesis and Mesomorphic Properties of Rigid-Core Ionic Liquid Crystals" J. AM. CHEM. SOC., Bd. 129, Nr. 45, 20. Oktober 2007 (2007-10-20), Seiten 14042-14052, XP002529225
- H. V. HUYNH, J. WU: "Rotamers of palladium complexes bearing IR active N-heterocyclic carbene ligands: Synthesis, structural characterization and catalytic activities" J. ORGANOMET. CHEM., Bd. 694, 5. November 2008 (2008-11-05), Seiten 323-331, XP002529226
- A. A. DANOPOULOS ET AL.: "Copper and palladium complexes with N-heterocyclic carbene ligands functionalised with carboxylate groups" J. ORGANOMET. CHEM., Bd. 693, 22. Juli 2008 (2008-07-22), Seiten 3369-3374, XP002529227
- W.-C. SHIH ET AL.: "Synthesis and Structure of an Amino-Linked N-Heterocyclic Carbene and the Reactivity of its Aluminum Adduct" ORGANOMETALLICS, Bd. 28, Nr. 4, 20. Januar 2009 (2009-01-20), Seiten 1060-1067, XP002529228
- J. WOLF ET AL.: "Nickel(II), Palladium(II) and Rhodium(I) Complexes of New NHC-Thioether Ligands: Efficient Ketone Hydrosilylation Catalysis by a Cationic Rh Complex" EUR. J. INORG. CHEM., 2007, Seiten 5069-5079, XP002529229
- R. CARIOU ET AL.: "A Bidentate NHC-Alkenyl Ruthenium(II) Complex via Vinyl C-H Bond Activation" ORGANOMETALLICS, Bd. 25, Nr. 9, 25. März 2006 (2006-03-25), Seiten 2126-2128, XP002529230

## Beschreibung

Die vorliegende Erfindung betrifft Salze aus neuen Aryl-Alkyl-substituierten Imidazolium- und Triazoliumkationen und beliebigen Anionen. Die Erfindung betrifft weiterhin Verfahren zur chemischen Umwandlung und Trennung von Stoffen umfassend die erfindungsgemäßen Salze als Lösungsmittel, Lösungsmittelzusätze oder Extraktionsmittel sowie die Verwendung der erfindungsgemäßen Salze, beispielweise als Lösungsmittel oder Lösungsmittelzusatz in chemischen Reaktionen, als Extraktionsmittel zur Trennung von Stoffen oder zur Speicherung von Wasserstoff.

Organische Lösungsmittel finden heutzutage Anwendungen in vielen unterschiedlichen Bereichen, angefangen in der Chemie, aber auch in der Biologie und in der Materialentwicklung. In traditionellen katalytischen Prozessen bringen organische Lösungsmittel jedoch aufgrund ihrer Flüchtigkeit, Entflammbarkeit und Toxizität oft das Problem mit sich, dass Gefahren für die Sicherheit, Gesundheit und Umwelt mit aufwendigen technischen Maßnahmen verhindert werden müssen (vgl. Wasserscheid/Welton, "Ionic Liquids in Synthesis", Wiley-VCH 2007).

Neue umweltfreundliche Synthesemethoden, die ohne Lösungsmittel auskommen oder umweltfreundliche alternative Lösungsmittel verwenden, gewinnen daher mehr und mehr an Bedeutung. In diesem Kontext richtet sich die Aufmerksamkeit in den letzten Jahren besonders auf ionische Flüssigkeiten.

Die Definition von ionischen Flüssigkeiten ("ionic liquids", "ILs") bezeichnet Stoffe, die vollständig aus organischen Kationen und organischen oder anorganischen Anionen bestehen und zusätzlich einen niedrigen Schmelzpunkt von unter 100 °C und eine verhältnismäßig niedrige Viskosität besitzen.

Ionische Flüssigkeiten zeigen weitere vorteilhafte Eigenschaften, wie z. B. niedrige Dampfdrücke, geringe Brennbarkeit sowie eine einstellbare Polarität und Mischbarkeit mit anderen organischen und anorganischen Substanzen. Sie stellen daher in der Synthese und homogenen Katalyse eine interessante Alternative zu den heute verwendeten organischen Lösungsmitteln dar. Der niedrige Dampfdruck, die damit verbundene geringe Entflammbarkeit, die geringe Toxizität und Variabilität dieser Verbindungen macht sie zu idealen "grünen" Lösungsmitteln für eine Vielzahl chemischer Prozesse.

Bekannte ionische Flüssigkeiten enthalten als Kationen z.B. Alkylammonium-, Alkylphosphonium-, N,N'-Dialkylimidazolium- und N-Alkylpyridinium-Kationen:

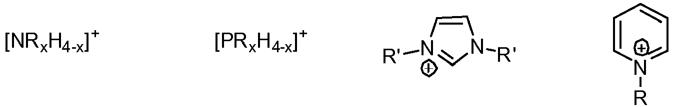

Bisher gibt es jedoch keinen zuverlässigen Weg, die Schmelzpunkte organischer Salze vorherzusagen. Das Auffinden von neuen organischen Salzen, die die Definition von ionischen Flüssigkeiten erfüllen, ist derzeit nur experimentell möglich.

Die elektronischen Eigenschaften der im Handel befindlichen und üblicherweise genutzten ionischen Flüssigkeiten können nur in geringem Maße verändert werden.

Aus dem Stand der Technik (z.B. WO 03 / 074494 A1, DE 10 2004 060 247 A1 oder EP 1 201 657 A1, WO 04/096776 A1, CN 101182308A, CN 1012562 A, WO 08/095069 A) sind N,N'-substituierte Imidazolium- und Triazoliumsalze bekannt, die als ionische Flüssigkeiten zum Einsatz kommen. Die dort offenbarten einzelnen Verbindungen sind aliphatisch substituierte Imidazolium- und Triazoliumsalze, bei denen die sp³-hybridisierten Substituenten an den Stickstoffen die Möglichkeiten einer Beeinflussung des Systems auf die induktiven Effekte reduzieren. Mesomere Effekte können nicht genutzt werden.

Baltus RE et al. (J. Phys. Chem. B. 2004, 108, 721-727) beschreiben die Löslichkeit von CO₂ in ionischen Flüssigkeiten. Neben aliphatisch N,N'-substituierten Imidazoliumsalzen werden hier auch 1,4-Dibutyl-3-phenylimidazolium und 1-Butyl-3-phenylimidazolium beschrieben.

Gegenwärtig konzentrieren sich die Forschungsaktivitäten weitgehend auf die Synthese neuer Anionen. So sind aus WO 03/074494 A1 ionische Flüssigkeiten mit halogenfreien Anionen bekannt, aus WO 2007/131498 A2 hydrophobe Anionen, aus WO 2004/054991 A1 solche mit [N(CF₃)₂⁻]-Anionen, aus EP 1 512 460 A1 mit Thiocyanat-Anionen und aus WO 2004/096776 A1 mit Alkylsulfat-Anionen.

Aufgabe der Erfindung ist es daher, Salze mit neuen organischen Kationen bereitzustellen, deren elektronische Eigenschaften gezielt variiert werden können und deren Schmelzpunkte und Lösungseigenschaften einstellbar sind, so dass sie zur Verwendung als ionische Flüssigkeiten geeignet sind.

Erfindungsgemäß wird die Aufgabe gelöst durch Salze der allgemeinen Formel **(I),** 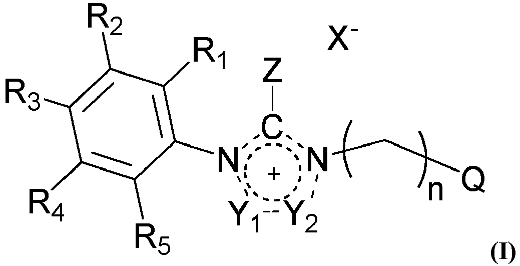 wobei
X⁻ **ein** wie in Anspruch 1 definiertes Anion ist,
Y₁ und Y₂ CH sind oder Y₁ CH und Y₂ N ist oder Y₁ N und Y₂ CH ist,
n eine Zahl von 2 bis einschließlich 14 ist,
Q ausgewählt ist aus -CH₃, -OH, -ORₓ, -SO₃H, -SO₃Rₓ, -COOH, -COORₓ, -CORₓ, NH₂,-NHRₓ, N(Rₓ)₂, und -CH(Rₓ)₂,
Z H oder Rₓ ist,
R₁, R₂, R₃, R₄ und R₅ unabhängig voneinander -H, -Cl, -Br, -I, -NO₂, -N(Rₓ)₂, -Rₓ, -COORₓ oder -ORₓ sind,
wobei Rₓ ein gegebenenfalls substituierter und / oder verzweigter C₁ bis C₁₈-Alkylrest ist

Verbindungen der allgemeinen Formel **(I),** bei denen Y₁ und Y₂ CH sind und R₁, R₂, R₃, R₄ und R₅ H sind, sind von der Erfindung ausgenommen.

Verbindungen der allgemeinen Formel **(I),** bei denen Y₁ und Y₂ CH sind, R₁= R₃=R₅=CH₃ sind, n= 2 oder 6 ist und Q=CH₃ sind, sind von der Erfindung ausgenommen.

Verbindungen der allgemeinen Formel **(I),** bei denen Y₁ und Y₂ CH sind, R₁, R₂, R₄, R₅=H sind, R₃= ORₓ ist und Rₓ ein Kohlenwasserstoff mit 3 oder 12 Kohlenstoffatomen ist, sind von der Erfindung ausgenommen.

Rₓ ist ein gesättigter C₁ bis C₁₈-Alkylrest, der gegebenenfalls substituiert und / oder verzweigt ist und der ein oder mehrere Sauerstoffatome als Heteroatome enthalten kann. Bevorzugt sind in Rₓ zwischen 1 und 6, besonders bevorzugt zwischen 2 und 4 Sauerstoffatome als Heteroatome enthalten. Bevorzugt bilden die Sauerstoffatome in Rₓ dabei Etherbindungen. Rₓ umfasst keine aromatischen Substituenten. Eine Biphenylsubstitution am Imidazol- bzw-Triazolring ist daher von den Verbindungen der allgemeinen Formel (I) nicht umfasst.

Auch Dikationen, die zwei Imidazol- bzw. Triazolringe umfassen, sind von den Verbindungen der allgemeinen Formel (I) nicht umfasst. Der Imidazol- bzw- Triazolring mit Arylring als "Kernelement" der erfindungsgemäßen Verbindungen ist in diesen also nur einmal umfasst.

Im Folgenden bedeutet Substituenten am Aromaten bzw. Phenylrest, dass mindestens einer der Reste R₁, R₂, R₃, R₄ und R₅ nicht H ist. Zugelassen sind dabei von eins bis fünf Substituenten.

Die Substituenten R₁, R₂, R₃, R₄ und R₅ am Phenylring anellieren nicht, d. h. sie sind in den Verbindungen der allgemeinen Formel (I) nicht derart miteinander oder mit einem Atom des Imidazol- bzw. Triazolrings verbunden, dass dadurch eine ringartige Struktur entsteht.

Bei aus dem Stand der Technik bekannten aliphatisch substituierten Imidazolium- und Triazoliumsalzen reduzieren die sp³-hybridisierten Substituenten an den Stickstoffen die Möglichkeiten einer Beeinflussung des Systems auf die induktiven Effekte. Mesomere Effekte können nicht genutzt werden.

Die erfindungsgemäßen Imidazolium- und Triazoliumkationen sind hingegen mit gegebenenfalls substituierten Aromaten substituiert. Durch die sp²-hybridisierten Substituenten an den Stickstoffen sind aufgrund der mesomeren Effekte Schmelzpunkte und Lösungseigenschaften der daraus abgeleiteten organischen Salze gezielt einstellbar.

Die asymmetrische N,N'-Substitution der erfindungsgemäßen Imidazolium- und Triazoliumkationen durch einen Alkyl- und einen aromatischen Substituenten führt zu vorteilhaften Eigenschaften, die dadurch verstärkt werden, dass am aromatischen Substituenten Gruppen mit +I/+M und -I/-M-Effekt angebracht werden. Für die Wechselwirkung zwischen dem Arylsubstituent und dem Imidazol- bzw. Triazolring ist es zwingend nötig, dass die Konjugation über das ganze System erfolgt, d.h. keine sp³-hybridisierten Atome in den Ringen vorliegen.

Die Stabilität - d.h. die Differenz zwischen Schmelzpunkt und Zersetzungspunkt - der erfindungsgemäßen organischen Salze auf der Basis der Aryl-Alkyl-substituierten Imidazolium- und Triazoliumkationen ist im Vergleich zu den entsprechenden Alkyl-Alkyl-substituierten analogen Verbindungen deutlich erhöht.

Mit Hilfe des mesomeren Effektes unterschiedlicher Aromaten am Imidazol- bzw. Triazolring lässt sich die Ladungsverteilung im Imidazolium- bzw. Triazoliumkation gezielt beeinflussen und der Schmelzpunkt der Verbindungen steuern. Ein Aromat mit +M-Effekt bewirkt hierbei eine bessere Ladungsverteilung und einen niedrigeren Schmelzpunkt des Salzes, während Aromaten mit -M-Effekt den Schmelzpunkt des Salzes ansteigen lassen. Zusätzlich beeinflussen lassen sich die Schmelzpunkte dieser Imidazolium- bzw. Triazoliumsalze über die Symmetrie der Substitution an den Aromaten. Aromaten mit +M-Effekt sowie unsymmetrische Substitutionsmuster am Aromaten führen zu einer deutlichen Absenkung der Schmelzpunkte, während substituierte Aromaten mit -M-Effekt oder hoher Symmetrie in der Substitution zu einer Erhöhung der Schmelztemperatur führen.

In einer Ausführungsform der Erfindung werden am Phenylring Substituenten unter Beachtung der Sterik mit einem +I und/oder +M-Effekt angebracht, wenn der Schmelzpunkt der Verbindung niedrig sein soll. Bevorzugt sind dies die Substituenten Cl, Br, I, ORₓ , Rₓ und N(Rₓ)₂.

In einer anderen Ausführungsform der Erfindung werden am Phenylring unter Beachtung der Sterik Substituenten mit einem -I und/oder -M-Effekt angebracht, wenn der Schmelzpunkt der Verbindung hoch sein soll. Bevorzugt sind dies die Substituenten NO₂ und COORₓ.

Der Schmelzpunkt ist dabei von dem verwendeten Anion abhängig, wobei auch mit sehr verschiedenen funktionellen Gruppen am Aromaten der Austausch der Anionen noch in guten Ausbeuten möglich ist.

Durch die Einführung verschiedener Substituenten an den Aromaten am Imidazol- bzw. Triazoliumring können die physikalischen und chemischen Eigenschaften der Imidazolium- bzw. Triazoliumsalze zusätzlich zu den bestehenden Möglichkeiten der Variation der Anionen und des Alkylrestes spezifisch eingestellt werden. Zu diesem Zweck werden sowohl Einfachals auch Mehrfachsubstitution eingesetzt. Die erfindungsgemäßen aromatisch substituierten Imidazolium- bzw. Triazoliumsalze erlauben es, das ionische Reaktionsmedium für eine spezifische Anwendung durch ein schrittweises Tuning der relevanten Lösungsmitteleigenschaften gezielt zu optimieren. Hier lässt sich der elektronische Einfluss von unterschiedlichen Substituenten am Aromaten nutzen. Die möglichen Substituenten R₁ bis R₅ werden dabei einzeln, mehrfach oder auch in Kombination eingesetzt, um gezielt den gewünschten induktiven oder mesomeren Effekt zu erreichen.

Auch die Symmetrie der Substitutionsmuster am Aromaten hat einen gezielten Einfluß auf die Eigenschaften der Salze und insbesondere auf die Schmelzpunkte. So sind Verbindungen mit Substituenten in der 4- und 2-Position, d. h. mit R1/R5 und R3 nicht gleich H, sowie mit mehreren Substituenten bevorzugte Verbindungen.

Ganz besonders bevorzugt sind erfindungsgemäße Verbindungen, bei denen der Phenylring an der 2- (R₁), der 4- (R₃) und/oder der 6-Position (R₅) substituiert ist, d.h. Verbindungen der allgemeinen Formel (I), bei denen R₂ und R₄ H sind und mindestens ein Rest ausgewählt aus R₁, R₃ und R₅ nicht H ist.

Vorzugsweise ist der aromatische Substituent der erfindungsgemäßen Verbindungen substituiert durch ein oder mehrere ORₓ, Cl, Br, I, N(Rₓ)₂, Rₓ und/oder NO₂, bevorzugt durch ORₓ, Cl, Br, I, N(Rₓ)₂ und/oder Rₓ, besonders bevorzugt durch ORₓ, Rₓ und N(Rₓ)₂. In einer ganz besonders bevorzugten Ausführungsform sind in den Verbindungen der allgemeinen Formel (I) ein oder mehrere der Substituenten R₁, R₂, R₃, R₄ und R₅ unabhängig voneinander ausgewählt aus ORₓ oder Rₓ.

Weiterhin wurde beobachtet, dass durch die Variation der Kettenlänge n der aliphatischen Reste der Schmelzpunkt systematisch und selektiv beeinflusst wird. Während sich bei den Alkyl-Alkyl-substituierten Verbindungen der Schmelzpunkt ab einer gewissen Länge der Alkylkette stark ändert, ist im Falle der Aryl-Alkyl-substituierten Verbindungen ein konstanteres Verhalten beobachtbar. Hier kann eine Korrelation zwischen der Zahl an Kohlenstoffen der Alkylkette und dem erwarteten Schmelzpunkt hergestellt werden. Polare, saure und basische Gruppen können sich sowohl am aromatischen Ring als auch an der aliphatischen Kette befinden.

In den erfindungsgemäßen Verbindungen ist n eine natürliche Zahl zwischen 2 und 14, bevorzugt 4 bis 10, besonders bevorzugt 4 bis 8.

Die Eigenschaften der erfindungsgemäßen Aryl-Alkyl-substituierten Imidazolium- bzw. Triazoliumkationen und ihrer Salze sind somit durch die Variation des aliphatischen Substituenten, die Substituenten am aromatischen Ring (welche wiederum den mesomeren Effekt und die Symmetrie des Aromaten beeinflussen) sowie die Auswahl des Anions über einen extrem weiten Bereich sehr genau einstellbar. Damit ist es synthetisch möglich, für viele Anwendungen ein passendes Lösungsmittel durch die Anpassung der oben genannten Parameter maßzuschneidern.

Die Anionen in den erfindungsgemäßen Verbindungen sind Halogenide (F⁻, Cl⁻, Br⁻, I⁻), Acetat (CH₃COO⁻), Trifluoracetat (CF₃COO⁻), Triflat (CF₃SO₃⁻), Sulfat (SO₄²), Hydrogensulfat (HSO₄⁻), Methylsulfat (CH₃OSO₃⁻), Ethylsulfat (C₂H₅OSO₃⁻), Sulfit (SO₃²⁻), Hydrogensulfit (HSO₃⁻), Aluminiumchloride (AlCl₄⁻, Al₂Cl₇⁻, Al₃Cl₁₀⁻), Aluminiumtribromid (AlBr₄⁻), Nitrit (NO₂⁻), Nitrat (NO₃⁻), Kupferchlorid (CuCl₂⁻), Phosphat (PO₄³⁻), Hydrogenphosphat (HPO₄²⁻), Dihydrogenphosphat (H₂PO₄⁻), Carbonat (CO₃²⁻), Hydrogencarbonat (HCO₃⁻), Hexafluorophosphat (PF₆⁻), Tetrafluoroborat (BF₄⁻), Bis(trifluormethylsulfon)imid (BTSA⁻, (F₃CSO₂)₂N⁻); Tosylat (p-CH₃C₆H₄SO₃⁻); Cyanat (OCN⁻); Isocyanat (NCO⁻); Thiocyanat (SCN⁻), Isothiocyanat (NCS⁻) oder Borate wie z.B. Tetracyanoborat (B(CN)₄⁻); B(OR_{y})₄⁻, wobei R_{y} gegebenenfalls unabhängig voneinander ausgewählte substituierte und / oder verzweigte C₁ bis C₁₈-Alkylreste, C₁ bis C₁₈-Alkenylreste, C₁ bis C₁₈-Alkinylreste, C₆ bis C₁₂-Arylreste und / oder C₇ bis C₃₀-Aralkylreste, die gegebenenfalls ein oder mehrere, bevorzugt zwischen 1 und 4, Sauerstoff- und/oder Stickstoffatome als Heteroatome enthalten, sind; Borate der Grundstruktur B(O-A-O)₂⁻ gemäß den Formeln a) bis c); Borate der Grundstruktur BX₂(OR)⁻ oder BX₂(O-A-O)-gemäß den Formeln d) bis i), N,N-Bis(trifluormethyl)imid (N(CF₃)₂⁻), N(CN)₂⁻ oder N(SO₂C_{z}F_{2z+1})₂⁻ wobei z eine natürliche Zahl zwischen 1 und 20 ist. 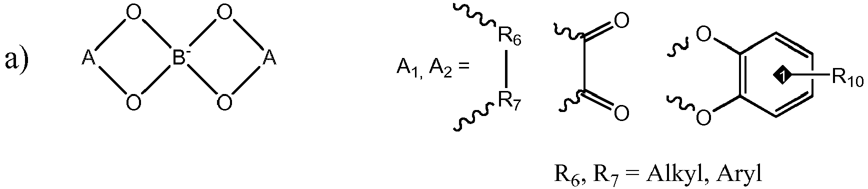 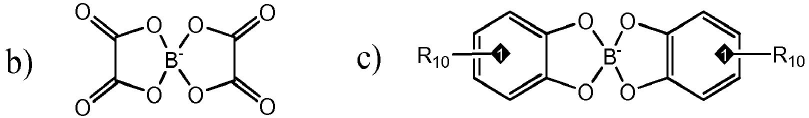 R₁₀ = Alkyl, Aryl, H, Halogen, -NO₂, -NH₂, -NHR₆, -N(R₆)₂, -COOR₆ oder -OR₆

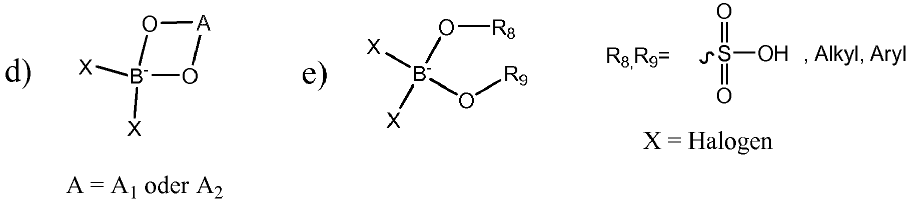

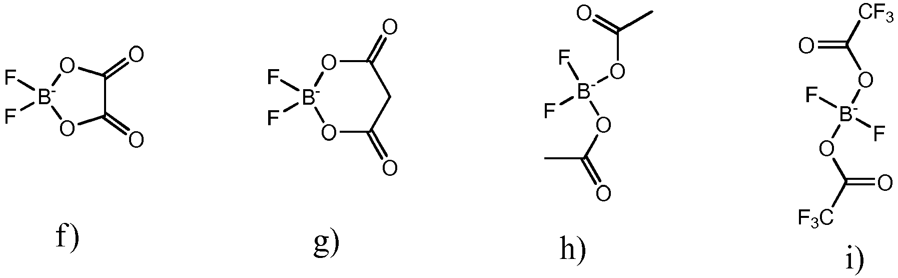

Besonders bevorzugte Anionen sind Cl⁻, Br⁻, I⁻, CH₃COO⁻, CF₃COO⁻, CF₃SO₃⁻, SO₄²⁻, HSO₄⁻, SO₃²⁻, HSO₃⁻, NO₃⁻, CuCl₂⁻, H₂PO₄⁻, HCO₃⁻, PF₆⁻, BF₄⁻, (F₃CSO₂)₂N⁻ und p-CH₃C₆H₄SO₃⁻ oder Borate wie z.B. Tetracyanoborat (B(CN)₄⁻); B(OR_{y})₄⁻, wobei R_{y} gegebenenfalls unabhängig voneinander ausgewählte substituierte und / oder verzweigte C₁ bis C₁₈-Alkylreste, C₁ bis C₁₈-Alkenylreste, C₁ bis C18-Alkinylreste, C₆ bis C₁₂-Arylreste und / oder C₇ bis C₃₀-Aralkylreste, die gegebenenfalls ein oder mehrere, bevorzugt zwischen 1 und 4, Sauerstoff- und/oder Stickstoffatome als Heteroatome enthalten, sind; Borate der Grundstruktur B(O-A-O)₂⁻ gemäß den Formeln a) bis c); Borate der Grundstruktur BX₂(OR)⁻ oder BX₂(O-A-O)⁻ gemäß den Formeln d) bis i), N,N-Bis(trifluormethyl)imid (N(CF₃)₂⁻), N(CN)₂⁻ oder N(SO₂C_{z}F_{2z+1})₂⁻ wobei z eine natürliche Zahl zwischen 1 und 20 ist. Ganz besonders bevorzugte Anionen sind Cl⁻, Br⁻, I⁻ CH₃COO⁻, CF₃COO⁻, CF₃SO3⁻, PF₆⁻, BF₄⁻, (F₃CSO₂)₂N⁻ oder Borate wie z.B. Tetracyanoborat (B(CN)₄⁻); B(OR_{y})₄⁻, wobei R_{y} gegebenenfalls unabhängig voneinander ausgewählte substituierte und / oder verzweigte C₁ bis C₁₈-Alkylreste, C₁ bis C₁₈ Alkenylreste, C₁ bis C₁₈-Alkinylreste, C₆ bis Cₗ₂-Arylreste und / oder C₇ bis C₃₀-Aralkylreste, die gegebenenfalls ein oder mehrere, bevorzugt zwischen 1 und 4, Sauerstoff- und/oder Stickstoffatome als Heteroatome enthalten, sind; Borate der Grundstruktur B(O-A-O)₂⁻ gemäß den Formeln a) bis c); Borate der Grundstruktur B(O-A-O)₂⁻ gemäß den Formeln a) bis c); Borate der Grundstruktur BX₂(OR)⁻ oder BX₂(O-A-O)⁻ gemäß den Formeln d) bis i), N,N-Bis(trifluormethyl)imid (N(CF₃)₂⁻), N(CN)₂⁻ oder N(SO₂C_{z}F_{2z+1})₂⁻ wobei z eine natürliche Zahl zwischen 1 und 20 ist.

In einer bevorzugten Ausführungsform der erfindungsgemäßen Imidazolium- oder Triazoliumsalze ist das Anion X⁻ ausgewählt aus der Gruppe Br⁻ (Bromid), I⁻ (Iodid), PF₆⁻ (Hexafluorophosphat), BF₄⁻ (Tetrafluoroborat) und N(SO₂CF₃)₂⁻ (BTSA, Bis(trifluormethylsulfon)imid).

In einer weiteren bevorzugten Ausführungsform des erfindungsgemäßen Salzes sind Y₁ und Y₂ CH, R₁, R₂, R₄ und R₅ sind H und R₃ ist ausgewählt aus -NO₂, -Cl, -Br, -COOEt, -CH₃, - OEt, -OMe und -OH.

In einer weiteren bevorzugten Ausführungsform des erfindungsgemäßen Salzes sind Y₁ und Y₂ CH, R₂ und R₄ sind H und R₁, R₃ und R₅ sind -CH₃.

In einer weiteren bevorzugten Ausführungsform des erfindungsgemäßen Salzes sind Y₁ und Y₂ CH, R₂, R₃ und R₄ sind H und R₁ und R₅ sind ausgewählt aus -CH(CH₃)₂, -Cl und -CF₃.

In einer weiteren bevorzugten Ausführungsform des erfindungsgemäßen Salzes sind Y₁ und Y₂ CH, R₁, R₃ und R₅ sind H und R₂ und R₄ sind -CF₃.

In einer weiteren bevorzugten Ausführungsform des erfindungsgemäßen Salzes ist Z H oder-CH₃.

Bevorzugt ist Q ausgewählt aus Rₓ, ORₓ, CH(Rₓ)₂, SO₃Rₓ und COORₓ, vorzugsweise aus Rₓ, ORₓ, CH(Rₓ)₂ und SO₃Rₓ, besonders bevorzugt aus Rₓ" ORₓ und CH(Rₓ)₂.

Weiter bevorzugt ist Q ausgewählt aus -CH₃, -OH, -OCH₃, -SO₃H, -SO₃Rₓ, -COOH,-COOCH₃, -COCH₃, NH₂, -NHCH₃, N(CH₃)₂, und -CH(CH₃)₂.

Ganz besonders bevorzugt sind Verbindungen der allgemeinen Formel mit Q = ORₓ.

Besonders bevorzugte Ausführungsformen des erfindungsgemäßen Imidazoliumsalzes sind die folgenden Einzelverbindungen:
3-Butyl-1-mesitylimidazoliumbromid
1-Mesityl-3-pentylimidazoliumbromid
3-Hexyl-1-mesitylimidazoliumbromid
1-Mesityl-3-octylimidazoliumbromid
1-Mesityl-3-undecylimidazoliumbromid
1-Mesityl-3-tetradecylimidazoliumbromid
1-Isopentyl-3-mesitylimidazoliumbromid
3-(2-Hydroxyethyl-) 1-mesityl-imidazoliumbromid
3-(2-Carboxyethyl)-1-mesityl-imidazoliumbromid
3-(2-Carboxyethyl)-1-(4-nitrophenyl)imidazoliumbromid
1-Mesitylimidazolium-3-propan-1-sulfonat
1-(2-Methoxyphenyl)-3-propylimidazoliumbromid
1-(4-Bromphenyl)-3-propylimidazoliumbromid
1-(4-Bromphenyl)-3-butylimidazoliumbromid
1-(4-Bromphenyl)-3-hexylimidazoliumbromid
1-(4-Bromphenyl)-3-heptylimidazoliumbromid
1-(4-Bromphenyl)-3-tetradecylimidazoliumbromid
1-(4-Chlorphenyl)-3-propylimidazoliumbromid
1-(4-Chlorphenyl)-3-heptylimidazoliumbromid
1-(4-Chlorphenyl)-3-tetradecylimidazoliumbromid
1-(4-Ethylcarboxyphenyl)-3-propylimidazoliumbromid
1-(4-Ethylcarboxyphenyl)-3-hexylimidazoliumbromid
1-(4-Ethylcarboxyphenyl)-3-heptylimidazoliumbromid
1-(4-Ethylcarboxyphenyl)-3-tetradecylimidazoliumbromid
1-(3,5-Bis(trifluormethyl)phenyl)-3-propylimidazoliumbromid
3-Propyl-1-(4-nitrophenyl)-imidazoliumbromid
1-(4-Nitrophenyl)-3-heptylimidazoliumbromid
1-(4-Nitrophenyl)-3-tetradecylimidazoliumbromid
1-(4-Methylphenyl)-3-propylimidazoliumbromid
1-(2-Methylphenyl)-3-propylimidazoliumbromid
1-(2,6-Diisopropylphenyl)-3-hexylimidazoliumbromid
1-(2-Ethoxyphenyl)-3-propylimidazoliumbromid
1-(2-Ethylphenyl)-3-propylimidazoliumbromid
1-(2-Ethoxyphenyl)-3-hexylimidazoliumbromid
1-(2-Ethoxyphenyl)-3-heptylimidazoliumbromid
1-(4-Ethoxyphenyl)-3-hexylimidazoliumbromid
1-(4-Methoxyphenyl)-3-propylimidazoliumbromid
3-Butyl-1-(2-Ethylphenyl)-imidazoliumbromid
3-Butyl-1-(4-Ethylphenyl)-imidazoliumbromid
3-Butyl-1-(4-Ethoxylphenyl)-imidazoliumbromid
3-Hexyl-1-(4-Ethylphenyl)-imidazoliumbromid
3-Hexyl-1-(4-methoxyphenyl)imidazoliumbromid
3-Hexyl-1-(4-methylphenyl)imidazoliumbromid
3-Hexyl-1-(2-Ethylphenyl)imidazoliumbromid
1-(4-Methoxyphenyl)-3-heptylimidazoliumbromid
3-Hexyl-1-(2-methoxyphenyl)imidazoliumbromid
3-Hexyl-1-(2-methylphenyl)imidazoliumbromid
3-Heptyl-1-(2-methoxyphenyl)imidazoliumbromid
1-(3,5-Bistrifluormethylphenyl)-3-heptylimidazoliumbromid
3-Hexyl-1-(4-methylphenyl)imidazoliumiodid
3-Hexyl-1-(2-Methoxyphenyl)imidazoliumiodid
1-(4-Bromphenyl)-3-hexylimidazoliumiodid
1-(4-Bromphenyl)-3-butylimidazoliumiodid
3-Butyl-1-(4-Methoxyphenyl)-imidazoliumiodid
3-Butyl-1-(4-Chlorphenyl)-imidazoliumiodid
3-Butyl-1-(2-Methylphenyl)-imidazoliumiodid
3-Butyl-1-(4-Ethylphenyl)-imidazoliumiodid
3-Hexyl-1-(4-Ethylphenyl)-imidazoliumiodid
3-Butyl-1-(2-Ethylphenyl)-imidazoliumiodid
3-Hexyl-1-(2-Ethylphenyl)-imidazoliumiodid
1-(2-Ethylphenyl)-3-undecylimidazoliumiodid
3-Butyl-1-(4-Ethoxylphenyl)-imidazoliumiodid
3-Hexyl-1-(4-Ethoxyphenyl)-imidazoliumiodid
1-(4-Ethoxylphenyl)-3-undecylimidazoliumiodid
1-Butyl-3-mesitylimidazoliumbis(trifluormethylsulfon)imid
1-Mesityl-3-pentylimidazoliumbis(trifluormethylsulfon)imid
1-Hexyl-3-mesitylimidazoliumbis(trifluormethylsulfon)imid
1-Mesity-3-octylimidazoliumbis(trifluormethylsulfon)imid
1-Mesityl-3-undecylimidazoliumbis(trifluormethylsulfon)imid
1-Mesityl-3-tetradecylimidazoliumbis(trifluormethylsulfon)imid
1-(4-Bromphenyl)-3-propylimidazoliumbis(trifluormethylsulfon)imid
1-(4-Bromphenyl)-3-butylimidazoliumbis(trifluormethylsulfon)imid
1-(4-Bromphenyl)-3-heptylimidazoliumbis(trifluormethylsulfon)imid
1-(4-Bromphenyl)-3-tetradecylimidazoliumbis(trifluormethylsulfon)imid
1-(4-Chlorphenyl)-3-propylimidazoliumbis(trifluormethylsulfon)imid
1-(4-Chlorphenyl)-3-heptylimidazoliumbis(trifluormethylsulfon)imid
1-(4-Chlorphenyl)-3-tetradecylimidazoliumbis(trifluormethylsulfon)imid
1-(4-Methylphenyl)-3-propylimidazoliumbis(trifluormethylsulfon)imid
1-(4-Ethylcarboxyphenyl)-3-propylimidazoliumbis(trifluormethylsulfon)imid
1-(4-Ethylcarboxyphenyl)-3-heptylimidazoliumbis(trifluormethylsulfon)imid
1-(4-Ethylcarboxyphenyl)-3-tetradecylimidazoliumbis(trifluormethylsulfon)imid
1-(4-Nitrophenyl)-3-propylimidazolium-bis(trifluormetylsulfon)imid
1-(4-Nitrophenyl)-3-heptylimidazolium bis(trifluormetylsulfon)imid
1-Butyl-3-mesitylimidazoliumtetrafluoroborat
1-Mesityl-3-pentylimidazoliumtetrafluoroborat
1-Hexyl-3-mesitylimidazoliumtetrafluoroborat
3-Mesityl-1-octyl-imidazoliumtetrafluoroborat
1-Mesityl-3-undecylimidazoliumtetrafluoroborat
1-Mesityl-3-tetradecylimidazoliumtetrafluoroborat
1-Butyl-3-mesitylimidazoliumhexafluorophosphat
1-Mesityl-3-pentylimidazoliumhexafluorophosphat
1-Hexyl-3-mesitylimidazoliumhexafluorophosphat
1-Mesityl-3-octyl-imidazoliumhexafluorophosphat
1-Mesityl-3-undecylimidazoliumhexafluorophosphat
1-Mesityl-3-tetradecylimidazoliumtetrafluoroborat

In einer bevorzugten Ausführungsform der Erfindung ist Y₁ N, Y₂ ist CH und R₁, R₂, R₃, R₄ und R₅ sind H.

Besonders bevorzugte Ausführungsformen der erfindungsgemäßen Triazoliumsalze der allgemeinen Formel (I) mit Y₁ = N sind die folgenden Einzelverbindungen:
1-Phenyl-4-(prop-1-yl)triazoliumbromid
1-Phenyl-4-(hex-1-yl)triazoliumbromid
1-Phenyl-4-(tetradec-1-yl)triazoliumbromid
1-Phenyl-4-(hept-1-yl)triazoliumbromid
1-Phenyl-4-(pent-1-yl)triazoliumbromid
1-Phenyl-4-(undec-1-yl)triazoliumbromid
1-Phenyl-4-(hex-1-yl)triazoliumbis(trifluormethylsulfon)imid
1-Phenyl-4-(prop-1-yl)triazoliumbis(trifluormethylsulfon)imid
1-Phenyl-4-(pent-1-yl)triazoliumbis(trifluormethyl)sulfonimid
1-Phenyl-4-(hept-1-yl)triazoliumbis(trifluormethyl)sulfonimid
1-Phenyl-4-(undec-1-yl)triazoliumbis(trifluormethylsulfon)imid
1-Phenyl-4-(tetradec-1-yl)triazoliumbis(trifluormethylsulfon)imid

In einer bevorzugten Ausführungsform der Erfindung ist Y₁ CH, Y₂ ist N und R₁, R₂, R₃, R₄ und R₅ sind H.

Besonders bevorzugte Ausführungsformen der erfindungsgemäßen Triazoliumsalze der allgemeinen Formel (I) mit Y₂ = N sind die folgenden Einzelverbindungen:
4-Phenyl-1-(prop-1-yl)triazoliumbromid
4-Phenyl-1-(pent-1-yl)triazoliumbromid
4-Phenyl-1-(hept-1-yl)triazoliumbromid
4-Phenyl-1-(hex-1-yl)triazoliumbromid
4-Phenyl-1-(undec-1-yl)triazoliumbromid
4-Phenyl-1-(tetradec-1-yl)-(1,2,4)-triazoliumbromid
4-Phenyl-1-(prop-1-yl)triazoliumbis(trifluormethylsulfon)imid
1-Phenyl-4-(pent-1-yl)triazoliumbis(trifluormethyl)sulfonimid
4-Phenyl-1-(hex-1-yl)triazoliumbis(trifluormethyl)sulfonimid
4-Phenyl-1-(hept-1-yl)triazoliumbis(trifluormethyl)sulfonimid
4-Phenyl-1-(undec-1-yl)triazoliumbis(trifluormethylsulfon)imid
4-Phenyl-1-(tetradec-1-yl)triazoliumbis(trifluormethylsulfon)imid.

Die erfindungsgemäßen Salze der allgemeinen Formel (I) werden wie folgt hergestellt.

Zur Herstellung eines erfindungsgemäßen Salzes der allgemeinen Formel (I), bei dem Y₁ und Y₂ H sind, wird beispielsweise zunächst das entsprechende N-substituierte Imidazol aus einem aromatischen Amin hergestellt. Dazu werden das entsprechende Anilin und eine Glyoxallösung vorgelegt und gerührt, bis ein Niederschlag aus der Lösung ausfällt. Die so gebildete Suspension wird mit einem geeigneten Lösungsmittel, wie z. B. Methanol verdünnt und mit einer Ammoniumverbindung, wie z. B. Ammoniumchlorid, Ammoniumcarbonat, Ammoniumhydrogencarbonat, Ammoniumsulfat und einem Aldehyd, z. B. Formaldehyd versetzt. Anschließend wird die Reaktionsmischung gegebenenfalls erhitzt.

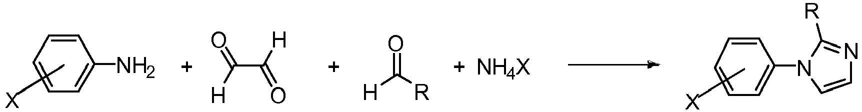

Das so erhaltene N-substituierte Imidazol wird in einem geeigneten Lösungsmittel, wie z. B. THF gelöst, anschließend wird das 1-Halogenalkan zugegeben. Gegebenenfalls wird die Reaktionsmischung erwärmt. Anschließend wird das Produkt abgetrennt, gewaschen und getrocknet.

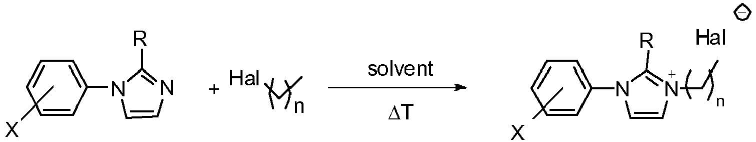

Neben der angeführten Synthese gibt es noch weitere Synthesemethoden, die dem Fachmann bekannt sind. Der Aromat kann auch im Falle des Imidazols über bekannte Kupplungsreaktionen (vgl. z.B. Buchwald-Hartwig-Kupplung, im Detail unten bei den Triazoliumverbindungen beschrieben) eingebracht werden oder über bekannte Synthesen, ausgehend von Isonitrilen (R.S. Bon et al., J. Org. Chem. 2005, 70, 3542; R.S. Bon et al., Org. Lett. 2003, 5, 3759) und anschließender Oxidation oder über Arine (H. Yoshida, S. Sugiura, A. Kunai, Org. Lett. 2002, 4, 2767-2769), hergestellt werden.

Der Austausch des Halogenid-Anions erfolgt nach einem dem Fachmann bekannten Verfahren, z.B. mit Hilfe von Silber-, Alkalimetall- oder Ammoniumsalzen, oder aber durch Säure/Base-Neutralisationsreaktionen (Hurley, F. H.; Wier, T. P., Jr., J. Electrochem. Soc. 1951, 98, 203-206; Chan, B. K. M.; Chang, N.-H.; Grimmett, M. R., Austr. J. Chem. 1977, 30, (9), 2005-2013; Wasserscheid/Welton, "Ionic liquids in Synthesis", Wiley-VCH 2007).

Die erfindungsgemäßen Triazoliumsalze der allgemeinen Formel (I), bei dem Y₁ CH und Y₂ N ist oder Y₁ N und Y₂ CH ist werden wie folgt hergestellt.

Die Herstellung eines erfindungsgemäßen Triazoliumsalzes der allgemeinen Formel (I), bei dem Y₁ CH und Y₂ N ist, erfolgt in zwei Reaktionsschritten. Dabei wird der 1,2,4-Triazolring (z.B. gemäß H.G.O. Becker, Gerda Hoffman, Kim Mun Gwan, L. Knüpper, Journal für praktische Chemie, Bnd. 330, Heft3, 1988, 325-337 und darin zitierter Referenzen) aus dem entsprechenden ggf. substituierten Anilin aufgebaut. Im zweiten Schritt wird - wie bereits oben beschrieben - das organische Salz hergestellt. Auch hier werden die Anionen nach bekannten Verfahren ausgetauscht.

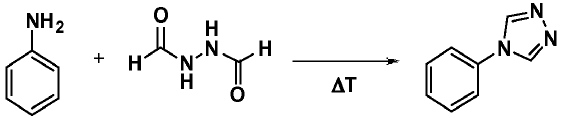

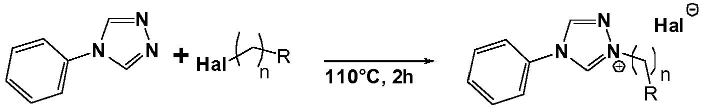

Triazole mit verschiedenen Substituenten in 3-Position können durch einfache Reaktionen synthetisiert werden. Die Einführung von Substituenten in der 3-Position erfolgt durch dem Fachmann bekannte Ringaufbaureaktionen.

Die Herstellung eines erfindungsgemäßen Triazoliumsalzes der allgemeinen Formel (I), bei dem Y₁ N und Y₂ CH ist, erfolgt ebenfalls in zwei Reaktionsschritten. Dabei wird der Triazolring vorzugsweise durch die Buchwald-Hartwig-Kupplung (z.B. nach Henri-Jean Cristau, Pascal P. Cellier, Jean-Francis Spindler, Marc Taillefer, Chem. Eur. J. 2004, 10, 5607-5622), aufgebaut. Im zweiten Schritt wird - wie bereits oben beschrieben - das organische Salz hergestellt. Auch hier werden die Anionen nach bekannten Verfahren ausgetauscht.

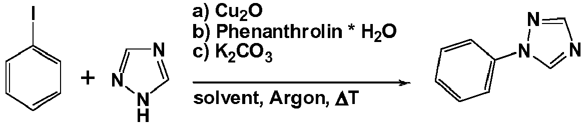

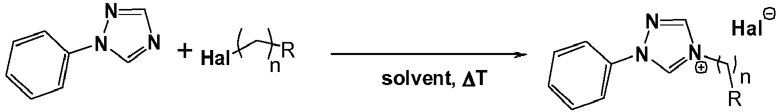

Weitere Verfahren zur Herstellung der erfindungsgemäßen Salze der allgemeinen Formel (I) sind dem Fachmann beispielsweise aus M. R. Grimmett et. al.; Imidazole and Benzimidazole Synthesis; Academic Press; (1997) oder aus Gilchrist, Thomas; Heterocyclenchemie; VCH (1995) sowie A.R. Katritzky et al.; Comprehensive Heterocyclic Chemistry III: Elsevier; (2008) bekannt.

Bestandteil der Erfindung ist auch die Verwendung eines erfindungsgemäßen Salzes, welches ein geeignetes Anion aufweist, als ionische Flüssigkeit.

Bei Auswahl eines geeigneten Anions sind die erfindungsgemäßen Imidazolium- oder Triazoliumsalze bei Temperaturen unter 100°C, vorzugsweise bei Raumtemperatur, flüssig und als ionische Flüssigkeit einsetzbar.

Ein Austausch der Bromid-Anionen der erfindungsgemäßen Salze gegen die nicht koordinierenden, voluminöseren BTSA⁻, BF₄⁻ oder PF₆⁻ Anionen führt z.B. zu einer starken Absenkung der Schmelzpunkte dieser Salze gegenüber den Schmelzpunkten der Bromid-Salze mit dem gleichen Kation.

Die so erhältlichen ionischen Flüssigkeiten mit BTSA⁻-Anionen zeichnen sich weiterhin durch eine hervorragende thermische Stabilität (Zersetzung bei > 400 °C) und einen flüssigen Bereich von mehr als 300 °C aus.

Die beschriebenen Eigenschaften eignen sich daher besonders für eine Verwendung in Batterien (bzw. Doppelschichtkondensatoren) und Brennstoffzellen, wo eine hohe Temperaturstabilität in Kombination mit guter Leitfähigkeit nötig ist. Bestandteil der Erfindung ist daher die Verwendung der beschriebenen Verbindungen als Zusatz in Batterien, Doppelschichtkondensatoren und Brennstoffzellen.

Ebenfalls Bestandteil der Erfindung ist die Verwendung eines erfindungsgemäßen Salzes oder einer erfindungsgemäßen ionischen Flüssigkeit als Lösungsmittel oder Lösungsmittelzusatz bei der elektrochemischen Anwendungen, z.B. der Abscheidung von Metallen ("Plating"). Hier erlaubt das besonders weite elektrochemische Fenster in Kombination mit der Stabilisierung der bei der Abscheidung entstehenden Intermediate durch die besonderen Eigenschaften der gezielt substituierten Aromaten

Die beschriebenen Eigenschaften eignen sich aufgrund der großen Schmelzwärme ebenfalls für die Verwendung als "Phase Changing Materials", also bei Materialien, die extreme Temperaturschwankungen ausgleichen. Bestandteil der Erfindung ist daher die Verwendung der beschriebenen Verbindungen bei thermodynamischen Anwendungen.

Ebenfalls Bestandteil der Erfindung ist die Verwendung eines erfindungsgemäßen Salzes oder einer erfindungsgemäßen ionischen Flüssigkeit als Lösungsmittel oder Lösungsmittelzusatz, z.B. in Trennprozessen oder bei synthetischen oder katalytischen Reaktionen.

Solche Lösungen können auch in Solarzellen oder OLEDs eingesetzt werden, da sich durch die Substituenten am aromatischen Ring für die Lichteinwirkung vorteilhafte Eigenschaften erzielen lassen, z.B. hohe Quantenausbeuten (Fluoreszenz).

Ebenfalls Bestandteil der Erfindung ist die Verwendung eines erfindungsgemäßen Salzes oder einer erfindungsgemäßen ionischen Flüssigkeit als Lösungsmittel für nachwachsende Rohstoffe wie z.B. Cellulose.

Eine weitere Ausführungsform der Erfindung sind auch Verfahren zur chemischen Umwandlung, umfassend das erfindungsgemäße Salz oder die erfindungsgemäße ionischen Flüssigkeit als Lösungsmittel oder Lösungsmittelzusatz. Ein solches erfindungsgemäßes Verfahren kann Übergangsmetall-, Enzym- oder mit anderen Biokatalysatoren katalysierte Reaktionsschritte umfassen, die vorzugsweise ausgewählt sind aus Hydroformulierungsreaktionen, Oligomerisierungsreaktionen und anderen C-C-Bindungsverknüpfungsreaktionen, Veresterungen, Isomerisierungsreaktionen und Reaktionen zur Amidbindungsverknüpfung.

Ebenfalls Bestandteil der Erfindung ist die Verwendung eines erfindungsgemäßen Salzes oder einer erfindungsgemäßen ionischen Flüssigkeit als Lösungsmittel bei der Depolymerisation von Polymeren.

Eine Ausführungsform der Erfindung ist auch die Verwendung des erfindungsgemäßen Salzes oder der erfindungsgemäßen ionischen Flüssigkeit zur Stofftrennung, z.B. zur Extraktion aus Ethergemischen. Die Stofftrennung kann dabei auch mittels Gaschromatographie-Verfahren stattfinden, die sowohl die π-π-Wechselwirkungen des aromatischen Ringes und/oder die elektronischen Effekte der Substituenten R₁-R₅ nutzen.

Die erfindungsgemäßen ionischen Flüssigkeiten lösen sich z.B. in zyklischen Ethern wie Tetrahydrofuran (THF), jedoch nicht in nicht-zyklischen Ethern wie Diethylether. Die unterschiedliche Löslichkeit der ionischen Flüssigkeit in Ethern kann zur Abtrennung von in offenkettigen Ethern löslichen Verbindungen ausgenutzt werden, da sich die ionische Flüssigkeit darin nicht löst. Jedoch erlaubt ihre hervorragende THF-Löslichkeit das Auswaschen der ionischen Flüssigkeit.

Die vorliegende Erfindung umfasst daher auch ein Verfahren zur Stofftrennung, umfassend die erfindungsgemäße ionische Flüssigkeit als Lösungsmittel oder Lösungsmittelzusatz, z.B. ein Verfahren zur Extraktion oder zur Trennung von Gemischen mittels organischer zyklischer und offenkettiger Ether.

Die erfindungsgemäßen Salze bzw. ionischen Flüssigkeiten eignen sich auch als Material zur Wasserstoffspeicherung.

Die Erfindung umfasst daher auch die Verwendung der erfindungsgemäßen Salze bzw. ionischen Flüssigkeiten zur Wasserstoffspeicherung.

Wasserstoff wird derzeit in vielen Bereichen als sauberer Energieträger in Betracht gezogen. Im Gegensatz zu den heute verwendeten Kohlenwasserstoffen wird bei der Umwandlung von Wasserstoff in Energie kein CO₂ freigesetzt. Eines der wichtigsten ungelösten Probleme, das einer weitergehenden Nutzung von Wasserstoff aber derzeit noch entgegensteht, ist ein Verfahren für eine sichere und wirtschaftliche Speicherung von Wasserstoff. Als konventionelle Speichersysteme für Wasserstoff werden heute vorwiegend Hochdrucktanks und isolierte Flüssigwasserstofftanksysteme, wie auch die Speicherung in Kohlenwasserstoffen verwendet (E. Fakioglu, Y. Yurum, T. Nejat Veziroglu, Int. J. Hydrogen Energy 2004, 29, 1371). Chemische Methoden zur Speicherung von Wasserstoff beinhalten die Verwendung von Metallhydriden (z.B. MgH₂) (S. R. Johnson, P. A. Anderson, P. P. Edwards, I. Gameson, J. W.Prendergast, M. Al-Mamouri, D. Book, I. R. Harris, J. D. Speight, A. Walton, Chem. Commun. 2005, 2823), Imiden (z.B. LiNH₂) (P. Chen, Z. Xiong, J. Luo, J. Lin, K. L. Tan, Nature 2002, 420, 302), metallorganischen Gerüsten (MOF) (z.B. Zn₄O(1,4-benzodicarboxylat) (N. L. Rosi, J. Eckert, M. Eddaoudi, D. T. Vodak, J. Kim, M. OMKeeffe, O. M. Yaghi, Science 2003, 300, 1127; M. Latroche, S. Surblé, N. C. Serre, C. Mellot-Draznieks, P. L.Llewellyn, J. Lee, J. Chang, S. H. Jhung, G. Férey, Angew. Chem. Int. Ed. 2006, 45, 8227), Alkalimetalltetrahydroboriden (z.B. LiBH₄) (A. ZOttel, P. Wenger, S. Rentsch, P. Sudan, P. Mauron, C.Emmenegger, J. Power Sources 2003, 118, 1), Alanaten (z.B. NaAlH₄) (A. F. Hollemann, N. Wiberg, Lehrbuch der Anorganischen Chemie, 1995, Walter de Gruyter, Berlin New York, 249-264) und chemischen Hydriden.

Wasserstoff ist bei Zimmertemperatur ein farb- und geruchloses Gas, das ca. 14,4 mal leichter als Luft ist (Dichte bei 0°C und 70 torr: 0,089870 g/L) (A. F. Hollemann, N. Wiberg, Lehrbuch der Anorganischen Chemie, 1995, Walter de Gruyter, Berlin New York, 249-264). Weiterhin hat Wasserstoff nach Helium die tiefste Schmelz- (-259,19 °C) und Siedetemperatur (-252,76 °C). Die kritische Temperatur von Wasserstoff, überhalb der Wasserstoff nicht mehr verflüssigt werden kann, liegt bei -239,96 °C. Der kritische Druck bei 13,10 bar (A. F. Hollemann, N. Wiberg, Lehrbuch der Anorganischen Chemie, 1995, Walter de Gruyter, Berlin New York, 249-264). Für eine Speicherung von Wasserstoff in flüssiger Form werden also extrem aufwendige technische Maßnahmen für Kühlung und Temperaturisolation notwendig. Zudem beträgt die Dichte selbst am kritischen Punkt nur 31 g/L (A. F. Hollemann, N. Wiberg, Lehrbuch der Anorganischen Chemie, 1995, Walter de Gruyter, Berlin New York, 249-264). Wasserstoff ist außerdem das Element mit der geringsten Dichte. Daher diffundiert Wasserstoff leicht durch poröse Trennwände und sogar durch Metalle (z.B. Platin).

Von besonderem Interesse ist die Speicherung von Wasserstoff in einem geeigneten Lösungsmittel. Die Löslichkeit von Wasserstoff in gängigen Lösungsmitteln ist aber sehr gering, in 100 L Wasser lösen sich bei 0 °C und einem Druck von 1013 mbar 2.15 L (0,1932205 g) Wasserstoff.

Durch eine chemische Speicherung in Form der aus dem Stand der Technik bekannten ionischen Flüssigkeiten können einige der Probleme der "konventionellen" Wasserstoffspeichersysteme vermieden werden. Zudem wird in einigen Fällen auch eine höhere Speicherdichte an Wasserstoff als bei konventionellen Hochdruck- oder Flüssigwasserstofftanksystemen erreicht, allerdings stehen diese Systeme nach der Freisetzung des Wasserstoffes in der Regel nicht oder nur nach aufwendiger Aufbereitung für eine erneute Wasserstoffspeicherung zur Verfügung. Weiterhin werden zur Freisetzung des gespeicherten Wasserstoffs häufig erhöhte Temperaturen, zum Teil von > 500 °C benötigt (V. Sit, R. A. Geanangel, W. W. Wendlandt, Thermochim. Acta 1987, 113, 379; J. S. Wang, R. A. Geanangel, Inorg. Chim. Acta 1988, 148, 185; G. Wolf, J. Baumann, F. Baitalow, F. P. Hoffmann, Thermochim. Acta 2000, 343, 19; F. Baitalow, J. Baumann, G. Wolf, K. Jaenicke-RPßbler, G. Leitner, Thermochim. Acta 2002, 391, 159; J. Baumann, F. Baitalow, G. Wolf, Thermochim. Acta 2005, 343,19).

Aus den erfindungsgemäßen ionischen Flüssigkeiten lässt sich der gespeicherte Wasserstoff hingegen einfacher wieder freisetzen. Zudem kann die erfindungsgemäße ionische Flüssigkeit nach der Freisetzung des gelösten Wasserstoffs ohne weitere Nachbehandlung direkt wieder für die erneute Wasserstoffspeicherung genutzt werden.

Die Wasserstoffspeicherung erfolgt dabei bevorzugt durch das Lösen von Wasserstoff in einer erfindungsgemäßen ionischen Flüssigkeit bei Temperaturen zwischen -50 °C und + 300 °C, besonders bevorzugt bei -10 °C - 180 °C, ganz besonders bevorzugt bei 0-60 °C sowie einem Druck von 0,1-400 bar, bevorzugt von 0,5-300 bar, besonders bevorzugt von 1-20 bar.

Die Wasserstofffreisetzung aus der erfindungsgemäßen ionischen Flüssigkeit kann aufgrund der niedrigen Dampfdrücke dieser erfindungsgemäßen ionischen Flüssigkeiten durch eine Verringerung des umgebenden Gasdrucks erfolgen. Bevorzugt für die Wasserstofffreisetzung sind dabei Drücke < 5 bar, besonders bevorzugt < 1 bar, ganz besonders bevorzugt < 0,8 bar. Weiterhin kann die Wasserstofffreisetzung auch durch eine Temperaturerhöhung der ionischen Flüssigkeit erfolgen. Bevorzugt sind dabei Temperaturen von 0 bis 350 °C, besonders bevorzugt von 20 bis 180 °C, ganz besonders bevorzugt von 60 bis 140 °C.

Anhand folgender Ausführungsbeispiele wird die Erfindung näher erläutert.

### Allgemeine synthetische Arbeitstechniken

Umsetzungen mit metallorganischen Verbindungen wurden, soweit angegeben unter Ausschluss von Luft und Feuchtigkeit in ausgeheizten Glasapparaturen unter Anwendung der Schlenktechnik durchgeführt. Als Inertgase dienen Stickstoff oder Argon (Reinheit 99,996%), welche ohne weitere Reinigung verwendet wurden.

Pentan und Hexan wurden über CaCl₂ getrocknet. THF wurde durch Kochen unter Rückfluss über Natriummetall getrocknet und gereinigt, Dichlormethan wurde über wasserfreiem CaCl₂ vorgetrocknet, die Haupttrocknung erfolgt durch Destillation über Calciumhydrid unter Argon. DMF wurde durch Destillation unter Argon und Methanol durch mehrtägiges Erhitzen über Magnesium von restlichem Wasser befreit.

### NMR Spektroskopie

Die Messung der NMR Spektren wurden entweder mit einem Bruker AC 300 P (¹H: 300,1 MHz, ¹³C:75,5 MHz, ¹⁹F: 282,4 MHz) oder einem Bruker DRX 500 (¹H: 499,8 MHz, ¹³C: 125,8 MHz, ¹⁹F: 470,3 MHz) Kernresonanzspektrometer in deuterierten Lösungsmitteln durchgeführt. Verschiebungen der ¹H- und ¹³C-Resonanzen sind in ppm relativ zu Tetramethylsilan angegeben, referenziert wird dabei auf das Restsignal des deuterierten Lösungsmittels. Um die richtige Zuordnung der ¹³C-Signale zu gewährleisten, wurden von allen Verbindungen sowohl die ¹³C-Spektren wie auch die DEPT-Spektren aufgenommen. Verschiebungen der ¹⁹F-Resonanzen sind relativ zu CCl₃F angegeben. Kopplungskonstanten werden ohne Angabe des Vorzeichens in Hz angegeben, wobei für die Multiplizitäten der einzelnen Signale folgende Abkürzungen benutzt werden: s: Singulett; d: Dublett; t: Triplett; q: Quartett; qi: Quintett; sept: Septett; m: Multiplett; b: breit.

### Allgemeine Vorschrift zur Synthese von N-substituierten Imidazolen aus aromatischen Aminen

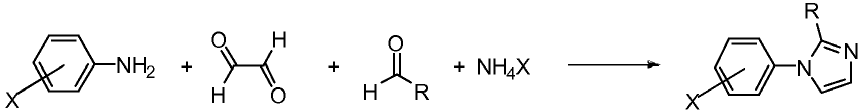

Im Folgenden wird die allgemeine Synthesevorschrift beispielhaft für die Verwendung von Formaldehyd als Aldehyd und Ammoniumchlorid als Ammoniumverbindung beschrieben. In einem 1L-Zweihalskolben mit Rückflusskühler werden in 50 mL Methanol 0,1 mol des entsprechenden Anilins und 11,4 mL einer 40%-igen wässrigen Glyoxallösung (14,42 g, 0,1 mol) vorgelegt und zunächst bei Raumtemperatur gerührt, bis ein voluminöser gelber Niederschlag aus der Lösung ausfällt. Bei aktivierten Aromaten, wie z. B. 4-Anisidin geschieht dies innerhalb von wenigen Minuten, bei desaktivierten Aromaten innerhalb von bis zu 72 h. Die so gebildete Suspension wird daraufhin mit weiteren 400 mL Methanol verdünnt und mit 10,7 g Ammoniumchlorid (0,2 mol) und 16 mL 37%iger Formaldehydlösung (17,40 g, 0,21 mol) versetzt. Anschließend wird für die Dauer von einer Stunde unter Rückfluss erhitzt. Über einen Tropftrichter werden dann in 10 min 14 mL 85%ige Phosphorsäure zugesetzt und im Anschluss wird die Reaktionsmischung für weitere 4-8 Stunden erhitzt.

Nach der Reaktion werden etwa 80% des Lösungsmittels am Rotationsverdampfer entfernt und 300 mL Eiswasser zur Reaktionsmischung zugegeben, bevor die Reaktionsmischung mit Kaliumhydroxidlösung (40% in Wasser) auf pH= 9 gebracht wird. Das Produkt wird nun drei Mal mit je 300 mL Dichlormethan extrahiert, die vereinigten organischen Phasen über Magnesiumsulfat getrocknet und das Lösungsmittel am Rotationsverdampfer entfernt. Zur Aufreinigung wird das Produkt entweder im Hochvakuum mit der Kugelrohrdestille destilliert, oder aus 10 mL heißem Essigester umkristallisiert.

### Synthesevorschrift für 1-(2-Ethoxyphenyl)imidazol (Referenzbeispiel)

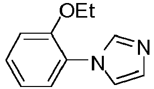

Diese Synthese wird im Gegensatz zur allgemeinen Synthesevorschrift in Ethanol anstelle von Methanol durchgeführt, um eine Umetherung des o-Phenetidins ausschließen zu können. Es werden 13,718 g (0,1 mol) o-Phenetidin eingesetzt. Ein gelber Niederschlag bildet sich sofort nach Zugabe der Glyoxallösung zur Lösung des o-Phenetidins in Ethanol. Nach der Reaktion wird das Produkt durch eine Destillation im Hochvakuum in der Kugelrohrdestille gereinigt und als gelbes Öl erhalten.
**Summenformel**: C₁₁H₁₂N₂O (188,23 g/mol)
**Ausbeute:** 12,58 g (66,8%)
**¹H-NMR (300 MHz, CDCl₃, ppm):**
δ=1.29 (t, J=6,9 Hz, 3 H, C*H*₃), 4.10 (q, J=6,9 Hz, 2 H, OC*H₂*)*,* 7.03 (d, J= 7,6 Hz, 1 H, arom. C3H), 7.07 (s, 1 H, NC*H*CHN), 7.20 (d, J=8,0, C6H), 7.31-7.40 (m, 2 H, arom. CH), 7.54 (s, 1 H, NCHC*H*N), 7.95 (s, 1 H, NC*H*N).
**¹³C-NMR (75,5 MHz, CDCl₃, ppm):**
δ=11.3 (CH₃), 64.1 (O*C*H₂), 113.7 (arom. C3H), 120.3 (arom. C6H), 120.8 (NCHCHN), 125.2 (arom. C5H), 126.1 (arom *C*1), 128.2 (NCHCHN), 128.7 (arom. C4H), 137.5 (NCHN), 151.1 (arom. C2).

### Synthesevorschrift für 1-(4-Ethoxyphenyl)imidazol (Referenzbeispiel)

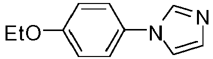

Diese Synthese wird im Gegensatz zur allgemeinen Synthesevorschrift in Ethanol anstelle von Methanol durchgeführt, um eine Umetherung des *p*-Phenetidins ausschließen zu können. Es werden 13,718 g (0,1 mol) *p*-Phenetidin eingesetzt. Ein gelber Niederschlag bildet sich sofort nach der Zugabe der Glyoxallösung zur Lösung des *p*-Phenetidins in Ethanol. Nach der Reaktion wird das Produkt durch eine Destillation im Hochvakuum in der Kugelrohrdestille gereinigt und als oranger Feststoff erhalten.
**Summenformel:** C₁₁H₁₂N₂O (188,23 g/mol)
**Ausbeute:** 13,91 g (73,9%)
**¹H-NMR (300 MHz, CDCl₃, ppm):**
δ=1.44 (t, J=7,0 Hz, 3 H, C*H*₃), 4.05 (q, J=7,0 Hz, 2 H, OC*H₂*)*,* 6.98 (d, J= 8,9 Hz, 2 H, arom C*H*), 7.27 (s, 1 H, NC*H*CHN), 7.30 (s, 1 H, NCHC*H*N), 7.76 (s, 1 H, NC*H*N).
**¹³C-NMR (75,5 MHz, CDCl₃, ppm):**
δ=14.7 (*C*H₃), 63.8 (O*C*H₂), 115.3 (arom. *C*3H und *C*5H), 118.6 (N*C*HCHN), 123.1 (arom. *C*2H und C6H), 130.0 (NCH*C*HN), 130.5 (arom *C*1), 135.8 (N*C*HN), 158.2 (arom. *C4).*

### Synthesevorschrift für 1-(2-Methoxyphenyl)imidazol (Referenzbeispiel)

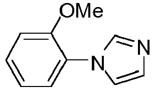

Es werden 24,63 g (0,2 mol) o-Anisidin eingesetzt. Ein gelber Niederschlag bildet sich einige Minuten nach der Zugabe der Glyoxallösung zur Lösung des o-Anisidins in Methanol. Nach der Reaktion wird das Produkt durch eine Destillation im Hochvakuum in der Kugelrohrdestille gereinigt und als oranges Öl erhalten.
**Summenformel:** C₁₀H₁₀N₂O (174,20 g/mol)
**Ausbeute:** 29,59 g (84,9%)
**¹H-NMR (300 MHz, CDCl₃, ppm):**
δ=3.85 (s, 3 H, OC*H*₃), 7.01 (s, 1 H, NC*H*CHN), 7.06 (d, J= 7,8 Hz, 1 H, arom C3*H*), 7.20 (d, J=7,8 Hz, 1 H, C6*H*), 7.38 (s, 1 H, NCHC*H*N), 7.42 (m, 2 H, arom. C*H*), 7.89 (s, 1 H, NC*H*N).
**¹³C-NMR (75,5 MHz, CDCl₃, ppm):**
δ=55.9 (O*C*H₃), 112.8 (arom. *C3*H), 120.5 (N*C*HCHN), 120.9 (arom. *C*6H), 125.3 (arom. *C*5H), 126.0 (arom C1), 128.3 (NCH*C*HN), 128.9 (arom. C4H), 137.6 (N*C*HN), 152.1 (arom. C2).

### Synthesevorschrift für 1-(4-Methoxyphenyl)imidazol (Referenzbeispiel)

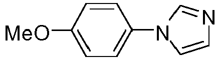

Es werden 24,6 g (0,2 mol) *p*-Anisidin eingesetzt. Ein gelber Niederschlag bildet sich einige Minuten nach der Zugabe der Glyoxallösung zur Lösung des *p*-Anisidins in Methanol. Nach der Reaktion wird das Produkt durch eine Destillation im Hochvakuum in der Kugelrohrdestille gereinigt und als oranger Feststoff erhalten.
**Summenformel:** C₁₀H₁₀N₂O (174,20 g/mol)
**Ausbeute:** 31,90 g (91,7%)
**¹H-NMR (300 MHz, d₆-DMSO, ppm):**
δ=3.79 (s, 3 H, OC*H₃*), 7.05 (d, J=9,0 Hz, 2 H, arom. C*H*), 7.10 (s, 1 H, NC*H*CHN), 7.55 (d, J=9,0 Hz, 2 H, arom. C*H*), 7.61 (s, 1 H, NCHC*H*N), 8.12 (s, 1 H, NC*H*N).
**¹³C-NMR (75,5 MHz, d₆-DMSO, ppm):**
δ=55.3 (O*C*H₃), 114.9 *(C3, C5* von C₆H₄OMe), 118.3 (N*C*HCHN), 122.0 (*C2, C6* von C₆H₄OMe), 129.5 (NCH*C*HN), 130.3 (arom. *C1*), 135.5 (N*C*N), 159.9 (*C*OMe).

### Synthesevorschrift für 1-(p-Methylphenyl)imidazol (Referenzbeispiel)

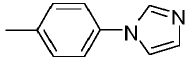

Nach der allgemeinen Synthesevorschrift werden 10,716 g (0,1 mol) *p*-Toluidin eingesetzt. Bereits nach etwa 10 min des Rührens mit der Glyoxallösung in 50 mL Methanol bildet sich ein gelber Nierderschlag. Nach der Reaktion und Destillation in der Kugelrohrdestille erhält man das Produkt als gelbes Öl.
**Summenformel:** C₁₀H₁₀N₂ (158,20 g/mol)
**Ausbeute:** 9,99 g (63,2%)
**¹H-NMR (300 MHz, d₆-DMSO, ppm):**
δ=2.32 (s, 3 H, C*H*₃), 7.10 (s, 1 H, NC*H*CHN), 7.29 (d, J=8,3 Hz, 2 H, arom. C2*H* und C6*H*), 7.61 (d, J=8,3 Hz, 2 H, arom. C3*H* und C5*H*), 7.68 (s, 1 H, NCHC*H*N), 8.21 (s, 1 H, NC*H*N).
**¹³C-NMR (75,5 MHz, d₆-DMSO, ppm):**
δ=20.2 (*C*H₃), 117.7 (NCHCHN), 120.0 (arom. *C*2H und *C*6H), 129.1 (NCH*C*HN), 129.9 (arom. *C*3H und *C*5H), 134.4 (arom. C1), 135.2 (N*C*HN), 136.0 (arom. *C*5).
**Elementaranalyse:** C₁₀H₁₀N₂ * 0,35 H₂O ber.: C 73,01%, H 6,56%, N 17,03%, gef.: C 73,21%, H 6,55%, N 16,55%.

### Synthesevorschrift für 1-(2,4-Dimethylphenyl)imidazol (Referenzbeispiel)

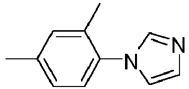

Nach der allgemeinen Synthesevorschrift werden 0,2 mol 2,4-Dimethylanilin eingesetzt. Nach etwa einer Stunde des Rührens mit der Glyoxallösung in 150 mL Methanol bildet sich ein gelber Niederschlag. Nach der Reaktion und Destillation in der Kugelrohrdestille erhält man das Produkt als gelbes Öl.
**Summenformel:** C₁₁H₁₂N₂ (172,23 g/mol)
**Ausbeute:** 8,32 g (24,2%)
**¹H-NMR (300 MHz, CDCl₃, ppm):**
δ=2.32 (s, 3 H, *o*-C*H*₃), 2.42 (s, 3 H, *p*-C*H₃*) 7.08 (s, 1 H, NC*H*CHN), 7.11 (s, 2 H, arom. C5*H* und C6*H*), 7.18 (s, arom. C3*H*), 7.20 (s, 1 H, NCHC*H*N), 7.55 (s, 1 H, NC*H*N).
**¹³C-NMR (75,5 MHz, d₆-DMSO, ppm):**
δ=17.6 (o-*C*H₃), 21.0 (*p-C*H₃)*,* 120.6 (N*C*HCHN), 126.3 (arom. *C*H), 127.4 (arom. *C*H), 129.2 (NCH*C*HN), 130.7 (arom. *C*H), 133.0 (arom. *C*2), 134.0 (arom. *C1*)*,* 137.1 (N*C*HN), 138.2 (arom. C4).

### Synthesevorschrift für 1-(2,4,6-Trimethylphenyl)imidazol (Referenzbeispiel)

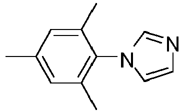

Entsprechend der allgemeinen Arbeitsvorschrift werden 13,521 g (0,1 mol) Mesitylamin eingesetzt. Das Produkt wird nach der Reaktion und zweifacher Umkristallisation als hellbrauner kristalliner Feststoff gewonnen.
**Summenformel:** C₁₂H₁₄N₂ (186,12 g/mol)
**Ausbeute:** 12,10 g (65,0%)
**¹H-NMR (300 MHz, CDCl₃, ppm):**
δ=1.91 (s, 6 H, *o*-C*H₃*), 2.26 (s, 3 H, *p-*C*H₃*)*,* 6.81 (s, 1 H, NC*H*CHN), 6.89 (s, 2 H, arom. C*H*), 7.15 (s, 1 H, NCHC*H*N), 7.37 (s, 1 H, NC*H*N).
**¹³C-NMR (75,5 MHz, CDCl₃, ppm):**
δ=17.2 (*o*-*C*H₃), 20.9 (*p-C*H₃)*,* 120.0 (N*C*HCHN), 128.9 (arom. *C*H), 129.3 (NCH*C*HN), 133.3 (arom. *o-C*H), 135.3 (arom. *C*1), 137.3 (N*C*HN), 138.8 (arom. *C*4).
**Elementaranalyse:** C₁₂H₁₄N₂ ber.: C 77,38%, H 7,58%, N 15,04%, gef.: C 77,31%, H 7,54%, N 15,08%.

### Synthesevorschrift für 1-(4-Ethylcarboxyphenyl)imidazol (Referenzbeispiel)

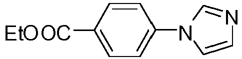

Um während der Reaktion eine Umesterung des Ethylesters zu vermeiden, wurde diese Reaktion in Ethanol, anstelle von Methanol als Lösungsmittel durchgeführt. Entsprechend der allgemeinen Synthesevorschrift werden für die Reaktion 16,519 g (0,1 mol) 4-Aminobenzoesäureethylester benötigt. Das Produkt wird am Ende der Reaktion durch Umkristallisation aus 10 mL Ethylacetat gereinigt und als dunkelroter Feststoff erhalten.
**Summenformel:** C₁₂H₁₂N₂O₂ (216,14 g/mol)
**Ausbeute:** 15,93g (73,7%)
**¹H-NMR (300 MHz, CDCl₃, ppm):**
δ=1.37 (t, J=7,1 Hz, 3 H, C*H*₃), 4.36 (q, 2 H, J=7,1 Hz, C*H₂*)*,* 7.18 (s, 1 H, NC*H*CHN), 7.31 (s, 1 H, NCHC*H*N), 7.42 (d, J=8,7 Hz, 2 H, arom. C2*H* und C6*H*), 7.90 (s, 1 H, NC*H*N), 8.11 (d, J=8,7 Hz, 2 H, arom. C3*H* und C5*H*).
**¹³C-NMR (75,5 MHz, CDCl₃, ppm):**
δ= 14.2 (*C*H₃), 61.1 (*C*H₂), 117.6 (N*C*HCHN), 120.4 (arom. *C*3H und *C*5H), 129.2 (arom. *C*1), 130.9 (NCH*C*HN), 131.2 (arom. *C*2H und *C*6H), 135.3 (N*C*HN), 140.5 (arom. *C*4), 165.3 (*C*OO).
**Elementaranalyse:** C₁₂H₁₂N₂O₂ ber.: C 66,65%, H 5,59%, N 12,96%, gef.: C 66,42%, H 5,54%, N 12,57%.

### Synthesevorschrift für 1-(4-Chlorphenyl)imidazol (Referenzbeispiel)

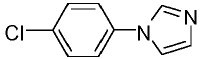

Entsprechend der allgemeinen Arbeitsvorschrift werden 12,758 g (0,1 mol) 4-Chloranilin benötigt. Nach der Reaktion wird das Produkt durch Umkristallisation mit 7 mL Ethylacetat gereinigt und als brauner Feststoff erhalten.
**Summenformel:** C₉H₇N₂Cl (178,62 g/mol)
**Ausbeute:** 13,78g (77,2%)
**¹H-NMR (300 MHz, CDCl₃, ppm):**
δ=7.19 (s, 1 H, NC*H*CHN), 7.23 (s, 1 H, NCHC*H*N), 7.31 (d, J=9,2 Hz, 2 H, arom. C3*H* und C5*H*), 7.43 (d, 2 H, J=9,2 Hz, arom. C2*H* und C6*H*), 7.81 (s, 1 H, NC*H*N).
**¹³C-NMR (75,5 MHz, CDCl₃, ppm):**
δ=118.2 (NCHCHN), 122.6 (arom. C2H und C6H), 130.0 (arom. *C*3H und *C*5H), 130.7 (NCHCHN), 133.2 (arom. *C*1), 135.5 (N*C*HN), 135.8 (arom. *C*4).
**Elementaranalyse:** C₉H₇N₂Cl ber.: C 60,52%, H 3,95%, N 15,68%, gef.: C 60,63%, H 3,84%, N 15,56%.

### Synthesevorschrift für 1-(4-Bromphenyl)imidazol (Referenzbeispiel)

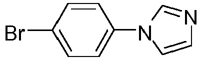

Entsprechend der allgemeinen Arbeitsvorschrift werden 17,20 g (0,1 mol) 4-Bromanilin benötigt. Nach der Reaktion wird das Produkt durch Umkristallisation mit 7 mL Ethylacetat gereinigt und als brauner Feststoff erhalten.
**Summenformel:** C₉H₇N₂Br (223,07 g/mol)
**Ausbeute:** 13,54 g (60,7%)
**¹H-NMR (300 MHz, CDCl₃, ppm):**
δ=7.19 (s, 1 H, NCHCHN), 7.23 (s, 1 H, NCHC*H*N), 7.25 (d, J=9,0 Hz, 2 H, arom. C*H*), 7.59 (d, 2 H, J=9,0 Hz, arom. C*H*), 7.88 (s, 1 H, NC*H*N).
**¹³C-NMR (75,5 MHz, CDCl₃, ppm):**
δ=118.1 (NCH*C*HN), 121.1 (arom. *C4*H), 122.9 (arom. *C*H), 130.3 (N*C*HCHN), 133.0 (arom. *C*H), 135.5 (NC*H*N), 136.2 (arom. *C1*).
**Elementaranalyse:** C₉H₇N₂Br ber.: C 48,46%, H 3,16%, N 12,56%, gef.: C 48,67%, H 3,16%, N 12,43%.

### Synthesevorschrift für 1-(4-Nitrophenyl)imidazol (Referenzbeispiel)

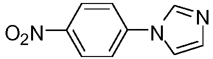

Entsprechend der allgemeinen Arbeitsvorschrift werden 17,20 g (0,2 mol) 4-Nitroanilin benötigt. Nach der Reaktion wird das Produkt durch Umkristallisation mit 20 mL Ethylacetat gereinigt und als gelber Feststoff erhalten.
**Summenformel:** C₉H₇N₃O₂ (189,17 g/mol)
**Ausbeute:** 18,54 g (49,0%)
**¹H-NMR (300 MHz, CDCl₃, ppm):**
δ=7.26 (s, 1 H, NC*H*CHN); 7.37 (s, 1 H, NCHC*H*N); 7.57 (d, J=8,8 Hz, 2 H, arom. C*H*); 7.98 (s, 1 H, NC*H*N); 8.36 (d, J=8.8 Hz, 2 H, arom. C*H*).
**¹³C-NMR (75,5 MHz, CDCl₃, ppm):**
δ=117.7 (NCH*C*HN); 121.1 (*C3, C5* von C₆H₄NO₂); 125.8 (*C2, C6* von C₆H₄NO₂); 131.7 (N*C*HCHN); 135.6 (N*C*HN); 142.0 (arom. *C1*); 146.3 (arom. *C4).*
**Elementaranalyse:** C₉H₇N₃O₂ ber.: C 57,14%, H 3,72%, N 22,21%, gef.: C 56,94%, H 3,64%, N 21,75%.

### Synthesevorschrift für 1-(3,5-Di-(trifluormethyl)phenyl)imidazol (Referenzbeispiel)

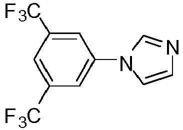

Nach der allgemeinen Synthesevorschrift werden 45,8 g (0,2 mol) 3,5-Di-(trifluormethyl)anilin eingesetzt. Nach 48 h des Rührens mit der Glyoxallösung in 80 mL Methanol bildet sich ein weißer Niederschlag, welcher sich beim Kochen unter Rückfluss wieder löst. Nach der Reaktion und Umkristallisation aus Essigester erhält man das Produkt als weißes kristallines Pulver.
**Summenformel:** C₁₁H₆F₆N₂ (280,17 g/mol)
**Ausbeute:** 20,70 g (37,0%)
**¹H-NMR (300 MHz, CDCl₃, ppm):**
δ=7.30 (s, 1 H, NC*H*CHN), 7.37 (s, 1 H, NCHC*H*N), 7.86 (s, 2 H, arom. *o*-C*H*), 7.89 (s, 1 H, arom. *p*-C*H*), 7.96 (s, 1 H, NC*H*N).
**¹³C-NMR (75,5 MHz, CDCl₃, ppm):**
δ=118.0 (N*C*HCHN), 120.9 (m, arom. p*-C*H), 121.0 (arom o-*C*H), 122.6 (q, ¹J_{C-F}=246 Hz, *C*F₃), 131.8 (NCHCHN), 133.7 (q, ²J_{C-F}=31 Hz, arom *m*-*C*), 135.5 (N*C*HN), 138.6 (arom. *C1*)*.*
**¹⁹F-NMR (282,4 MHz, CDCl₃, ppm):**
δ=-61,20 (C*F₃*).
**Elementaranalyse** : C₁₁H₆F₆N₂ ber.: C 47,16%, H 2,16%, N 10,00%, gef.: C 47,15%, H 2,06%, N 9,99%.

### Allgemeine Synthesevorschrift für Aryl-Alkyl-substituierte Imidazoliumbromid-Salze

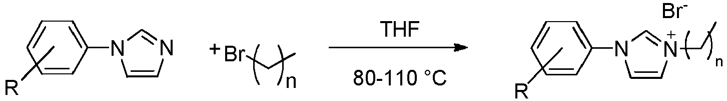

In einem ACE Druckrohr werden 1,0 eq. 1-*N*-substituiertes Imidazol in 10 mL THF gelöst und anschließend werden 1,1 eq. 1-Bromalkan zugegeben. Das ACE Druckrohr wird verschlossen, man erwärmt die Reaktionsmischung unter ständigem Rühren auf 80-110 °C und rührt bei dieser Temperatur für 8-10 Stunden. Anschließend wird das ausgefallene Produkt abgetrennt, mehrfach mit THF gewaschen und im Vakuum getrocknet.

### Ausführungsbeispiel 1

### 3-Butyl-1-mesitylimidazoliumbromid

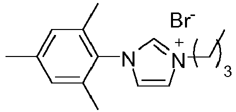

Es werden nach der allgemeinen Synthesevorschrift 5,37 mmol (0,883 g) Mesitylimidazol und 6,44 mmol (0,883 g, 0,70 mL) 1-Brombutan in 5 mL THF gelöst und für 19 h auf 100 °C erhitzt.
**Summenformel:** C₁₆H₂₃BrN₂ (323,27 g/mol)
**Ausbeute:** 0,275 g (86,8%)
**Schmelzpunkt:** 174 °C
**¹H-NMR (500 MHz, d₆-DMSO, ppm):**
δ=0.91 (t, J=7,4 Hz, 3 H, CH₂C*H*₃), 1.27 (tq, J=7,4 Hz, J=7,4 Hz, 2 H, C*H₂*CH₃), 1.88 (p, J=7.4Hz, 2 H, NCH₂CH₂), 2.01 (s, 6 H, arom. *o-*C*H₃*)*,* 2.33 (s, 3 H, *p*-C*H*₃), 4.29 (t, J=7,1 Hz, 2 H, NC*H₂*CH₃), 7.15 (s, 2 H, arom. C*H*), 7.95 (s, 1 H, NC*H*CHN), 8.13 (s, 1 H, NCHC*H*N), 9.50 (s, 1 H, NC*H*N).
**¹³C-NMR (125,8 MHz, d₆-DMSO, ppm):**
δ=13.3 (CH₂*C*H₃), 16.8 (o-*C*H₃), 18.7 (*C*H₂CH₃), 20.6 (*p-C*H₃)*,* 31.0 (NCH₂*C*H₂), 49.0 (N*C*H₂CH₃), 123.2 (N*C*HCHN), 123.9 (NCH*C*HN), 129.3 (arom. *C*H), 131.1 (arom. *C1*), 134.3 (arom. *C*2 und *C*6), 137.3 (N*C*HN), 140.2 (arom. C4).
**Elementaranalyse:** C₁₆H₂₃BrN₂ ber.: C 59,45%, H 7,17%, N 8,67%, gef.: C 58,48%, H 7,25%, N 8,58%.

### Ausführungsbeispiel 2

### 1-Mesityl-3-pentylimidazoliumbromid

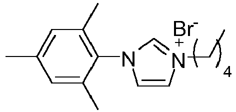

Es werden nach der allgemeinen Synthesevorschrift 2,68 mmol (0,500 g) Mesitylimidazol und 2,95 mmol (0,446 g, 0,40 mL) 1-Brompentan in 5 mL THF gelöst und für 12 h auf 80 °C erhitzt.
**Summenformel:** C₁₇H₂₅BrN₂ (337,30 g/mol)
**Ausbeute:** 0,861 g (95,1%)
**Schmelzpunkt:** 142 °C
**¹H-NMR (300 MHz, d₆-DMSO, ppm):**
δ=0.89 (t, J=7,3 Hz, 3 H, CH₂C*H*₃), 1.25-1.40 (m, 4 H, C*H₂*C*H₂*)*,* 1.92 (p, J=7,3 Hz, 2 H, NCH₂C*H*₂), 1.96 (s, 6 H, arom. *o*-C*H₃*)*,* 2.34 (s, 3 H, *p*-C*H₃*), 4.28 (t, J=7,3 Hz, 2 H, NC*H₂)*, 7.16 (s, 2 H, arom. C*H*), 7.94 (s, 1 H, NC*H*CHN), 8.12 (s, 1 H, NCHC*H*N), 9.48 (s, 1 H, NC*H*N).
**¹³C-NMR (75,5 MHz, d₆-DMSO, ppm):**
δ=13.8 (CH₂CH₃), 16.9 (*o-C*H₃), 20.6 (*p-C*H₃)*,* 21.4 (Alkyl-*C*H₂), 27.6 (Alkyl-*C*H₂), 28.7 (NCH₂*C*H₂), 49.3 (N*C*H₂CH₃), 121.2 (N*C*HCHN), 123.9 (NCH*C*HN) 129.3 (arom. *C*H), 131.2 (arom. *C1*)*,* 134.3 (arom. *C*2 und *C*6), 137.3 (N*C*HN), 140.3 (arom. C4).
**Elementaranalyse:** C₁₇H₂₅BrN₂ ber.: C 60,54%, H 7,47%, N 8,31%, gef.: C 60,67%, H 7,65%, N 8,21 %.

### Ausführungsbeispiel 3

### 3-Hexyl-1-mesitylimidazoliumbromid

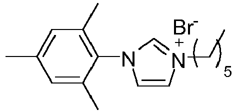

Es werden nach der allgemeinen Synthesevorschrift 1,62 mmol (0,301 g) Mesitylimidazol und 1,78 mmol (0,294 g, 0,25 mL) 1-Bromhexan in 5 mL THF gelöst und für 12 h auf 80 °C erhitzt.
**Summenformel:** C₁₈H₂₇BrN₂ (351,32 g/mol)
**Ausbeute:** 0,538 g (94,5%)
**Schmelzpunkt:** 114 °C
**¹H-NMR (300 MHz, d₆-DMSO, ppm):**
δ=0.93 (t, J=6,8 Hz, 3 H, CH₂C*H₃*), 1.29-1.41 (m, 6 H, C*H₂*C*H₂*C*H₂*)*,* 2.08 (p, J=6,9 Hz, 2 H, NCH₂C*H*₂), 2.07 (s, 6 H, arom. *o-*C*H₃*)*,* 2.39 (s, 3 H, *p*-C*H*₃), 4.33 (t, J=7,0 Hz, 2 H, NC*H₂*)*,* 7.21 (s, 2 H, arom. C*H*), 8.00 (s, 1 H, NC*H*CHN), 8.17 (s, 1 H, NCHC*H*N), 9.51 (s, 1 H, NC*H*N).
**¹³C-NMR (75,5 MHz, d₆-DMSO, ppm):**
δ=13.7 (CH₂*C*H₃), 16.8 (*o*-*C*H₃), 20.6 (*p-C*H₃)*,* 21.9 (Alkyl-*C*H₂), 25.0 (Alkyl-*C*H₂), 29.0 (Alkyl-*C*H₂), 30.4 (NCH₂*C*H₂), 49.3 (N*C*H₂CH₃), 121.2 (N*C*HCHN), 124.0 (NCH*C*HN) 129.3 (arom. *C*H), 131.2 (arom. *C1*), 134.3 (arom. *C*2 und *C*6), 137.3 (N*C*HN), 140.3 (arom. C4).
**Elementaranalyse:** C₁₈H₂₇BrN₂ ber.: C 61,54%, H 7,75%, N 7,97%, gef.: C 61,45%, H 7,88%, N 7,98%.

### Ausführungsbeispiel 4

### 1-Mesity-3-octylimidazoliumbromid

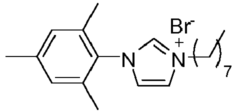

Es werden nach der allgemeinen Synthesevorschrift 5,40 mmol (1,00 g) Mesitylimidazol und 6,48 mmol (1,161 g, 1,02 mL) 1-Bromoctan in 5 mL THF gelöst und für 24 h auf 90 °C erhitzt.
**Summenformel:** C₂₀H₃₁BrN₂ (379,38 g/mol)
**Ausbeute:** 1,72 g (83,0%)
**Schmelzpunkt:** 96 °C
**¹H-NMR (300 MHz, d₆-DMSO, ppm):**
δ=0.85 (t, J=6,9 Hz, 3 H, CH₂C*H₃*), 1.27-1.41 (m, 10 H, Alkyl-C*H₂*),1.89 (p, J=6,6 Hz, 2 H, NCH₂C*H*₂), 2.02 (s, 6 H, arom. *o-*C*H₃*)*,* 2.33 (s, 3 H, *p*-C*H*₃), 4.29 (t, J=7,0 Hz, 2 H, N*CH₂*)*,* 7.16 (s, 2 H, arom. C*H*), 7.95 (s, 1 H, NC*H*CHN), 8.13 (s, 1 H, NCHC*H*N), 9.18 (s, 1 H, NC*H*N).
**¹³C-NMR (75,5 MHz, d₆-DMSO, ppm):**
δ=13.9 (CH₂*C*H₃), 16.8 (*o*-*C*H₃), 20.5 (*p-C*H₃)*,* 22.0 (C*H₂*CH₂CH₃), 25.4 (Alkyl-*C*H₂), 28.1 (Alkyl-*C*H₂), 28.4 (Alkyl-*C*H₂), 29.0 (Alkyl-*C*H₂), 31.0 (NCH₂*C*H₂), 49.3 (N*C*H₂), 123.1 (NCHCHN), 123.9 (NCH*C*HN) 129.2 (arom. *C*H), 131.1 (arom. *C1*), 134.2 (arom. *C*2 und *C*6), 137.2 (N*C*HN), 140.2 (arom. *C*4).
**Elementaranalyse:** C₂₀H₃₁BrN₂* 0,4 H₂O ber: C 62,14%, H 8,29%, N 7,25%, gef.: C 62,34%, H 8,50%, N 7,45%.

### Ausführungsbeispiel 5

### 1-Mesityl-3-undecylimidazoliumbromid

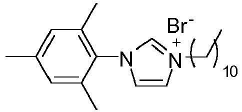

Es werden nach der allgemeinen Synthesevorschrift 5,40 mmol (1,00 g) Mesitylimidazol und 6,44 mmol (1,51 g, 1,44 mL) 1-Bromundecan in 5 mL THF gelöst und für 22,5 h auf 90 °C erhitzt. Anschließend wird das Produkt mit Diethylether ausgefällt.
**Summenformel:** C₂₃H₃₇BrN₂ (421,46 g/mol)
**Ausbeute:** 1,830 g (80,6%)
**Schmelzpunkt:** 63 °C
**¹H-NMR (300 MHz, d₆-DMSO, ppm):**
δ=0.91 (t, J=6,4 Hz, 3 H, CH₂C*H₃*), 1.29 (bs, 16 H, Alkyl-C*H₂*), 1.96 (m, 2 H, NCH₂C*H₂*), 2.06 (s, 6 H, arom. *o-*C*H₃*)*,* 2.36 (s, 3 H, *p*-C*H₃*), 4.33 (t, J=6,7 Hz, 2 H, NC*H*₂), 7.21 (s, 2 H, arom. C*H*), 8.00 (s, 1 H, NC*H*CHN), 8.16 (s, 1 H, NCHC*H*N), 9.50 (s, 1 H, NC*H*N).
**¹³C-NMR (75,5 MHz, d₆-DMSO, ppm):**
δ=13.9 (CH₂*C*H₃), 16.8 (*o*-*C*H₃), 20.6 (*p-C*H₃)*,* 22.1 (C*H*₂CH₃), 25.4 (Alkyl-*C*H₂), 28.2 (Alkyl-*C*H₂), 28.7 (Alkyl-*C*H₂), 28.8 (Alkyl-*C*H₂), 28.8 (Alkyl-*C*H₂), 28.9 (Alkyl-*C*H₂), 29.0 (Alkyl-*C*H₂), 31.3 (NCH₂*C*H₂), 49.3 (N*C*H₂), 123.2 (N*C*HCHN), 124.0 (NCH*C*HN), 129.2 (arom. *C*H), 131.1 (arom. *C1*), 134.3 (arom. *C*2 und *C*6), 137.2 (N*C*HN), 140.3 (arom. *C*4).
**Elementaranalyse:** C₂₃H₃₇BrN₂ ber: C 65,55%, H 8,85%, N 6,65%, gef.: C 65,53%, H 9,13%, N 6,31%.

### Ausführungsbeispiel 6

### 1-Mesityl-3-tetradecylimidazoliumbromid

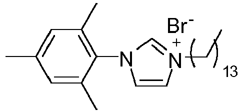

Es werden nach der allgemeinen Synthesevorschrift 5,40 mmol (1,00 g) Mesitylimidazol und 6,48 mmol (1,80 g, 1,77 mL) 1-Bromtetradecan in 5 mL THF gelöst und für 24 h auf 90 °C erhitzt.
**Summenformel:** C₂₆H₄₃BrN₂ (463,54 g/mol)
**Ausbeute:** 1,990 g (79,6%)
**Schmelzpunkt:** 85 °C
**¹H-NMR (300 MHz, d₆-DMSO, ppm):**
δ=0.83 (t, J=6,7 Hz, 3 H, CH₂C*H*₃), 1.23 (bs, 22 H, Alkyl-C*H*₂), 1.90 (p, J=7,0 Hz, 2 H, NCH₂C*H*₂), 2.01 (s, 6 H, arom. *o*-C*H₃*)*,* 2.33 (s, 3 H, *p*-C*H*₃), 4.27 (t, J=7,0 Hz, 2 H, NC*H₂*)*,* 7.15 (s, 2 H, arom. C*H*), 7.94 (s, 1 H, NC*H*CHN), 8.12 (s, 1 H, NCHC*H*N), 9.47 (s, 1 H, NC*H*N).
**¹³C-NMR (75,5 MHz, d₆-DMSO, ppm):**
δ=13.9 (CH₂*C*H₃), 16.8 (*o*-*C*H₃), 20.5 (*p-C*H₃)*,* 22.1 (C*H₂*CH₃), 25.3(Alkyl-*C*H₂), 28.2 (Alkyl-*C*H₂), 28.7 (Alkyl-*C*H₂), 28.8, 28.8, 28.8, 28.9, 28.9, 28.9, 28.9, 29.0 (Alkyl-*C*H₂), 31.3 (NCH₂*C*H₂), 49.3 (N*C*H₂), 123.1 (N*C*HCHN), 123.9 (NCH*C*HN) 129.2 (arom. *C*H), 131.1 (arom. *C1*)*,* 134.2 (arom. *C*2 und *C*6), 137.2 (NCHN), 140.2 (arom. *C*4).
**Elementaranalyse:** C₂₆H₄₃BrN₂ ber: C 67,37%, H 9,35%, N 6,04%, gef.: C 67,35%, H 9,39%, N 6,13%.

### Ausführungsbeispiel 7

### 1-Isopentyl-3-mesitylimidazoliumbromid

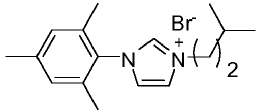

Es werden nach der allgemeinen Synthesevorschrift 2,68 mmol (0,500 g) Mesitylimidazol und 3,22 mmol (0,490 g, 0,34 mL) Isopentylbromid in 5 mL THF gelöst und für 11 h auf 100 °C erhitzt.
**Summenformel:** C₁₇H₂₅BrN₂ (337,30 g/mol)
**Ausbeute:** 0,693 g (76,6%)
**Schmelzpunkt:** 136 °C
**¹H-NMR (300 MHz, d₆-DMSO, ppm):**
δ=0.94 (d, J=6,6 Hz, 6 H, CH(C*H*₃)₂), 1.25-1.40 (m, 1 H, C*H*(CH₃)₂), 1.86 (pseudo q, J=7,6 Hz, 2 H, NCH₂C*H*₂), 2.01 (s, 6 H, arom. *o-*C*H₃*)*,* 2.33 (s, 3 H, *p*-C*H₃*), 4.28 (t, J=7,5 Hz, 2 H, NC*H₂*)*,* 7.15 (s, 2 H, arom. C*H*), 7.95 (s, 1 H, NC*H*CHN), 8.15 (s, 1 H, NCHC*H*N), 9.51 (s, 1 H, NC*H*N).
**¹³C-NMR (75,5 MHz, d₆-DMSO, ppm):**
δ=16.8 (*o*-*C*H₃), 20.6 (*p-C*H₃)*,* 22.0 (CH(*C*H₃)₂), 24.9 (*C*H(CH₃)₂), 37.7 (NCH₂*C*H₂), 47.7 (N*C*H₂), 123.1 (N*C*HCHN), 123.9 (NCH*C*HN) 129.2 (arom. *C*H), 131.1 (arom. *C1*), 134.2 (arom. *C*2 und *C*6), 137.2 (N*C*HN), 140.2 (arom. *C*4).
**Elementaranalyse:** C₁₇H₂₅BrN₂ * 0,35 H₂O ber: C 59,42%, H 7,54%, N 8,15%, gef.: C 59,44%, H 7,68%, N 8,26%.

### Ausführungsbeispiel 8

### 3-(2-Hydroxyethyl-)1-mesityl-imidazoliumbromid

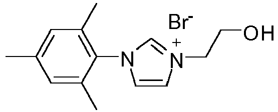

Es werden nach der allgemeinen Synthesevorschrift 5,4 mmol (1,00 g) Mesitylimidazol und 5,80 mmol (0,73 g) 2-Bromethanol in 5 mL THF gelöst und für 6 h auf 60 °C erhitzt.
**Summenformel:** C₁₄H₁₉BrN₂O (311,22 g/mol)
**Ausbeute:** 1,22 g (72,6%)
**Schmelzpunkt:** 164 °C
**¹H-NMR (500 MHz, d₆-DMSO, ppm):**
δ=1.99 (s, 6 H, arom. *o-*C*H₃*)*,* 2.33 (s, 3 H, *p*-C*H₃*), 3.80 (t, J=4,8 Hz, 2 H, HOC*H*₂CH₂), 4.34 (t, J=4,8 Hz, 2 H, NC*H₂*CH₂), 5.20 (bs, 1 *H,* OH), 7.15 (s, 2 H, arom. C*H*), 7.92 (s, 1 H, NC*H*CHN), 8.07 (s, 1 H, NCHC*H*N), 9.44 (s, 1 H, NC*H*N).
**¹³C-NMR (125,8 MHz, d₆-DMSO, ppm):**
δ=16.9 (*o*-*C*H₃), 20.6 (*p-C*H₃)*,* 52.0 (CH₂*C*H₂N), 59.0 (NCH₂*C*H₂), 123.4 (N*C*HCHN), 123.7 (NCHCHN), 129.2 (arom. *C*H), 131.2 (arom. *C1*)*,* 134.3 (arom. *C*2 und *C*6), 137.7 (N*C*HN), 140.1 (arom. *C*4).
**Elementaranalyse:** C₁₄H₁₉BrN₂O ber.: C 54,03%, H 6,15%, N 9,00%, gef.: C 53,81%, H 6,44%, N 8,70%.

### Ausführungsbeispiel 9

### 3-(2-Carboxyethyl)-1-mesityl-imidazoliumbromid

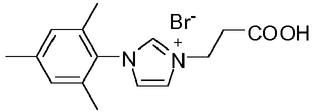

Es werden nach der allgemeinen Synthesevorschrift 5,4 mmol (1,00 g) Mesitylimidazol und 5,80 mmol (0,894 g) 3-Brompropionsäure in 5 mL THF gelöst und für 6 h auf 60 °C erhitzt.
**Summenformel:** C₁₅H₁₉BrN₂O₂ (339,23 g/mol)
**Ausbeute:** 1,06 g (55,9%)
**Schmelzpunkt:** 160 °C
**¹H-NMR (500 MHz, d₆-DMSO, ppm):**
δ=2.00 (s, 6 H, arom. *o*-C*H₃*)*,* 2.32 (s, 3 H, *p*-C*H₃*)*,* 3.08 (t, J=6,3 Hz, 2 H, C*H₂*C*H₂*), 4.50 (t, J=6,3 Hz, 2 H, NC*H*₂CH₂), 7.15 (s, 2 H, arom. C*H*), 7.94 (s, 1 H, NC*H*CHN), 8.17 (s, 1 H, NCHC*H*N), 9.56 (s, 1 H, NC*H*N), 12.60 (bs, 1 H, COO*H*).
**¹³C-NMR (125,8 MHz, d₆-DMSO, ppm):**
δ=16.9 (*o*-*C*H₃), 20.6 (*p-C*H₃)*,* 33.4 (*C*H₂CH₂N), 45.2 (N*C*H₂CH₂), 123.1 (N*C*HCHN), 123.8 (NCHCHN) 129.2 (arom. CH), 131.1 (arom. *C1*)*,* 134.3 (arom. *C*2 und *C*6), 138.0 (N*C*HN), 140.2 (arom. *C*4); 171.7 (COO).

### Ausführungsbeispiel 10

### 3-(2-Carboxyethyl)-1-(4-nitrophenyl)imidazoliumbromid

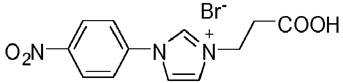

Es werden nach der allgemeinen Synthesevorschrift 0,756 mmol (0,250g) 1-(4-Nitrophenyl)imidazol und 0,756 mmol (0,115 g) 3-Brompropansäure in 5 mL THF gelöst und für 17 h auf 90 °C erhitzt.
**Summenformel:** C₁₂H₁₂BrN₃O₄ (342,15 g/mol)
**Ausbeute:** 0,186 g (71,9%)
**Schmelzpunkt:** 145 °C
**¹H-NMR (300 MHz, d₆-DMSO, ppm):**
δ=3.00 (t, J=7,6 Hz, 3 H, CH₂C*H*₂), 4.51 (t, J=7,6 Hz, 2 H, NCH₂C*H*₂), 4.25 (t, J=7,1 Hz, 2 H, NC*H₂*), 8.10 (d, J=9,0 Hz, 2 H, arom. C*H*), 8.15 (s, 1 H, NC*H*CHN), 8.48 (s, 1 H, NCHC*H*N), 8.54 (d, J=9,2 Hz, 2 H, arom. CH), 10.05 (s, 1 H, NC*H*N), 12.73 (bs, 1 H, COOH).
**¹³C**-**NMR (75,5 MHz, d₆-DMSO, ppm):**
δ=33.4 (CH₂*C*H₂), 45.4 (*C*H₂CH₂), 121.1 (N*C*HCHN), 120.8 (NCH*C*HN), 122.8 (arom. CH), 123.9 (N*C*HCHN), 125.5 (arom. *C*H), 136.6 (N*C*HN), 139.2 (arom. *C1*), 147.6 (arom. *C*4), 171.6 (*C*OOH).

### Ausführungsbeispiel 11

### 1-Mesitylimidazolium-3-propan-1-sulfonat

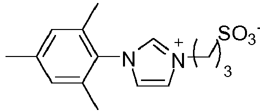

Es werden nach der allgemeinen Synthesevorschrift 5,4 mmol (1,0 g) Mesitylimidazol und 5,7 mmol (0,668 g) 1,3 Propansulton in 5 mL Aceton gelöst und für 5 Tage bei Raumtemperatur gerührt.
**Summenformel:** C₁₅H₂₀N₂O₃S (308,40 g/mol)
**Ausbeute:** 1,331 g (80,0%)
**Schmelzpunkt:** 329 °C (Zers.)
**¹H-NMR (300 MHz, d₆-DMSO, ppm):**
δ=2.03 (s, 6 H, arom. *o*-C*H₃*), 2.21 (tt, J=7,0 Hz, 2 H, NCH₂CH₂), 2.33 (s, 3 H, *p*-C*H₃*), 2.44 (t, J=7,0 Hz, 2 H, C*H₂*S), 4.42 (t, J=7,0 Hz, 2 H, NC*H₂*), 7.15 (s, 2 H, arom. C*H*), 7.93 (s, 1 H, NC*H*CHN), 8.13 (s, 1 H, NCHC*H*N), 9.40 (s, 1 H, NC*H*N).
**¹³C**-**NMR (75 MHz, d₆-DMSO, ppm):**
δ=16.9 (*o*-*C*H₃), 20.6 (*p-C*H₃), 26.0 (CH₂*C*H₂), 47.3 (S*C*H₂), 48.3 (N*C*H₂), 123.2 (N*C*HCHN), 123.8 (NCH*C*HN), 129.2 (arom. *C*H), 131.2 (arom. *C1*), 134.3 (arom. *C*2 und *C*6), 137.5 (N*C*HN), 140.2 (arom. *C*4).
**Elementaranalyse:** C₁₅H₂₀N₂O₃S ber.: C 58,42%, H 6,54%, N 9,08%, S 10,40% gef.: C 58,34%, H 6,43%, N 8,97%, S 10,56%.

### Ausführungsbeispiel 12

### 3-Propyl-1-(2,4-dimethylphenyl)-imidazoliumbromid

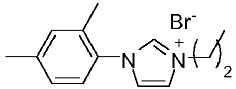

Es werden nach der allgemeinen Synthesevorschrift 2,91 mmol (0,500g) 1-(2,4-Dimethylphenyl)imidazol und 3,20 mmol (0,392 g) 1-Brompropan in 5 mL THF gelöst und für 6 h auf 120 °C erhitzt.
**Summenformel:** C₁₄H₁₉BrN₂ (295,22 g/mol)
**Ausbeute:** 1,270 g (75,1%)
**Schmelzpunkt:** 62 °C

### Ausführungsbeispiel 13

### 1-(2-Methoxyphenyl)-3-propylimidazoliumbromid

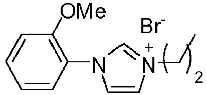

Es werden nach der allgemeinen Synthesevorschrift 4,48 mmol (0,700 g) 1-(2-Methoxyphenyl)imidazol und 4,93 mmol (0,606 g, 0,45 mL) 1-Brompropan in 5 mL THF gelöst und für 5 h auf 80 °C erhitzt.
**Summenformel:** C₁₃H₁₇BrN₂O (197,19 g/mol)
**Ausbeute:** 0,820 g (92,9%)
**Schmelzpunkt:** 126 °C
**¹H-NMR (300 MHz, d₆-DMSO, ppm):**
δ=0.92 (t, J=7,3 Hz, 3 H, CH₂*CH*₃), 1.91 (qt, J=7,3 Hz, 2 H, NCH₂C*H*₂), 3.89 (s, 3 H, OC*H*₃), 4.26 (t, J=7,1 Hz, 2 H, NC*H₂*), 7.18 (t, J=7,5 Hz, 1 H, arom. C*H*), 7.39 (d, J=8,2 Hz, 1 H, arom. C*H*), 7.51-7.70 (m, 2 H, arom. CH), 8.04 (s, 1 H, NC*H*CHN), 8.08 (s, 1 H, NCHC*H*N), 9.64 (s, 1 H, NC*H*N).
**¹³C**-**NMR (75,5 MHz, d₆-DMSO, ppm):**
δ=10.4 (CH₂*C*H₃), 22.7 (*C*H₂CH₃), 50.7 (N*C*H₂), 56.4 (OCH₃), 113.2 (arom. *C*3H), 121.1 (arom. *C*5H), 122.3 (N*C*HCHN), 123.4 (arom. *C*1), 123.8 (NCH*C*HN), 126.2 (arom. *C*6H), 131.6 (arom.*C*4H), 137.1 (N*C*HN), 152.1 (arom. *C*20).

### Ausführungsbeispiel 14

### 1-(4-Bromphenyl)-3-propylimidazoliumbromid

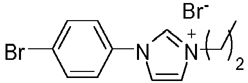

Es werden nach der allgemeinen Synthesevorschrift 4,48 mmol (1,00g) 1-(4-Bromphenyl)imidazol und 5,40 mmol (0,660 g, 0,49 mL) 1-Brompropan in 5 mL THF gelöst und für 20 h auf 90 °C erhitzt.
**Summenformel:** C₁₂H₁₄Br₂N₂ (343,95 g/mol)
**Ausbeute:** 1,06 g (68,4%)
**Schmelzpunkt:** 155 °C
**¹H-NMR (300 MHz, d₆-DMSO, ppm):**
δ=0.93 (t, J=7,4 Hz, 3 H, CH₂C*H*₃), 1.91 (qt, J=7,3 Hz, J=7,1 Hz, 2 H, NCH₂C*H*₂), 4.22 (t, J=7,1 Hz, 2 H, NC*H*₂), 7.78 (d, J=9,0 Hz, 2 H, arom. C*H*), 7.90 (d, J=9,1 Hz, 2 H, arom. CH), 8.07 (s, 1 H, NC*H*CHN), 8.35 (s, 1 H, NCHC*H*N), 9.90 (s, 1 H, NC*H*N).
**¹³C-NMR (75,5 MHz, d₆-DMSO, ppm):**
δ=10.5 (CH₂*C*H₃), 22.6 (NCH₂*C*H₂), 50.9 (N*C*H₂), 121.1 (N*C*HCHN), 122.6 (arom. *C*4), 123.3 (NCH*C*HN) 123.9 (arom. CH), 133.0 (arom. CH), 134.1 (arom. *C1*), 135.4 (N*C*HN).
**Elementaranalyse:** C₁₂H₁₄Br₂N₂ ber.: C 41,65%, H 4,08%, N 8,09%, gef.: C 41,36%, H 4,38%, N 8,07%.

### Ausführungsbeispiel 15

### 1-(4-Bromphenyl)-3-hexylimidazoliumbromid

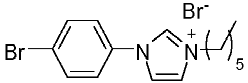

Es werden nach der allgemeinen Synthesevorschrift 10,4 mmol (2,32g) 1-(4-Bromphenyl)imidazol und 11,44 mmol (1,889 g, 2,17 mL) 1-Bromhexan in 5 mL THF gelöst und für 23 h auf 80 °C erhitzt.
**Summenformel:** C₁₅H₂₀Br₂N₂ (388,14 g/mol)
**Ausbeute:** 0,946 g (23,4%)
**Schmelzpunkt:** 121 °C
**¹H-NMR (300 MHz, d₆-DMSO, ppm):**
δ=0.87 (t, J=6,2 Hz, 3 H, CH₂C*H*₃), 1.31 (bs, 6 H, Alkyl-CH₂), 1.88 (m, 2 H, NCH₂C*H*₂), 4.23 (t, J=7,3 Hz, 2 H, NC*H*₂), 7.76 (d, J=9,0 Hz, 2 H, arom. C*H*), 7.89 (d, J=9,0 Hz, 2 H, arom. CH), 8.06 (s, 1 H, NC*H*CHN), 8.33 (s, 1 H, NCH*C*HN), 9.87 (s, 1 H, NC*H*N).
**¹³C-NMR (75,5 MHz, d₆-DMSO, ppm):**
δ=13.8 (CH₂CH₃), 21.9 (Alkyl-*C*H₂), 25.2 (Alkyl-CH₂), 29.0 (Alkyl-*C*H₂), 30.6 (NCH₂*C*H₂), 49.4 (N*C*H₂), 121.1 (N*C*HCHN), 122.6 (arom. C4), 123.4 (NCH*C*HN), 123.9 (arom. C*H*), 133.0 (arom. *C*H), 134.1 (arom. *C1*), 135.5 (N*C*HN).

### Ausführungsbeispiel 16

### 1-(4-Bromphenyl)-3-heptylimidazoliumbromid

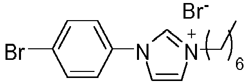

Es werden nach der allgemeinen Synthesevorschrift 1,62 mmol (0,361 g) 1-(4-Bromphenyl)imidazol und 1,78 mmol (0,319 g, 0,28 mL) 1-Bromheptan in 5 mL THF gelöst und für 2,5 h auf 100 °C erhitzt.
**Summenformel:** C₁₆H₂₂Br₂N₂ (402,17 g/mol)
**Ausbeute:** 0,484 g (74,1%)
**Schmelzpunkt:** 100 °C
**¹H-NMR (300 MHz, d₆-DMSO, ppm):**
δ=0.86 (t, J=6,8 Hz, 3 H, CH₂C*H*₃), 1.26-1.39 (m, 8 H, Alkyl-CH₂), 1.88 (m, 2 H, NCH₂C*H*₂), 4.24 (t, J=7,3 Hz, 2 H, NC*H₂*), 7.78 (d, J=8,9 Hz, 2 H, arom. C*H*), 7.90 (d, J=8,9 Hz, 2 H, arom. CH), 8.07 (s, 1 H, NC*H*CHN), 8.33 (s, 1 H, NCHC*H*N), 9.92 (s, 1 H, NC*H*N).
**¹³C**-**NMR (75,5 MHz, d₆-DMSO, ppm):**
δ=13.8 (CH₂*C*H₃), 21.8 (Alkyl-*C*H₂), 25.3 (Alkyl-CH₂), 27.9 (Alkyl-*C*H₂), 29.0 (Alkyl-*C*H₂), 30.9 (NCH₂*C*H₂), 49.3 (N*C*H₂), 120.9 (N*C*HCHN), 122.5 (arom. *C*4), 123.2 (NCH*C*HN), 123.8 (arom. *C*H), 132.9 (arom. CH), 133.9 (arom. *C1*), 135.4 (N*C*HN).
**Elementaranalyse:** C₁₆H₂₂Br₂N₂ ber.: C 47,78%, H 5,51%, N 6,91%, gef.: C 47,85%, H 5,60%, N7,11%.

### Ausführungsbeispiel 17

### 1-(4-Bromphenyl)-3-tetradecylimidazoliumbromid

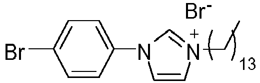

Es werden nach der allgemeinen Synthesevorschrift 4,5 mmol (1,00 g) 1-(4-Bromphenyl)imidazol und 5,4 mmol (1,50 g, 1,47 mL) 1-Bromtetradecan in 5 mL THF gelöst und für 69 h auf 90 °C erhitzt. Das Produkt wird nach der Reaktion mit Diethylether aus der Reaktionslösung ausgefällt.
**Summenformel:** C₂₃H₃₆Br₂N₂ (500,36 g/mol)
**Ausbeute:** 1,87 g (83,5%)
**Schmelzpunkt:** 75 °C
**¹H-NMR (300 MHz, d₆-DMSO, ppm):**
δ=0.86 (t, J=6,8 Hz, 3 H, CH₂C*H*₃), 1.20-1.35 (m, 22 H, Alkyl-CH₂), 1.87 (m, 2 H, NCH₂C*H*₂), 4.24 (t, J=7,2 Hz, 2 H, NC*H₂*), 7.77 (d, J=9,0 Hz, 2 H, arom. C*H*), 7.85 (d, J=8,9 Hz, 2 H, arom. CH), 8.06 (s, 1 H, NC*H*CHN), 8.34 (s, 1 H, NCHC*H*N), 9.87 (s, 1 H, NC*H*N).
**¹³C**-**NMR (75,5 MHz, d₆-DMSO, ppm):**
δ=13.9 (CH₂*C*H₃), 22.1 (Alkyl-*C*H₂), 25.5 (Alkyl-*C*H₂), 28.4 (Alkyl-*C*H₂), 28.7 (Alkyl-*C*H₂), 28.8 (Alkyl-*C*H₂), 28.9 (Alkyl-*C*H₂), 29.0 (Alkyl-*C*H₂), 29.0 (Alkyl-*C*H₂), 31.3 (NCH₂*C*H₂), 49.4 (N*C*H₂), 121.1 (N*C*HCHN), 122.6 (arom. *C*4), 123.3 (NCH*C*HN), 123.9 (arom. *C*H), 133.0 (arom. CH), 134.1 (arom. *C1*), 135.5 (N*C*HN).
**Elementaranalyse:** C₂₃H₃₆Br₂N₂ * 0,4 H₂O ber.: C 54,43%, H 7,91%, N 5,52%, gef.: C 54,33%, H 7,98%, N 5,60%.

### Ausführungsbeispiel 18

### 1-(4-Chlorphenyl)-3-propylimidazoliumbromid

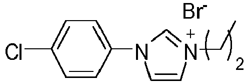

Es werden nach der allgemeinen Synthesevorschrift 5,60 mmol (1,00g) 1-(4-Chlorphenyl)imidazol und 6,7 mmol (0,830 g, 0,61 mL) 1-Brompropan in 5 mL THF gelöst und für 18,5 h auf 90 °C erhitzt.
**Summenformel:** C₁₂H₁₄BrClN₂ (301,61 g/mol)
**Ausbeute:** 1,310 g (77,5%)
**Schmelzpunkt:** 165 °C
**¹H-NMR (300 MHz, d₆-DMSO, ppm):**
δ=0.93 (t, J=7,4 Hz, 3 H, CH₂C*H*₃), 1.91 (qt, J=7,4 Hz, J=7,1 Hz, 2 H, NCH₂C*H*₂), 4.22 (t, J=7,1 Hz, 2 H, NC*H*₂), 7.77 (d, J=9,1 Hz, 2 H, arom. C*H*), 7.85 (d, J=9,1 Hz, 2 H, arom. CH), 8.07 (s, 1 H, NC*H*CHN), 8.35 (s, 1 H, NCHC*H*N), 9.85 (s, 1 H, NC*H*N).
**¹³C**-**NMR (75,5 MHz, d₆-DMSO, ppm):**
δ=10.5 (CH₂*C*H₃), 22.6 (NCH₂*C*H₂), 50.9 (N*C*H₂), 121.2 (N*C*HCHN), 123.3 (NCH*C*HN) 123.8 (arom. CH), 130.1 (arom. CH), 133.6 (arom. *C1*), 134.2 (arom. *C*4), 135.6 (N*C*HN).
**Elementaranalyse:** C₁₂H₁₄BrClN₂ ber.: C 47,79%, H 4,68% N 9,29%, gef.: C 47,63% H 4,43%, N 9,24%.

### Ausführungsbeispiel 19

### 1-(4-Chlorphenyl)-3-heptylimidazoliumbromid

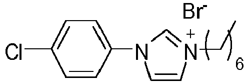

Es werden nach der allgemeinen Synthesevorschrift 5,6 mmol (1,00 g) 1-(4-Chlorphenyl)imidazol und 6,7 mmol (1,20 g, 1,1 mL) 1-Bromheptan in 5 mL THF gelöst und für 12 h auf 80 °C erhitzt.
**Summenformel:** C₁₆H₂₂BrClN₂ (357,72 g/mol)
**Ausbeute:** 1,54 g (77,0%)
**Schmelzpunkt:** 100 °C
**¹H-NMR (300 MHz, d₆-DMSO, ppm):**
δ=0.87 (t, J=6,5 Hz, 3 H, CH₂C*H*₃), 1.28-1.32 (m, 8 H, Alkyl-CH₂), 1.89 (m, 2 H, NCH₂C*H*₂), 4.23 (t, J=7,2 Hz, 2 H, NC*H*₂), 7.79 (d, J=7,3 Hz, 2 H, arom. C*H*), 7.85 (d, J=7,3 Hz, 2 H, arom. CH), 8.06 (s, 1 H, N*C*H*C*HN), 8.33 (s, 1 H, NCHC*H*N), 9.83 (s, 1 H, NC*H*N).
**¹³C**-**NMR (75,5 MHz, d₆-DMSO, ppm):**
δ=13.9 (CH₂CH₃), 22.0 (Alkyl-CH₂), 25.4 (Alkyl-*C*H₂), 28.0 (Alkyl-*C*H₂), 29.0 (Alkyl-*C*H₂), 31.0 (NCH₂*C*H₂), 49.4 (N*C*H₂), 121.1 (N*C*HCHN), 123.3 (NCH*C*HN), 123.7 (arom. *C*H), 130.1 (arom. CH), 133.7 (arom. *C*4), 134.2 (arom. *C1*), 135.6 (N*C*HN).
**Elementaranalyse:** C₁₆H₂₂BrClN₂ ber.: C 53,72%, H 6,20%, N 7,83%, gef.: C 53,53%, H 6,13%, N 7,80%.

### Ausführungsbeispiel 20

### 1-(4-Chlorphenyl)-3-tetradecylimidazoliumbromid

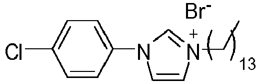

Es werden nach der allgemeinen Synthesevorschrift 5,6 mmol (1,00 g) 1-(4-Chlorphenyl)imidazol und 6,7 mmol (1,86 g, 1,82 mL) 1-Bromtetradecan in 5 mL THF gelöst und für 42 h auf 90 °C erhitzt. Am Ende der Reaktion wird die Reaktionsmischung auf Raumtemperatur abgekühlt und das Produkt mit Diethylether ausgefällt.
**Summenformel:** C₂₃H₃₆BrClN₂ (455,91 g/mol)
**Ausbeute:** 2,32 g (91,1%)
**Schmelzpunkt:** 58 °C
**¹H-NMR (300 MHz, d₆-DMSO, ppm):**
δ=0.86 (t, J=7,0 Hz, 3 H, CH₂C*H*₃), 1.24 (m, 22 H, Alkyl-CH₂), 1.88 (m, 2 H, NCH₂C*H*₂), 4.23 (t, J=7,2 Hz, 2 H, NC*H*₂), 7.78 (d, J=9,0 Hz, 2 H, arom. C*H*), 7.85 (d, J=9,1 Hz, 2 H, arom. CH), 8.05 (s, 1 H, NC*H*CHN), 8.34 (s, 1 H, NCHC*H*N), 9.83 (s, 1 H, NC*H*N).
**¹³C-NMR (75,5 MHz, d₆-DMSO, ppm):**
δ=13.9 (CH₂*C*H₃), 22.1 (Alkyl-*C*H₂), 25.5 (Alkyl-*C*H₂), 28.4 (Alkyl-*C*H₂), 28.7 (Alkyl-*C*H₂), 28.8 (Alkyl-*C*H₂), 28.9 (Alkyl-*C*H₂), 29.0 (Alkyl-*C*H₂), 29.0 (arom. *C*H₂), 31.3 (NCH₂*C*H₂), 49.4 (N*C*H₂), 121.2 (N*C*HCHN), 123.6 (NCH*C*HN), 123.7 (arom. *C*H), 130.1 (arom. CH), 133.6 (arom. *C*4), 134.2 (arom. *C1*), 135.6 (N*C*HN).
**Elementaranalyse:** C₂₃H₃₆BrClN₂ ber.: C 60,59%, H 7,96%, N 6,14%, gef.: C 60,24%, H 8,08%, N 6,09%.

### Ausführungsbeispiel 21

### 1-(4-Ethylcarboxyphenyl)-3-propylimidazoliumbromid

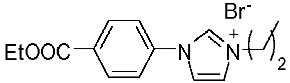

Es werden nach der allgemeinen Synthesevorschrift 4,60 mmol (1,00 g) 1-(4-Ethylcarboxyphenyl)imidazol und 5,50 mmol (0,680 g, 0,5 mL) 1-Brompropan in 5 mL THF gelöst und für 43 h auf 90 °C erhitzt.
**Summenformel:** C₁₅H₁₉BrN₂O₂ (339,23 g/mol)
**Ausbeute:** 0,838 g (52,4%)
**Schmelzpunkt:** 179 °C
**¹H-NMR (300 MHz, d₆-DMSO, ppm):**
δ=0.97 (t, J=7,3 Hz, 3 H, CH₂CH₂C*H*₃), 1.42 (t, J=7,3 Hz, 3 H, OCH₂C*H*₃), 1.99 (hept, J=7.3 Hz, 2 H, NCH₂C*H*₂), 4.25 (t, J=7,3 Hz, 2 H, NC*H₂*), 4.72 (q, J=7,1 Hz, 2 H, OC*H*₂), 8.00 (d, J=8,8 Hz, 2 H, arom. C*H*), 8.12 (s, 1 H, NC*H*CHN), 8.25 (d, J=8,8 Hz, 2 H, arom. CH), 8.48 (s, 1 H, NCHC*H*N), 10.02 (s, 1 H, NC*H*N)
**¹³C**-**NMR (75,5 MHz, d₆-DMSO, ppm):**
δ=10.5 (CH₂CH₂*C*H₃), 14.1 (OCH₂*C*H₃), 22.6 (Alkyl-*C*H₂), 51.0 (N*C*H₂), 61.3 (O*C*H₂), 121.0 (N*C*HCHN), 121.9 (arom. *C*H), 123.5 (NCH*C*HN), 130.7 (arom. *C1*), 131.0 (arom. *C*H), 135.8 (N*C*HN), 138.1 (arom. *C*4), 164.6 (COO).
**Elementaranalyse:** C₁₅H₁₉BrN₂O₂ * 0,35 H₂O ber.: C 52,14%, H 5,75%, N 8,11%, gef.: C 52,19%, H 5,79%, N 8,11%.

### Ausführungsbeispiel 22

### 1-(4-Ethylcarboxyphenyl)-3-hexylimidazoliumbromid

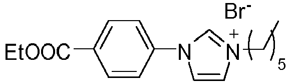

Es werden nach der allgemeinen Synthesevorschrift 1,62 mmol (0,350 g) 1-(4-Ethylcarboxyphenyl)imidazol und 1,78 mmol (0,294 g, 0,25 mL) 1-Bromhexan in 5 mL THF gelöst und für 2,5 h auf 100 °C erhitzt.
**Summenformel:** C₁₈H₂₅BrN₂O₂ (381,31 g/mol)
**Ausbeute:** 0,417 g (67,5%)
**Schmelzpunkt:** 115 °C
**¹H-NMR (300 MHz, d₆-DMSO, ppm):**
δ=0.87 (t, J=6,5 Hz, 3 H, CH₂CH₂C*H*₃), 1.31 (bs, 6 H, Alkyl-CH₂), 1.35 (t, J=7,1 Hz, 3 H, OCH₂C*H*₃), 1.89 (m, 2 H, NCH₂C*H*₂), 4.25 (t, J=7,3 Hz, 2 H, NC*H₂*), 4.36 (q, J=7,1 Hz, 2 H, OC*H₂*), 7.97 (d, J=8,7 Hz, 2 H, arom. C*H*), 8.09 (s, 1 H, NC*H*CHN), 8.20 (d, J=8,7 Hz, 2 H, arom. CH), 8.43 (s, 1 H, NCHC*H*N), 10.00 (s, 1 H, NC*H*N).
**¹³C**-**NMR (75,5 MHz, d₆-DMSO, ppm):**
δ=13.7 (CH₂CH₂*C*H₃), 14.0 (OCH₂*C*H₃), 21.7 (Alkyl-*C*H₂), 25.0 (Alkyl-*C*H₂), 28.9 (Alkyl-*C*H₂), 30.4 (NCH₂*C*H₂), 49.3 (N*C*H₂), 61.2 (O*C*H₂), 120.7 (NCHCHN), 121.8 (arom. CH), 123.4 (NCHCHN), 130.5 (arom. *C1),* 130.8 (arom. CH), 135.6 (NCHN), 138.0 (arom. *C*4), 164.5 (COO).

### Ausführungsbeispiel 23

### 1-(4-Ethylcarboxyphenyl)-3-heptylimidazoliumbromid

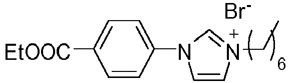

Es werden nach der allgemeinen Synthesevorschrift 4,60 mmol (1,00 g) 1-(4-Ethylcarboxyphenyl)imidazol und 5,50 mmol (0,990 g, 0,88 mL) 1-Bromheptan in 5 mL THF gelöst und für 70 h auf 90 °C erhitzt.
**Summenformel:** C₁₉H₂₇BrN₂O₂ (395,33 g/mol)
**Ausbeute:** 1,140 g (62,3%)
**Schmelzpunkt:** 114 °C
**¹H-NMR (300 MHz, d₆-DMSO, ppm):**
δ=0.86 (t, J=6,8 Hz, 3 H, CH₂CH₂*C*H₃), 1.27-1.35 (m, 8 H, Alkyl-CH₂), 1.36 (t, J=7,1 Hz, 3 H, OCH₂C*H*₃), 1.90 (m, 2 H, NCH₂C*H₂*), 4.25 (t, J=7,3 Hz, 2 H, NC*H*₂), 4.36 (q, J=7,1 Hz, 2 H, OC*H₂*), 7.99 (d, J=8,7 Hz, 2 H, arom. C*H*), 8.10 (s, 1 H, NC*H*CHN), 8.21 (d, J=8,7 Hz, 2 H, arom. C*H*), 8.45 (s, 1 H, NCHC*H*N), 10.03 (s, 1 H, NC*H*N).
**¹³C**-**NMR (75,5 MHz, d₆-DMSO, ppm):**
δ=13.8 (CH₂CH₂*C*H₃), 14.0 (OCH₂*C*H₃), 21.8 (Alkyl-*C*H₂), 25.3 (Alkyl-*C*H₂), 27.9 (Alkyl-*C*H₂), 29.0 (Alkyl-*C*H₂), 30.9 (NCH₂*C*H₂), 49.4 (N*C*H₂), 61.2 (O*C*H₂), 120.7 (N*C*HCHN), 121.8 (arom. *C*H), 123.4 (NCH*C*HN), 130.5 (arom. *C1*), 130.8 (arom. *C*H), 135.6 (N*C*HN), 138.0 (arom. *C*4), 164.5 (*C*OO).
**Elementaranalyse:** C₁₉H₂₇BrN₂O₂ ber.: C 57,72%, H 6,88%, N 7,09%, gef.: C 57,54%, H 6,99%, N 7,15%.

### Ausführungsbeispiel 24

### 1-(4-Ethylcarboxyphenyl)-3-tetradecylimidazoliumbromid

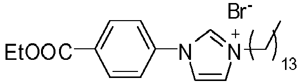

Es werden nach der allgemeinen Synthesevorschrift 4,60 mmol (1,00 g) 1-(4-Ethylcarboxyphenyl)imidazol und 5,50 mmol (1,50 g, 1,5 mL) 1-Bromtetradecan in 5 mL THF gelöst und für 68 h auf 90 °C erhitzt.
**Summenformel:** C₂₆H₄₁BrN₂O₂ (493,53 g/mol)
**Ausbeute:** 1,40 g (61,4%)
**Schmelzpunkt:** 126 °C
**¹H-NMR (300 MHz, d₆-DMSO, ppm):**
δ=0.85 (t, J=6,9 Hz, 3 H, CH₂CH₂C*H*₃), 1.27 (m, 22 H, Alkyl-CH₂), 1.40 (t, J=7,1 Hz, 3 H, OCH₂C*H₃*), 1.93 (m, 2 H, NCH₂C*H*₂), 4.25 (t, J=7,3 Hz, 2 H, NC*H₂*), 4.39 (q, J=7,1 Hz, 2 H, OC*H₂*), 7.97 (d, J=8,8 Hz, 2 H, arom. C*H*), 8.10 (s, 1 H, NC*H*CHN), 8.23 (d, J=8,8 Hz, 2 H, arom. C*H*), 8.45 (s, 1 H, NCHC*H*N), 9.96 (s, 1 H, NC*H*N)
**¹³C-NMR (75,5 MHz, d₆-DMSO, ppm):**
δ=13.9 (CH₂CH₂*C*H₃), 14.1 (OCH₂*C*H₃), 22.1 (Alkyl-*C*H₂), 25.5 (Alkyl-*C*H₂), 28.4 (Alkyl-*C*H₂), 28.7 (Alkyl-*C*H₂), 28.8 (Alkyl-*C*H₂), 28.9 (Alkyl-*C*H₂), 29.0 (Alkyl-*C*H₂), 29.0 (Alkyl-*C*H₂), 31.3 (NCH₂*C*H₂), 49.5 (N*C*H₂), 61.3 (O*C*H₂), 120.9 (N*C*HCHN), 121.9 (arom. *C*H), 123.5 (NCH*C*HN), 130.7 (arom. *C1*), 131.0 (arom. *C*H), 135.8 (N*C*HN), 138.1 (arom. *C*4), 164.6 (COO).
**Elementaranalyse:** C₂₆H₄₁BrN₂O₂ ber.: C 63,28%, H 8,37%, N 5,68%, gef.: C 63,20%, H 8,42%, N 5,76%

### Ausführungsbeispiel 25

### 1-(3,5-Bis(trifluormethyl)phenyl)-3-propylimidazoliumbromid

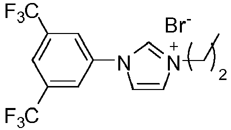

Es werden nach der allgemeinen Synthesevorschrift 3,60 mmol (1,00 g) 1-(3,5-Bis(trifluormethyl)phenyl)imidazol und 4,20 mmol (0,530 g, 0,39 mL) 1-Brompropan in 5 mL THF gelöst und für 18 h auf 90 °C erhitzt.
**Summenformel:** C₁₄H₁₃BrF₆N₂ (404,17 g/mol)
**Ausbeute:** 0,53 g (36,8%)
**Schmelzpunkt:** 188 °C
**¹H-NMR (500 MHz, d₆-DMSO, ppm):**
δ=0.95 (t, J=7,3 Hz, 3 H, CH₂C*H*₃), 1.97 (qt, J=7,3 Hz, J=7,1 Hz, 2 H, C*H*₂CH₃), 4.25 (t, J=7,1 Hz, 2 H, NC*H*₂), 8.05 (s, 1 H, NC*H*CHN), 8.42 (s, 1 H, arom. *p-CH*), 8.54 (s, 1 H, NCHC*H*N), 8.62(s, 2 H, arom. *o*-CH), 10.06 (s, 1 H, NC*H*N).
**¹³C**-**NMR (125,8 MHz, d₆-DMSO, ppm):**
δ=10.5 (CH₂*C*H₃), 22.6 (NCH₂*C*H₂), 51.1 (N*C*H₂), 121.5 (N*C*HCHN), 122.4 (q, J=271,3 Hz, *C*F₃), 122.5 (arom. C*H*), 123.3 (NCH*C*HN), 123.6 (arom. *C*H), 131.7 (q, J=34,0 Hz, arom. *C3, C5*), 136.4 (N*C*HN), 136.6 (arom. *C1*).
**¹⁹F-NMR (283 MHz, d₆-DMSO, ppm):**
δ=-61,2 (s, C*F*₃).
**Elementaranalyse:** C₁₄H₁₃BrF₆N₂ *0,25 H₂O ber.: C 41,25%, H 3,34%, N 6,87%, gef.: C 41,06%, H 3,50%, N 7,20%.

### Ausführungsbeispiel 26

### 3-Propyl-1-(4-nitrophenyl)-imidazoliumbromid

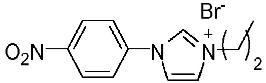

Es werden nach der allgemeinen Synthesevorschrift 5,3 mmol (1,00g) 1-(4-Nitrophenyl)imidazol und 6,3 mmol (0,780 g, 0,58 mL) 1-Brompropan in 5 mL THF gelöst und für 17 h auf 90 °C erhitzt.
**Summenformel:** C₁₂H₁₄BrN;O₂ (312,17 g/mol)
**Ausbeute:** 0,650 g (39,3%)
**Schmelzpunkt:** 207 °C
**¹H-NMR (300 MHz, d₆-DMSO, ppm):**
δ=0.85 (t, J=7,4 Hz, 3 H, CH₂C*H*₃), 1.94 (qt, J=7,4 Hz, J=7,1 Hz, 2 H, NCH₂C*H*₂), 4.25 (t, J=7,1 Hz, 2 H, NC*H₂*)*,* 8.10 (d, J=9,2 Hz, 2 H, arom. C*H*), 8.11 (s, 1 H, NC*H*CHN), 8.50 (s, 1 H, NCHC*H*N), 8.52 (d, J=9,2 Hz, 2 H, arom. CH), 10.05 (s, 1 H, NC*H*N).
**¹³C**-**NMR (75,5 MHz, d₆-DMSO, ppm):**
δ=10.5 (CH₂*C*H₃), 22.5 (NCH₂*C*H₂), 51.1 (N*C*H₂), 121.1 (NCHCHN), 122.9 (arom. CH), 123.6 (NCH*C*HN), 125.6 (arom. *C*H), 136.2 (N*C*HN), 139.1 (arom. *C1*), 147.5 (arom. *C*4).
**Elementaranalyse:** C₁₂H₁₄BrN₃O₂ * 0,3 H₂O ber.: C 45,39%, H 4,63%, N 13,23%, gef.: C 44,85%, H 4,00%, N 13,03%.

### Ausführungsbeispiel 27

### 1-(4-Nitrophenyl)-3-heptylimidazoliumbromid

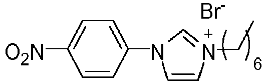

Es werden nach der allgemeinen Synthesevorschrift 5,3 mmol (1,00 g) 1-(4-Nitrophenyl)imidazol und 6,3 mmol (1,14 g, 1,0 mL) 1-Bromheptan in 5 mL THF gelöst und für 48 h auf 90 °C erhitzt.
**Summenformel:** C₁₆H₂₂BrN₃O₂ (368,27 g/mol)
**Ausbeute:** 1,81 g (92,8%)
**Schmelzpunkt:** 2 °C
**¹H-NMR (300 MHz, d₆-DMSO, ppm):**
δ=0.89 (t, J=6,9 Hz, 3 H, CH₂C*H*₃), 1.20-1.40 (m, 8 H, Alkyl-C*H*₂), 1.93 (m, 2 H, NCH₂C*H*₂), 4.26 (t, J=7,3 Hz, 2 H, NC*H₂*), 8.09 (d, J=8,7 Hz, 2 H, arom. *C*H), 8.10 (s, 1 H, NC*H*CHN), 8.47 (s, 1 H, NCHC*H*N), 8.59 (d, J=8,8 Hz, 2 H, arom. C*H*), 10.02 (s, 1 H, NC*H*N)
**¹³C-NMR (75,5 MHz, d₆-DMSO, ppm):**
δ=13.9 (CH₂*C*H₃), 22.0, 25.4, 28.1, 29.1, 30.7 (Alkyl-*C*H₂), 31.0 (NCH₂*C*H₂), 49.6 (N*C*H₂), 121.0 (N*C*HCHN), 122.9 (arom. *C*H), 123.6 (NCH*C*HN), 125.6 (arom. CH), 136.2 (N*C*HN), 139.3 (arom. *C*1), 147.5 (arom. *C4)*
**Elementaranalyse:** C₁₆H₂₂BrN₃O₂ * 0,5 H₂O ber.: C 50,94%, H 6,14%, N 11,14%, gef.: C 51,14%, H 6,36%, N 11,19%

### Ausführungsbeispiel 28

### 1-(4-Nitrophenyl)-3-tetradecylimidazoliumbromid

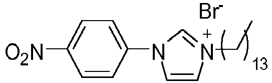

Es werden nach der allgemeinen Synthesevorschrift 5,3 mmol (1,00 g) 1-(4-Nitrophenyl)imidazol und 6,3 mmol (1,76 g, 1,70 mL) 1-Bromtetradecan in 5 mL THF gelöst und für 45 h auf 90 °C erhitzt. Am Ende der Reaktion wird die Reaktionsmischung auf Raumtemperatur abgekühlt und das ausgefallene Produkt mit THF und Diethylether gewaschen.
**Summenformel:** C₂₃H₃₆BrN₃O₂ (466,46 g/mol)
**Ausbeute:** 1,13 g (45,7%)
**Schmelzpunkt:** 114 °C
**¹H-NMR (300 MHz, d₆-DMSO, ppm):**
δ=0.85 (bs, 3 H, CH₂C*H*₃), 1.25 (m, 22 H, Alkyl-C*H*₂), 1.90 (m, 2 H, NCH₂C*H₂*), 4.25 (t, J=7,1 Hz, 2 H, NC*H₂*), 7.06 (m, 3 H, arom. C*H*, NC*H*CHN), 8.40 (s, 1 H, NCHC*H*N), 8.55 (d, J=9,0 Hz, 2 H, arom. C*H*), 10.01 (s, 1 H, NC*H*N).
**¹³C**-**NMR (75,5 MHz, d₆-DMSO, ppm):**
δ=13.9 (CH₂*C*H₃), 22.1 (Alkyl-*C*H₂), 25.5 (Alkyl-*C*H₂), 28.4 (Alkyl-CH₂), 28.7 (Alkyl-*C*H₂), 28.8 (Alkyl-*C*H₂), 28.9 (Alkyl-*C*H₂), 29.0 (Alkyl-*C*H₂), 29.0 (Alkyl-*C*H₂), 31.3 (NCH₂*C*H₂), 49.6 (N*C*H₂), 121.0 (N*C*HCHN), 122.8 (arom. CH), 123.6 (NCH*C*HN), 125.6 (arom. CH), 136.2 (N*C*HN), 139.3 (arom. *C1*), 147.5 (arom. *C*4).
**Elementaranalyse:** C₂₃H₃₆BrN₃O₂ * 0,4 H₂O ber.: C 55,57%, H 7,99%, N 8,45%, gef.: C 55,26%, H 7,67%, N 8,65%.

### Ausführungsbeispiel 29

### 1-(4-Methylphenyl)-3-propylimidazoliumbromid

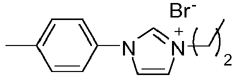

Es werden nach der allgemeinen Synthesevorschrift 6,30 mmol (1,00 g) 1-(4-Methylphenyl)imidazol und 7,60 mmol (0,930 g, 0,69 mL) 1-Brompropan in 5 mL THF gelöst und für 6 h auf 90 °C erhitzt.
**Summenformel:** C₁₃H₁₇BrN₂ (281,20 g/mol)
**Ausbeute:** 2,975 g (66,4%)
**¹H-NMR (500 MHz, d₆-DMSO, ppm):**
δ=0.80 (t, J₁=7,4 Hz, 3 H, CH₂C*H₃*), 1.82 (qt, J=7,3 Hz, J=7,2 Hz, 2 H, C*H₂*CH₃), 3.22 (s, 3 H, C*H₃*), 4.10 (t, J=7,2 Hz, 2 H, NC*H₂*), 7.35 (d, J=8,2 Hz, 2 H, arom. C*H*), 7.56 (d, J=8,2 Hz, 2 H, arom. CH), 7.95 (s, 1 H, NC*H*CHN), 8.20 (s, 1 H, NCHC*H*N), 9.72 (s, 1 H, NC*H*N)
**¹³C**-**NMR (125,8 MHz, d₆-DMSO, ppm):**
δ=10.5 (CH₂*C*H₃), 20.54 (*C*H₃), 22.6 (NCH₂*C*H₂), 50.8 (N*C*H₂), 121.1 (N*C*HCHN), 121.6 (arom. *C*H), 123.2 (NCH*C*HN) 130.5 (arom. *C*H), 132.4 (arom. *C1*), 135.1 (N*C*HN), 139.6 (arom. *C*4)

### Ausführungsbeispiel 30

### 1-(2,6-Diisopropylphenyl)-3-propylimidazoliumbromid

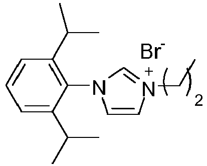

Es werden nach der allgemeinen Synthesevorschrift 4,4 mmol (1,00 g) 1-(2,6-Diisopropylphenyl)imidazol und 5,3 mmol (0,650 g, 0,48 mL) 1-Brompropan in 5 mL THF gelöst und für 20 h auf 90 °C erhitzt.
**Summenformel:** C₁₈H₂₇BrN₂ (351,33 g/mol)
**Ausbeute:** 1,13 g (73,4%)
**Schmelzpunkt:** 193,7 °C
**¹H-NMR (300 MHz, d₆-DMSO, ppm):**
δ=0.85 (t, J₁=7,4 Hz, 3 H, CH₂C*H*₃), 1.20 (d, J=6,9 Hz, 12H, CH(C*H*₃)₂), 1,85 (sept., J=7,0 Hz 2H, C*H*(CH₃)₂), 2.25 (m, 2H, NCH₂C*H*₂), 4.29 (t, J=6,8 Hz, 2 H, NC*H₂*), 7,42 (d, J=7,8 Hz, 2H, arom. *m*-C*H*), 7.65 (t, J=7,8 Hz, 1H, arom. *p*-CH), 8.11 (s, 1 H, NC*H*CHN), 8.18 (s, 1 H, NCHC*H*N), 9.62 (s, 1 H, NC*H*N)
**¹³C-NMR (75,5 MHz, d₆-DMSO, ppm):**
δ=10.1 (CH₂*C*H₃),22.5 (CH₂CH₃), 23.7 (iso-Propyl CH), 23.8 (iso-Propyl *C*H₃), 28,1 (iso-Propyl CH₃), 50.9 (N*C*H₂), 123.4 (N*C*HCHN), 124.4 (arom. *m*-*C*H), 125.2 (NCH*C*HN), 130.5 (arom. *C1*), 131.5 (arom. C*4*H), 137.6 (N*C*HN), 145.1 (arom. *C2*, *C6)*

### Ausführungsbeispiel 31

### 1-(2,6-Diisopropylphenyl)-3-hexylimidazoliumbromid

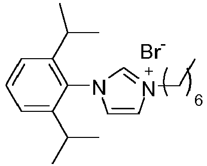

Es werden nach der allgemeinen Synthesevorschrift 4,4 mmol (1,00 g) 1-(2,6-Diisopropylphenyl)imidazol und 5,3 mmol (0,940 g, 0,83 mL) 1-Bromheptan in 5 mL THF gelöst und für 20 h auf 110 °C erhitzt.
**Summenformel:** C₂₂H₃₅BrN₂ (351,33 g/mol)
**Ausbeute:** 1,53 g (86,0%)
**Schmelzpunkt:** 68 °C
**¹H-NMR (300 MHz, d₆-DMSO, ppm):**
δ=0.85 (t, J=6,8 Hz, 3 H, CH₂C*H₃*), 1.20 (d, J=6,9 Hz, 12H, CH(C*H*₃)₂), 1.30-1.49 (m, 8 H, Alkyl-CH₂), 1.90 (m, 2 H, NCH₂C*H₂*), 2,15 (sept., J=7,0 Hz 2H, C*H*(CH₃)₂), 4.26 (t, J=7,3 Hz, 2 H, NC*H₂*), 7,40 (d, J=7,8 Hz, 2H, arom. *m*-C*H*), 7.55 (t, J=7,8 Hz, 1H, arom. *p*-CH), 8.08 (s, 1 H, NC*H*CHN), 8.11 (s, 1 H, NCHC*H*N), 9.58 (s, 1 H, NC*H*N)
**¹³C-NMR (75,5 MHz, d₆-DMSO, ppm):**
δ=13.9 (CH₂*C*H₃), 21.9 (*C*H₂CH₃), 23.7 (iso-Propyl *C*H₃), 23,8 (iso-Propyl CH₃),25,2, 27.8 (*C*H₂), 28.1 (iso-Propyl *C*H), 28,9 (*C*H₂), 31,1 (NCH₂*C*H₂), 49.4 (N*C*H₂), 123.4 (N*C*HCHN), 124.4 (arom. *m-C*H), 125.2 (NCH*C*HN), 130.5 (arom. *C1*), 131.5 (arom. *C4*H), 137.6 (N*C*HN), 145.1 (arom. *C2, C6*)

### Ausführungsbeispiel 32

### 1-(2-Ethoxyphenyl)-3-propylimidazoliumbromid

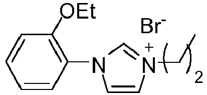

Es werden nach der allgemeinen Synthesevorschrift 5,3 mmol (1,00 g) 1-(2-Ethoxyphenyl)imidazol und 6,4 mmol (0,780 g, 0,58 mL) 1-Brompropan in 5 mL THF gelöst und für 21 h auf 90 °C erhitzt.
**Summenformel:** C₁₄H₂₉BrN₂O (311,22 g/mol)
**Ausbeute:** 1,390 g (84,2%)
**Schmelzpunkt:** 68,2 °C
**¹H-NMR (300 MHz, d₆-DMSO, ppm):**
δ=0.86 (t, J₁=7,4 Hz, 3 H, CH₂CH₂C*H*₃), 1.30 (t, J=7,0 Hz, 3H OCH₂C*H*₃), 1.92 (p, J=7,3 Hz 2 H, NCH₂C*H*₂), 4.12 (q, J=7,0 Hz, 2 H, OC*H*₂CH₃), 4.29 (t, J=7,3 Hz, 2 H, NC*H₂*), 7.12 (t, J=7,7 Hz, 1 H, arom. C*H*), 7.37 (d, J=7,4 Hz, 1 H, arom. C*H*), 7.51-7.70 (m, 2 H, arom. C*H*), 8.02 (s, 1 H, NC*H*CHN), 8.10 (s, 1 H, NCHC*H*N), 9.59 (s, 1 H, NC*H*N)
**¹³C-NMR (75,5 MHz, d₆-DMSO, ppm):**
δ=10.3 (CH₂CH₂*C*H₃), 14.2 (OCH₂*C*H₃), 22.7 (Alkyl-*C*H₂), 30.5 (NCH₂*C*H₂), 50.7 (N*C*H₂), 64.6 (OCH₂), 114.0 (arom. *C*3H), 121.0 (arom. *C*5H), 122.2 (N*C*HCHN), 123.5 (arom. *C*1), 123.8 (NCHCHN), 126.2 (arom. *C*6H), 131.6 (arom. *C*4H), 137.2 (N*C*HN), 151.4 (arom. *C*20)

### Ausführungsbeispiel 33

### 1-(2-Ethoxyphenyl)-3-hexylimidazoliumbromid

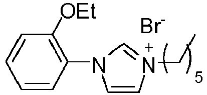

Es werden nach der allgemeinen Synthesevorschrift 10,00 mmol (1,883 g) 1-(2-Ethoxyphenyl)imidazol und 11,00 mmol (1,816 g, 1,59 mL) 1-Bromhexan in 3 mL Diethylether gelöst und für 12 h auf 60 °C erhitzt.
**Summenformel:** C₁₇H₂₅BrN₂O (353,30 g/mol)
**Ausbeute:** 3,549 g (100%)
**Schmelzpunkt:** -34 °C
**¹H-NMR (300 MHz, d₆-DMSO, ppm):**
δ=0.87 (t, J₁=6,7 Hz, 3 H, CH₂CH₂C*H*₃), 1.21-1.36 (m, 9 H, Alkyl C*H₂* und Ethoxy-C*H₃*), 1.90 (p, J=6,9 Hz 2 H, NCH₂C*H₂*), 4.17 (q, J=7.0 Hz, 2 H, OC*H₂*CH₃), 4.30 (t, J=7,1 Hz, 2 H, NC*H₂*), 7.10-7.22 (m, 1 H, arom. C*H*), 7.36 (d, J=8,4 Hz, 1 H, arom. C*H*), 7.51-7.70 (m, 2 H, arom. C*H*), 8.03 (s, 1 H, NC*H*CHN), 8.09 (s, 1 H, NCHC*H*N), 9.62 (s, 1 H, NC*H*N)
**¹³C-NMR (75,5 MHz, d₆-DMSO, ppm):**
δ=13.8 (CH₂CH₂*C*H₃), 14.2 (OCH₂*C*H₃), 21.8, 25.1, 29.1 (Alkyl-*C*H₂), 30.5 (NCH₂*C*H₂), 49.2 (N*C*H₂), 64.7 (OCH₂), 114.0 (arom. *C*3H), 121.0 (arom. *C*5H), 122.2 (N*C*HCHN), 123.5 (arom. *C*1), 123.8 (NCH*C*HN), 126.2 (arom. *C*6H), 131.6 (arom. *C*4H), 137.2 (N*C*HN), 151.4 (arom. *C*20)

### Ausführungsbeispiel 34

### 1-(2-Ethoxyphenyl)-3-heptylimidazoliumbromid

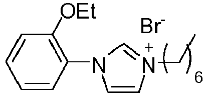

Es werden nach der allgemeinen Synthesevorschrift 1,62 mmol (0,305 g) 1-(2-Ethoxyphenyl)imidazol und 11,00 mmol (0,319 g, 0,280 mL) 1-Bromheptan in 3 mL Diethylether gelöst und für 4 h auf 100 °C erhitzt.
**Summenformel:** C₁₈H₂₇BrN₂O (367,32 g/mol)
**Ausbeute:** 0,064 g (10,7%)
**Schmelzpunkt:** -23 °C
**¹H-NMR (300 MHz, d₆-DMSO, ppm):**
δ=0.86 (t, J₁=6,8 Hz, 3 H, CH₂CH₂C*H*₃), 1.15-1.40 (m, 11 H, Alkyl C*H₂* und Ethoxy-C*H*₃), 1.88 (p, J=7,2 Hz 2 H, NCH₂C*H*₂), 4.16 (q, J=7.0 Hz, 2 H, OC*H₂*CH₃), 4.27 (t, J=7,0 Hz, 2 H, NC*H*₂), 7.17 (t, J=7,7 Hz, 1H, arom. *CH*), 7.35 (d, J=8,3 Hz, 1 H, arom. *CH*), 7.50-7.68 (m, 2 H, arom. *CH*), 8.02 (s, 1 H, NC*H*CHN), 8.07 (s, 1 H, NCHC*H*N), 9.58 (s, 1 H, NC*H*N)
**¹³C**-**NMR (75,5 MHz, d₆-DMSO, ppm):**
δ=13.8 (CH₂CH₂*C*H₃), 14.1 (OCH₂*C*H₃), 21.8, 25.2, 27.9, 29.1 (Alkyl-*C*H₂), 30.9 (NCH₂*C*H₂), 49.1 (N*C*H₂), 64.5 (OCH₂), 113.8 (arom. C3H), 120.9 (arom. *C*5H), 122.1 (N*C*HCHN), 123.4 (arom. *C*1), 123.6 (NCH*C*HN), 126.0 (arom. C6H), 131.5 (arom. C4H), 137.5 (N*C*HN), 151.3 (arom. *C*20)

### Ausführungsbeispiel 35

### 1-(4-Ethoxyphenyl)-3-hexylimidazoliumbromid

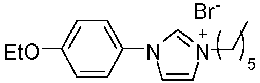

Es werden nach der allgemeinen Synthesevorschrift 10,00 mmol (1,883 g) 1-(4-Ethoxyphenyl)imidazol und 11,00 mmol (1,816 g, 1,59 mL) 1-Bromheptan in 3 mL Diethylether gelöst und für 12 h auf 60 °C erhitzt.
**Summenformel:** C₁₇H₂₅BrN₂O (353,30 g/mol)
**Ausbeute:** 1,400 g (39,6%)
**Schmelzpunkt:** -23 °C
**¹H-NMR (500 MHz, d₆-DMSO, ppm):**
δ=0.86 (t, J₁=6,6 Hz, 3 H, CH₂CH₂C*H*₃, 1.30 (s, 6 H, Alkyl-CH₂), 1.35 (t, J=7,0 Hz, 3 H, OCH₂C*H*₃), 1.88 (m, 2 H, NCH₂C*H₂*), 4.10 (q, J=7,0 Hz, 2 H, OC*H₂*), 4.25 (t, J=7,3 Hz, 2 H, NC*H₂*), 7.16 (d, J=9,0 Hz, 2 H, arom. C*H*), 7.72 (d, J=9,0 Hz, 2 H, arom. CH), 8.07 (s, 1 H, NC*H*CHN), 8.27 (s, 1 H, NCHC*H*N), 9.87 (s, 1 H, NC*H*N)
**¹³C**-**NMR (125,8 MHz, d₆-DMSO, ppm):**
δ=13.8 (CH₂CH₂*C*H₃), 14.5 (OCH₂*C*H₃), 21.8, 25.2, 29.1 (Alkyl-*C*H₂), 30.6 (NCH₂*C*H₂), 49.2 (N*C*H₂), 63.7 (OCH₂), 115.5 (arom. *C*H), 121.3 (N*C*HCHN), 123.1 (NCH*C*HN), 127.7 (arom. *C1*), 135.0 (N*C*HN), 138.0 (arom. C4), 159.1 (arom. *C*O)

### Ausführungsbeispiel 36

### 1-(4-Methoxyphenyl)-3-propylimidazoliumbromid

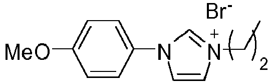

Es werden nach der allgemeinen Synthesevorschrift 4,48 mmol (0,700 g) 1-(4-Methoxyphenyl)imidazol und 4,93 mmol (0,606 g, 0,45 mL) 1-Brompropan in 5 mL THF gelöst und für 5 h auf 80 °C erhitzt.
**Summenformel:** C₁₃H₁₇BrN₂O (297,19 g/mol)
**Ausbeute:** 0,884 g (66,4%)
**Schmelzpunkt:** flüssig bei Raumtemperatur
**¹H-NMR (500 MHz, d₆-DMSO, ppm):**
δ=0.92 (t, J₁=7,3 Hz, 3 H, CH₂C*H*₃), 1.91 (qt, J=7,3 Hz, J=7,1 Hz, 2 H, C*H*₂CH₃), 3.85 (s, 3 H, OC*H*₃), 4.22 (t, J=7,1 Hz, 2 H, NC*H₂*), 7.20 (d, J=9,0 Hz, 2 H, arom. C*H*), 7.74 (d, J=9,0 Hz, 2 H, arom. CH), 8.05 (s, 1 H, NC*H*CHN), 8.27 (s, 1 H, NCHC*H*N), 9.82 (s, 1 H, NC*H*N)
**¹³C**-**NMR (125,8 MHz, d₆-DMSO, ppm):**
δ=10.6 (CH₂*C*H₃), 22.8 (NCH₂CH₂), 50.8 (N*C*H₂), 55.8 (O*C*H₃), 115.2 (arom. *C*H), 121.5 (N*C*HCHN), 123.2 (NCH*C*HN) 123.6 (arom. *C*H), 127.9 (arom. *C1*), 135.1 (N*C*HN), 160.0 (arom. *C*4)

### Ausführungsbeispiel 37

### 3-Hexyl-1-(4-methoxyphenyl)imidazoliumbromid

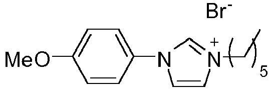

Es werden nach der allgemeinen Synthesevorschrift 10,40 mmol (1,812 g) 1-(4-Methoxyphenyl)imidazol und 11,44 mmol (1,889 g, 1,65 mL) 1-Bromhexan in 5 mL THF gelöst und für 12 h auf 80 °C erhitzt.
**Summenformel:** C₁₆H₂₃BrN₂O (339,27 g/mol)
**Ausbeute:** 2,085 g (59,1%)
**Schmelzpunkt:** -11 °C
**¹H-NMR (300 MHz, d₆-DMSO, ppm):**
δ=0.88 (t, J₁=6,5 Hz, 3 H, CH₂CH₂C*H*₃), 1.32 (bs, 6 H, Alkyl-CH₂), 1.89 (m, 2 H, NCH₂C*H₂*), 3.85 (s, 3 H, OC*H*₃), 4.24 (t, J=7,4 Hz, 2 H, NC*H*₂), 7.20 (d, J=9,1 Hz, 2 H, arom. C*H*), 7.73 (d, J=9,1 Hz, 2 H, arom. CH), 8.07 (s, 1 H, NC*H*CHN), 8.27 (s, 1 H, NCHC*H*N), 9.77 (s, 1 H, NC*H*N)
**¹³C-NMR (75,5 MHz, d₆-DMSO, ppm):**
δ=13.7 (CH₂*C*H₃), 21.6, 25.3, 29.0 (Alkyl-*C*H₂), 30.7 (*C*H₂CH₃), 49.3 (N*C*H₂), 55.8 (O*C*H₃), 115.0 (arom. *C*H), 121.4 (N*C*HCHN), 123.2 (NCH*C*HN) 123.6 (arom. *C*H), 128.0 (arom. *C1*), 135.2 (N*C*HN), 160.0 (arom. *C*4)

### Ausführungsbeispiel 38

### 1-(4-Methoxyphenyl)-3-heptylimidazoliumbromid

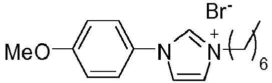

Es werden nach der allgemeinen Synthesevorschrift 5,7 mmol (1,00 g) 1-(4-Methoxyphenyl)imidazol und 6,9 mmol (1,23 g, 1,1 mL) 1-Bromheptan in 5 mL THF gelöst und für 18 h auf 90 °C erhitzt.
**Summenformel:** C₁₇H₂₅BrN₂O (353,30 g/mol)
**Ausbeute:** 1,860 g (91,6%)
**Schmelzpunkt:** flüssig bei Raumtemperatur
**¹H-NMR (500 MHz, d₆-DMSO, ppm):**
δ=0.87 (t, J₁=7,3 Hz, 3 H, CH₂C*H*₃), 1.30 (bs, 8 H, Alkyl-C*H*₂), 1.88 (m, 2H, NCH₂C*H*₂), 3.77 (s, 3 H, OC*H₃*), 4.23 (t, J=7,2 Hz, 2 H, NC*H₂*), 7.17 (d, J=9,1 Hz, 2 H, arom. CH), 7.74 (d, J=9,1 Hz, 2 H, arom. CH), 8.04 (s, 1 H, NC*H*CHN), 8.26 (s, 1 H, NCHC*H*N), 9.79 (s, 1 H, NC*H*N)

### Ausführungsbeispiel 39

### 3-Hexyl-1-(2-methoxyphenyl)imidazoliumbromid

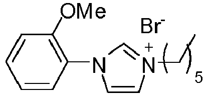

Es werden nach der allgemeinen Synthesevorschrift 10,40 mmol (1,812 g) 1-(2-Methoxyphenyl)imidazol und 11,44 mmol (1,889 g, 1,65 mL) 1-Bromhexan in 5 mL THF gelöst und für 5 h auf 80 °C erhitzt.
**Summenformel:** C₁₆H₂₃BrN₂O (339,27 g/mol)
**Ausbeute:** 2,202 g (62,4%)
**Schmelzpunkt:** -14 °C
**¹H-NMR (300 MHz, d₆-DMSO, ppm):**
δ=0.88 (t, J₁=6,8 Hz, 3H, CH₂C*H*₃), 1.31 (bs, 6H, Alkyl-C*H₂*), 1.87 (m, 2H, NCH₂C*H₂*), 3.89 (s, 3H, OC*H₃*), 4.30 (t, J=7,2 Hz, 2H, NC*H₂*), 7.20 (t, J=8,8 Hz, 1H arom. C*H*), 7.39 (d, J=8,4 Hz, 1H, arom. C*H*), 7.61-7.75 (m, 2H, arom. CH), 8.06 (s, 1H, NC*H*CHN), 8.08 (s, 1H, NCHC*H*N), 9.67 (s, 1H, N*CH*N)
**¹³C-NMR (75,5 MHz, d₆-DMSO, ppm):**
δ=13.8 (CH₂*C*H₃), 21.8, 25.1, 29.1 (Alkyl-CH₂), 30.5 (*C*H₂CH₃), 49.2 (N*C*H₂), 56.4 (O*C*H₃), 113.2 (arom. *C*3H), 121.1 (arom. *C*5H), 122.3 (N*C*HCHN), 123.4 (arom. *C*1), 123.8 (NCH*C*HN), 126.2 (arom. *C*6H), 131.6 (arom. *C*4H), 137.1 (N*C*HN), 152.1 (arom. *C*20)

### Ausführungsbeispiel 40

### 3-Heptyl-1-(2-methoxyphenyl)imidazoliumbromid

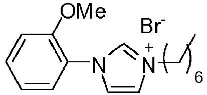

Es werden nach der allgemeinen Synthesevorschrift 5,8 mmol (1,00 g) 1-(2-Methoxyphenyl)imidazol und 7,0 mmol (1,25 g, 1,1 mL) 1-Bromheptan in 5 mL THF gelöst und für 17 h auf 90 °C erhitzt.
**Summenformel:** C₁₇H₂₅BrN₂O (353,30 g/mol)
**Ausbeute:** 1,26 g (62,4%)
**Schmelzpunkt:** 106 °C
**¹H-NMR (300 MHz, d₆-DMSO, ppm):**
δ=0.92 (t, J₁=6,8 Hz, 3H, CH₂C*H*₃), 1.30 (bs, 8H, Alkyl-C*H*₂), 1.90 (m, 2 H, NCH₂C*H*₂), 3.93 (s, 3 H, OC*H*₃), 4.30 (t, J=7,2 Hz, 2 H, NC*H₂*), 7.24 (t, J=8,2 Hz, 1 H arom. C*H*), 7.45 (d, J=8,3 Hz, 1 H, arom. C*H*), 7.59-7.65 (m, 2 H, arom. CH), 8.06 (s, 1 H, NC*H*CHN), 8.11 (s, 1 H, NCHC*H*N), 9.62 (s, 1 H, NC*H*N)
**¹³C**-**NMR (75,5 MHz, d₆-DMSO, ppm):**
δ=13.9 (CH₂*C*H₃), 22.0, 25.4, 28.0, 29.2 (Alkyl-*C*H₂), 31.0 (*C*H₂CH₃), 49.2 (N*C*H₂), 56.4 (O*C*H₃), 113.2 (arom. *C*3H), 121.1 (arom. *C*5H), 122.3 (N*C*HCHN), 123.4 (arom. *C*1), 123.8 (NCHCHN), 126.2 (arom. *C*6H), 131.7 (arom. *C*4H), 137.1 (N*C*HN), 152.2 (arom. *C*20)

### Ausführungsbeispiel 41

### 1-(3,5-Bistrifluormethylphenyl)-3-heptylimidazoliumbromid

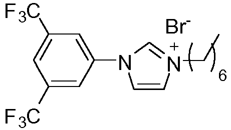

Es werden nach der allgemeinen Synthesevorschrift 3,60 mmol (1,00 g) 1-(3,5-Bistrifluormethylphenyl)imidazol und 4,30 mmol (0,770 g, 0,67 mL) 1-Bromheptan in 5 mL THF gelöst und für 18 h auf 90 °C erhitzt.
**Summenformel:** C₁₈H₂₁BrF₆N₂ (459,27 g/mol)
**Ausbeute:** 1,23 g (75,0%)
**¹H-NMR (500 MHz, d₆-DMSO, ppm):**
δ=0.85 (bs, 3 H, CH₂C*H₃*), 1.1-1.4 (m, 8 H, C*H₂*), 1.9 (bs, 2 H, NCH₂C*H₂*), 4.30 (t, J=7,1 Hz, 2 H, NC*H₂*), 8.05 (s, 1 H, NC*H*CHN), 8.39 (s, 1H, arom*. p-*C*H),* 8.52 (s, 1 H, NCHC*H*N), 8.62(s, 2H, arom. o-CH), 10.06 (s, 1 H, NCHN)
**¹³C**-**NMR (125,8 MHz, d₆-DMSO, ppm):**
δ=14.0 (CH₂*C*H₃), 22.0, 25.5, 28.0, 29.1 (C*H₂*), 31.1 (NCH₂*C*H₂), 49.6 (N*C*H₂), 121.5 (NCHCHN), 122.4 (q, J=271,5 Hz, *C*F₃), 122.5 (arom. C*H*), 123.3 (NCH*C*HN), 123.7 (arom. *C*H), 131.7 (q, J=33,8 Hz, arom. *C3, C5*), 136.5 (N*C*HN), 136.6 (arom. *C1*)
**¹⁹F-NMR (283 MHz, d₆-DMSO, ppm):**
-61,2 (s, C*F₃*)

### Allgemeine Synthesevorschrift für Imidazoliumsalze mit Bis(trifluormethylsulfon)imid-Anionen

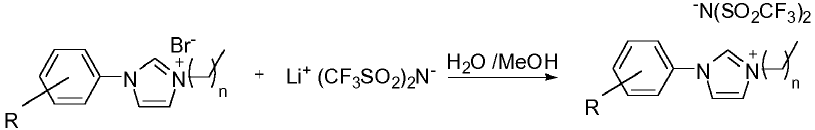

1,0 eq. des Imidazoliumbromid-Salzes wird in Wasser oder einer Wasser/Methanol Mischung vollständig gelöst. Unter ständigem Rühren werden 1,1 eq. Li⁺(CF₃SO₂)₂N⁻ zugegeben. Dabei bilden sich in der Reaktionsmischung nach wenigen Minuten zwei Phasen aus. Zur Vervollständigung der Reaktion wird für weitere 15 Minuten gerührt. Anschließend werden zu der Reaktionsmischung 15 mL Dichlormethan gegeben. Danach wird in einem Scheidetrichter die organische Phase von der wässrigen Phase abgetrennt. Die wässrige Phase wird noch zwei Mal mit je 10 mL Dichlormethan extrahiert. Die organischen Phasen werden vereinigt, über Magnesiumsulfat getrocknet und das Lösungsmittel wird im Vakuum entfernt.

### Ausführungsbeispiel 42

### 1-Butyl-3-mesitylimidazoliumbis(trifluormethylsulfon)imid

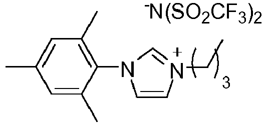

Es werden nach der allgemeinen Synthesevorschrift 1,55 mmol (0,500 g) 1-Butyl-3-mesitylimidazoliumbromid in 5 mL Wasser gelöst und 1,70 mmol (0,488 g) Lithiumbis(trifluormethylsulfon)imid zugegeben.
**Summenformel:** C₁₈H₂₃F₆N₃S₂O₄ (523,51 g/mol)
**Ausbeute:** 0,625 g (77,2%)
**Schmelzpunkt:** 26 °C
**Zersetzungspunkt:** 440 °C
**¹H-NMR (500 MHz, d₆-DMSO, ppm):**
δ=0.94 (t, J=7,4 Hz, 3 H, CH₂C*H₃*), 1.28 (dq, J=7,4 Hz, J=7,6 Hz, 2 H, C*H₂*CH₃), 1.89 (p, J=7,6 Hz, 2 H, NCH₂CH₂), 2.02 (s, 6 H, arom. *o*-C*H₃*), 2.34 (s, 3 H, *p*-C*H₃*), 4.28 (t, J=7,1 Hz, 2 H, NC*H₂*CH₃), 7.16 (s, 2 H, arom. C*H*), 7.94 (s, 1 H, NC*H*CHN), 8.11 (s, 1 H, NCHC*H*N), 9.43 (s, 1 H, NC*H*N).
**¹³C**-**NMR (125,8 MHz, d₆-DMSO, ppm):**
δ=13.2 (CH₂*C*H₃), 16.8 (*o*-*C*H₃), 18.7 (*C*H₂CH₃), 20.6 (*p-C*H3), 31.0 (NCH₂*C*H₂), 49.1 (N*C*H₂CH₃), 119.5 (q, J=326 Hz, *C*F₃), 123.2 (N*C*HCHN), 124.0 (NCH*C*HN), 129.2 (arom. CH), 131.2 (arom. *C1*), 134.3 (arom. *C*2 und *C*6), 137.2 (NCHN), 140.3 (arom. *C*4).
**¹⁹F-NMR (283 MHz, d₆-DMSO, ppm):**
δ=-78.7 ppm (C*F₃*).
**Elementaranalyse:** C₁₈H₂₃F₆N₃S₂O₄ ber.: C 41,30%, H 4,43%, N 8,03%, S 12,25%, gef.: C 41,64%, H 4,71%, N 8,11%, S 11,87%.

### Ausführungsbeispiel 43

### 1-Mesityl-3-pentylimidazoliumbis(trifluormethylsulfon)imid

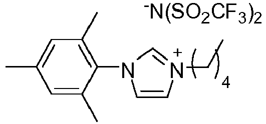

Es werden nach der allgemeinen Synthesevorschrift 1,48 mmol (0,500 g) 1-Mesityl-3-pentylimidazoliumbromid in 3 mL Wasser gelöst und 1,63 mmol (0,468 g) Lithiumbis(trifluormethylsulfon)imid zugegeben.
**Summenformel:** C₁₉H₂₅F₆N₃O₄S₂ (537,54 g/mol)
**Ausbeute:** 0,691 g (86,7%)
**Schmelzpunkt:** 21 °C
**Zersetzungspunkt:** 440 °C
**¹H-NMR (300 MHz, d₆-DMSO, ppm):**
δ=0.89 (t, J=7,3 Hz, 3 H, CH₂C*H₃*), 1.20-1.45 (m, 4 H, C*H₂*C*H₂*), 1.92 (p, J=7,3 Hz, 2 H, NCH₂C*H₂*), 2.03 (s, 6 H, arom. *o-*C*H₃*), 2.34 (s, 3 H, *p*-C*H₃*), 4.28 (t, J=7,3 Hz, 2 H, NC*H₂*), 7.16 (s, 2 H, arom. C*H*), 7.95 (s, 1 H, NC*H*CHN), 8.11 (s, 1 H, NCHC*H*N), 9.44 (s, 1 H, NC*H*N).
**¹³C-NMR (75,5 MHz, d₆-DMSO, ppm):**
δ=13.8 (CH₂*C*H₃), 16.8 (*o*-*C*H₃), 20.6 (*p-C*H₃), 21.4 (Alkyl-*C*H₂), 27.6 (Alkyl-*C*H₂), 28.7 (NCH₂*C*H₂), 49.3 (N*C*H₂CH₃), 119.5 (q, J=322 Hz, *C*F₃), 123.2 (N*C*HCHN), 124.0 (NCH*C*HN) 129.2 (arom. *C*H), 131.2 (arom. *C1*), 134.3 (arom. *C*2 und *C*6), 137.3 (N*C*HN), 140.3 (arom. *C*4).
**¹⁹F-NMR (283 MHz, d₆-DMSO, ppm):**
δ=-78.7 ppm (C*F₃*).
**Elementaranalyse:** C₁₉H₂₅F₆N₃O₄S₂ ber.: C 42,45%, H 4,69%, N 7,82%, S 11,93%, gef.: C 42,59%, H 4,82%, N 7,96%, S 11,94%.

### Ausführungsbeispiel 44

### 1-Hexyl-3-mesitylimidazoliumbis(trifluormethylsulfon)imid

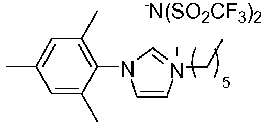

Es werden nach der allgemeinen Synthesevorschrift 1,42 mmol (0,500 g) 1-Hexyl-3-mesityl-imidazoliumbromid in 6 mL Wasser und 2 mL Methanol gelöst und 1,57 mmol (0,451 g) Lithiumbis(trifluormethylsulfon)imid zugegeben.
**Summenformel:** C₂₀H₂₇F₆N₃O₄S₂ (551,56 g/mol)
**Ausbeute:** 0,677 g (86,2%)
**Schmelzpunkt:** 40 °C
**Zersetzungspunkt:** 440 °C
**¹H-NMR (300 MHz, d₆-DMSO, ppm):**
δ=0.93 (t, J=6,8 Hz, 3 H, CH₂C*H*₃), 1.29-1.41 (m, 6 H, C*H₂*C*H₂*C*H₂*), 2.08 (p, J=6,9 Hz, 2 H, NCH₂C*H₂*), 2.07 (s, 6 H, arom. *o*-C*H₃*), 2.39 (s, 3 H, *p*-C*H₃*), 4.33 (t, J=7,0 Hz, 2 H, NC*H₂*), 7.21 (s, 2 H, arom. C*H*), 8.00 (s, 1 H, NC*H*CHN), 8.17 (s, 1 H, NCHC*H*N), 9.51 (s, 1 H, NC*H*N).
**¹³C-NMR (75,5 MHz, d₆-DMSO, ppm):**
δ=13.7 (CH₂*C*H₃), 16.8 (*o*-*C*H₃), 20.6 (*p-C*H₃), 21.9 (Alkyl-*C*H₂), 25.0 (Alkyl-*C*H₂), 28.9 (Alkyl-*C*H₂), 30.4 (NCH₂*C*H₂), 49.3 (N*C*H₂CH₃), 119.5 (q, *C*F₃), 123.2 (N*C*HCHN), 124.0 (NCH*C*HN) 129.2 (arom. *C*H), 131.2 (arom. *C1*), 134.3 (arom. *C*2 und *C*6), 137.2 (N*C*HN), 140.3 (arom. *C*4).
**¹⁹F-NMR (283 MHz, d₆-DMSO, ppm):**
δ=-78.7 ppm (C*F₃*).
**Elementaranalyse:** C₂₀H₂₇F₆N₃O₄S₂ ber.: C 43,55%, H 4,93%, N 7,62%, S 11,63%, gef.: C 43,78%, H 5,00%, N 7,58%, S 11,54%.

### Ausführungsbeispiel 45

### 1-Mesity-3-octylimidazoliumbis(trifluormethylsulfon)imid

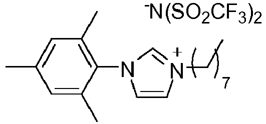

Es werden nach der allgemeinen Synthesevorschrift 2,36 mmol (1,00 g) 1-Mesityl-3-octylimidazoliumbromid in 7 mL Wasser und 3 mL Methanol gelöst und 2.89 mmol (0,830 g) Lithiumbis(trifluormethylsulfon)imid zugegeben.
**Summenformel:** C₂₂H₃₁F₆N₃O₄S₂ (579,62 g/mol)
**Ausbeute:** 1,202 g (61,5%)
**Schmelzpunkt:** 11 °C
**¹H-NMR (300 MHz, d₆-DMSO, ppm):**
δ=0.86 (t, J=7,0 Hz, 3 H, CH₂C*H₃*), 1.20-1.40 (m, 10 H, Alkyl-C*H₂*), 1.89 (p, J=7,0 Hz, 2 H, NCH₂C*H₂*), 2.02 (s, 6 H, arom. *o-*C*H₃*), 2.34 (s, 3 H, *p*-C*H₃*), 4.27 (t, J=7,0 Hz, 2 H, NC*H₂*), 7.16 (s, 2 H, arom. CH), 7.95 (s, 1 H, NC*H*CHN), 8.10 (s, 1 H, NCHCHN), 9.43 (s, 1 H, NCHN).
**¹³C**-**NMR (75,5 MHz, d₆**-**DMSO**, **ppm):**
δ=13.9 (CH₂*C*H₃), 16.8 (*o-C*H₃), 20.5 (*p-C*H₃), 22.0 (C*H₂*CH₂CH₃), 25.4 (Alkyl-*C*H₂), 28.2 (Alkyl-*C*H₂), 28.5 (Alkyl-*C*H₂), 29.0 (Alkyl-*C*H₂), 31.1 (NCH₂*C*H₂), 49.3 (N*C*H₂), 119.5 (q, CF₃), 123.2 (NCHCHN), 124.0 (NCHCHN) 129.2 (arom. CH), 131.2 (arom. *C1*), 134.3 (arom. C2 und C6), 137.2 (NCHN), 140.3 (arom. C4).
**¹⁹F**-**NMR (283 MHz, d₆-DMSO, ppm):**
δ=-78.7 ppm (C*F*₃).
**Elementaranalyse:** C₂₂H₃₁F₆N₃O₄S₂ ber: C 45,59%, H 5,39%, N 7,25%, S 11,06%, gef.: C 45,59%, H 5,48%, N 7,18%, S 10,83%.

### Ausführungsbeispiel 46

### 1-Mesityl-3-undecylimidazoliumbis(trifluormethylsulfon)imid

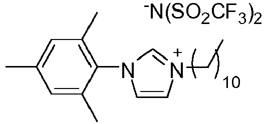

Es werden nach der allgemeinen Synthesevorschrift 1,20 mmol (0,500 g) 1-Mesityl-3-undecylimidazoliumbromid in 12 mL Wasser und 5 mL Methanol gelöst und 1,30 mmol (0,380 g) Lithiumbis(trifluormethylsulfon)imid zugegeben.
**Summenformel:** C₂₅H₃₇F₆N₂O₄S₂ (621,70 g/mol)
**Ausbeute:** 0,450 g (60,8%)
**Schmelzpunkt:** 0 °C
**¹H**-**NMR (300 MHz, d₆-DMSO, ppm):**
δ=0.89 (t, J=6,4 Hz, 3 H, CH₂C*H₃*), 1.26 (bs, 16 H, Alkyl-C*H*₂), 1.96 (m, 2 H, NCH₂C*H₂*), 2.07 (s, 6 H, arom. *o-*C*H₃*), 2.38 (s, 3 H, *p*-C*H₃*), 4.27 (t, J=6,7 Hz, 2 H, NC*H₂*), 7.18 (s, 2 H, arom. *C*H), 7.92 (s, 1 H, N*C*HCHN), 8.12 (s, 1 H, NCH*C*HN), 9.42 (s, 1 H, NC*H*N).
**¹³C**-**NMR (75,5 MHz, d₆-DMSO, ppm):**
δ=13.9 (CH₂*C*H₃), 16.8 (*o*-*C*H₃), 20.5 (*p-C*H₃), 22.1 (C*H₂*CH₃), 25.4 (Alkyl-*C*H₂), 28.2 (Alkyl-*C*H₂), 28.7 (Alkyl-*C*H₂), 28.8 (Alkyl-*C*H₂), 28.8 (Alkyl-*C*H₂), 28.9 (Alkyl-*C*H₂), 29.0 (Alkyl-*C*H₂), 31.3 (NCH₂*C*H₂), 49.3 (N*C*H₂), 119.5 (q, J=320 Hz, CF₃), 123.2 (NCHCHN), 124.0 (NCHCHN), 129.2 (arom. CH), 131.1 (arom. *C1*), 134.3 (arom. C2 und C6), 137.2 (NCHN), 140.3 (arom. C4).
**¹⁹F-NMR (283 MHz, d₆-DMSO, ppm):**
δ=-78.7 ppm (C*F*₃).

### Ausführungsbeispiel 47

### 1-Mesityl-3-tetradecylimidazoliumbis(trifluormethylsulfon)imid

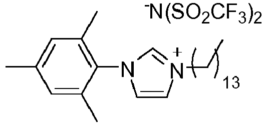

Es werden nach der allgemeinen Synthesevorschrift 1,08 mmol (0,500 g) 1-Mesityl-3-tetradecylimidazoliumbromid in 10 mL Wasser und 4 mL Methanol gelöst und 1,19 mmol (0,342 g) Lithiumbis(trifluormethylsulfon)imid zugegeben.
**Summenformel:** C₂₈H₄₃F₆N₃O₄S₂ (663,78 g/mol)
**Ausbeute:** 0,610 g (85,2%)
**Schmelzpunkt:** 29 °C
**¹H-NMR (300 MHz, d₆**-**DMSO**, **ppm):**
δ=0.86 (t, J=6,9 Hz, 3 H, CH₂C*H₃*), 1.24 (bs, 22 H, Alkyl-C*H₂*), 1.85 (m, 2 H, NCH₂C*H₂*), 2.02 (s, 6 H, arom. *o-*C*H₃*), 2.34 (s, 3 H, *p*-C*H₃*), 4.27 (t, J=7,0 Hz, 2 H, NC*H₂*), 7.16 (s, 2 H, arom. CH), 7.95 (s, 1 H, NC*H*CHN), 8.11 (s, 1 H, NCHCHN), 9.44 (s, 1 H, NCHN).
**¹³C**-**NMR (75,5 MHz, d₆-DMSO, ppm):**
δ=13.9 (CH₂*C*H₃), 16.8 (*o-C*H₃), 20.6 (*p-C*H₃), 21.8 (C*H₂*CH₃),22.1 (Alkyl-*C*H₂), 25.4 (Alkyl-*C*H₂), 28.2 (Alkyl-*C*H₂), 28.7 (Alkyl-*C*H₂), 28.8 (Alkyl-*C*H₂), 28.9 (Alkyl-*C*H₂), 29.0 (Alkyl-*C*H₂), 29.0 (Alkyl-*C*H₂), 31.3 (NCH₂*C*H₂), 49.3 (N*C*H₂), 119.5 (q, J=323 Hz, *C*F₃), 123.2 (NCHCHN), 124.0 (NCHCHN) 129.2 (arom. CH), 131.2 (arom. *C1*), 134.3 (arom. C2 und C6), 137.2 (NCHN), 140.3 (arom. C4).
**¹⁹F-NMR (283 MHz, d₆-DMSO, ppm):**
δ=-78.7 ppm (C*F₃*).
**Elementaranalyse:** C₂₈H₄₃F₆N₃O₄S₂ ber: C 50,67%, H 6,53%, N 6,33%, S 9,66%, gef.: C 50,88%, H 6,59%, N 6,13%, S 9,32%.

### Ausführungsbeispiel 48

### 1-(4-Bromphenyl)-3-propylimidazoliumbis(trifluormethylsulfon)imid

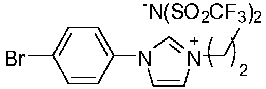

Es werden nach der allgemeinen Synthesevorschrift 1,44 mmol (0,500 g) 1-(4-Bromphenyl)-3-propylimidazoliumbromid in einer Mischung aus 12 mL Wasser und 12 mL Methanol gelöst und 1,59 mmol (0,460 g) Lithiumbis(trifluormethylsulfon)imid zugegeben.
**Summenformel:** C₁₄H₁₄BrF₆O₄S₂N₃ (546,30 g/mol)
**Ausbeute:** 0,720 g (91,3%)
**¹H-NMR (300 MHz, d₆-DMSO, ppm):**
δ=0.90 (t, J=7,4 Hz, 3 H, CH₂C*H₃*), 1.90 (qt, J=7,4 Hz, J=7,1 Hz, 2 H, NCH₂C*H₂*), 4.20 (t, J=7,2 Hz, 2 H, NC*H₂*), 7.78 (d, J=9,0 Hz, 2 H, arom. CH), 7.90 (d, J=9,1 Hz, 2 H, arom. CH), 8.10 (s, 1 H, NCHCHN), 8.35 (s, 1 H, NCHC*H*N), 9.82 (s, 1 H, NC*H*N).
**¹³C**-**NMR (75,5 MHz, d₆-DMSO, ppm):**
δ=10.5 (CH₂*C*H₃), 22.6 (NCH₂*C*H₂), 50.9 (N*C*H₂), 119.5 (q, J=321 Hz *C*F₃), 121.2 (NCHCHN), 122.6 (arom. C4), 123.3 (NCH*C*HN) 124.0 (arom. CH), 133.0 (arom. CH), 134.1 (arom. *C1*), 135.5 (N*C*HN).
**¹⁹F-NMR (283 MHz, d₆-DMSO, ppm):**
δ=-78.7 ppm (C*F₃*).
**Elementaranalyse:** C₁₄H₁₄BrF₆N₃O₄S₂ ber.: C 30,78%, H 2,58%, N 7,69%, S 11,74%, gef.: C 30,92%, H 2,41%, N 7,74%, S 11,49%.

### Ausführungsbeispiel 49

### 1-(4-Bromphenyl)-3-heptylimidazoliumbis(trifluormethylsulfon)imid

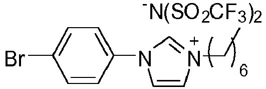

Es werden nach der allgemeinen Synthesevorschrift 1,24 mmol (0,500 g) 1-(4-Bromphenyl)-3-heptylimidazoliumbromid in einer Mischung aus 30 mL Wasser und 30 mL Methanol gelöst und bei einer Temperatur von 30 °C 1,37 mmol (0,390 g) Lithiumbis(trifluormethylsulfon)imid zugegeben.
**Summenformel:** C₁₈H₂₂BrF₆N₃O₄S₂ (602,40 g/mol)
**Ausbeute:** 0,690 g (92,0%)
**¹H-NMR (300 MHz, d₆-DMSO, ppm):**
δ=0.84 (t, J=6,8 Hz, 3 H, CH₂C*H₃*), 1.30 (m, 8 H, Alkyl-C*H₂*), 1.88 (m, 2 H, NCH₂C*H₂*), 4.20 (t, J=7,3 Hz, 2 H, NC*H₂*), 7.75 (d, J=8,9 Hz, 2 H, arom. CH), 7.90 (d, J=8,9 Hz, 2 H, arom. CH), 8.04 (s, 1 H, NC*H*CHN), 8.33 (s, 1 H, NCHC*H*N), 9.90 (s, 1 H, NC*H*N).
**¹³C**-**NMR (75,5 MHz, d₆-DMSO, ppm):**
δ=13.9 (CH₂*C*H₃), 22.0 (Alkyl-*C*H₂), 25.5 (Alkyl-*C*H₂), 28.0 (Alkyl-*C*H₂), 29.1 (Alkyl-*C*H₂), 31.0 (NCH₂*C*H₂), 49.4 (N*C*H₂), 119.5 (q, J=322 Hz *C*F₃), 121.1 (N*C*HCHN), 122.6 (arom. C4), 123.3 (NCHCHN), 123.9 (arom. *C*H), 133.0 (arom. CH), 134.1 (arom. *C1*), 135.5 (N*C*HN).
**¹⁹F-NMR (283 MHz, d₆-DMSO, ppm):**
δ=-78.7 ppm (C*F₃*).
**Elementaranalyse:** C₁₈H₂₂BrF₆N₃O₄S₂ ber.: C 35,89%, H 3,68%, N 6,98%, S 10,64%, gef.: C 35,74%, H 3,73%, N7,07%, S 10,76%.

### Ausführungsbeispiel 50

### 1-(4-Bromphenyl)-3-tetradecylimidazoliumbis(trifluormethylsulfon)imid

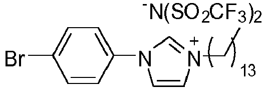

Es werden nach der allgemeinen Synthesevorschrift 1,0 mmol (0,500 g) 1-(4-Bromphenyl)-3-tetradecylimidazoliumbromid in einer Mischung aus 26 mL Wasser und 26 mL Methanol gelöst und bei einer Temperatur von 40 °C werden 1,1 mmol (0,320 g) Lithiumbis(trifluormethylsulfon)imid zugegeben.
**Summenformel:** C₂₅H₃₆BrF₆N₃O₄S₂ (700,59 g/mol)
**Ausbeute:** 0,63 g (90,0%)
**Schmelzpunkt:** 32 °C
**¹H**-**NMR (300 MHz, d₆**-**DMSO**, **ppm):**
δ=0.88 (t, J=6,8 Hz, 3 H, CH₂C*H₃*), 1.20-1.35 (m, 22 H, Alkyl-CH₂), 1.89 (m, 2 H, NCH₂C*H₂*), 4.22 (t, J=7,2 Hz, 2 H, NC*H₂*), 7.77 (d, J=8,9 Hz, 2 H, arom. *C*H), 7.90 (d, J=8,9 Hz, 2 H, arom. CH), 8.04 (s, 1 H, NC*H*CHN), 8.33 (s, 1 H, NCHC*H*N), 9.82 (s, 1 H, NC*H*N).
**¹³C**-**NMR (75,5 MHz, d₆-DMSO, ppm):**
δ=13.9 (CH₂*C*H₃), 22.1 (Alkyl-*C*H₂), 25.5 (Alkyl-*C*H₂), 28.4 (Alkyl-*C*H₂), 28.7 (Alkyl-*C*H₂), 28.8 (Alkyl-*C*H₂), 28.9 (Alkyl-*C*H₂), 29.0 (Alkyl-*C*H₂), 29.0 (Alkyl-*C*H₂), 31.3 (NCH₂*C*H₂), 49.4 (N*C*H₂), 119.5 (q, J=322 Hz *C*F₃), 121.1 (N*C*HCHN), 122.6 (arom. C4), 123.3 (NCH*C*HN), 123.9 (arom. *C*H), 133.0 (arom. CH), 134.1 (arom. *C1*), 135.5 (N*C*HN).
¹⁹**F-NMR (283 MHz, d₆-DMSO, ppm):**
δ=-78.7 ppm (C*F₃*).
**Elementaranalyse:** C₂₅H₃₆BrF₆N₃O₄S₂ ber.: C 42,86%, H 5,18%, N 6,00%, S 9,15%, gef.: C42,93%, H 5,12%, N 6,05%, S 9,22%.

### Ausführungsbeispiel 51

### 1-(4-Chlorphenyl)-3-propylimidazoliumbis(trifluormethylsulfon)imid

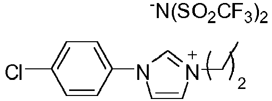

Es werden nach der allgemeinen Synthesevorschrift 1,7 mmol (0,500 g) 1-(4-Chlorphenyl)-3-propylimidazoliumbromid in einer Mischung aus 5 mL Wasser und 2 mL Methanol gelöst und 1,8 mmol (0,520 g) Lithiumbis(trifluormethylsulfon)imid zugegeben.
**Summenformel:** C₁₄H₁₄C1F₆N₃O₄S₂ (501,85 g/mol)
**Ausbeute:** 0,760 g (91,6%)
**¹H-NMR (300 MHz, d₆-DMSO, ppm):**
δ=0.86 (t, J=7,4 Hz, 3 H, CH₂C*H₃*), 1.83 (qt, J=7,3 Hz, J=7,1 Hz, 2 H, NCH₂C*H₂*), 4.14 (t, J=7,1 Hz, 2 H, NC*H₂*), 7.67 (d, J=9,1 Hz, 2 H, arom. *C*H), 7.82 (d, J=9,1 Hz, 2 H, arom. CH), 7.96 (s, 1 H, NC*H*CHN), 8.25 (s, 1 H, NCHC*H*N), 9.74 (s, 1 H, NC*H*N).
**¹³C-NMR (75,5 MHz, d₆-DMSO, ppm):**
δ=10.4 (CH₂*C*H₃), 22.6 (NCH₂*C*H₂), 50.9 (N*C*H₂), 118.5 (q, *C*F₃) 121.2 (N*C*HCHN), 123.3 (NCH*C*HN) 123.8 (arom. *C*H), 130.0 (arom. *C*H), 133.6 (arom. *C1*), 134.2 (arom. *C*4), 135.56 (N*C*HN).
**¹⁹F-NMR (283 MHz, d₆-DMSO, ppm):**
δ=-78.7 ppm (C*F₃*)
**Elementaranalyse:** C₁₄H₁₄C1F₆N₃O₄S₂ ber.: C 33,51%, H 2,81%, N 8,37%, S 12,78%, gef.: C 33,57%, H 2,45%, N 8,30%, S 12,96%.

### Ausführungsbeispiel 52

### 1-(4-Chlorphenyl)-3-heptylimidazoliumbis(trifluormethylsulfon)imid

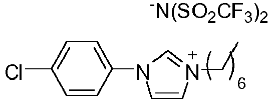

Es werden nach der allgemeinen Synthesevorschrift 1,4 mmol (0,500 g) 1-(4-Chlorphenyl)-3-heptylimidazoliumbromid in einer Mischung aus 10 mL Wasser und 7 mL Methanol gelöst und 1,5 mmol (0,440 g) Lithiumbis(trifluormethylsulfon)imid zugegeben.
**Summenformel:** C₁₈H₂₂C1F₆N₃O₄S₂ (557,95 g/mol)
**Ausbeute:** 0,733 g (94,0%)
**¹H-NMR (300 MHz, d₆-DMSO, ppm):**
δ=0.89 (t, J=6,8 Hz, 3 H, CH₂C*H₃*), 1.38 (m, 8 H, Alkyl-C*H₂*), 1.90 (m, 2 H, NCH₂C*H₂*), 4.24 (t, J=7,3 Hz, 2 H, NC*H₂*), 7.79 (d, J=9,1 Hz, 2 H, arom. *C*H), 7.85 (d, J=9,1 Hz, 2 H, arom. *C*H), 8.06 (s, 1 H, NC*H*CHN), 8.34 (s, 1 H, NCHC*H*N), 9.83 (s, 1 H, NC*H*N).
**¹³C**-**NMR (75,5 MHz, d₆-DMSO, ppm):**
δ=13.9 (CH₂*C*H₃), 22.0 (Alkyl-*C*H₂), 25.5 (Alkyl-*C*H₂), 28.0 (Alkyl-*C*H₂), 29.1 (Alkyl-*C*H₂), 31.0 (NCH₂*C*H₂), 49.4 (N*C*H₂), 119.5 (q, *C*F₃), 121.2 (N*C*HCHN), 123.3 (NCH*C*HN), 123.7 (arom. *C*H), 130.1 (arom. CH), 133.7 (arom. *C*4), 134.2 (arom. *C1*), 135.5 (N*C*HN).
**¹⁹F-NMR (283 MHz, d₆-DMSO, ppm):**
δ=-78.7 ppm (C*F₃*)
**Elementaranalyse:** C₁₈H₂₂ClF₁F₆N₃O₄S₂ ber.: C 38,75%, H 3,97%, N 7,53%, S 11,49%, gef.: C 38,89%, H 3,93%, N 7,74%, S 11,74%.

### Ausführungsbeispiel 53

### 1-(4-Chlorphenyl)-3-tetradecylimidazoliumbis(trifluormethylsulfon)imid

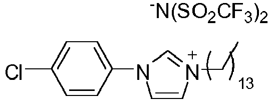

Es werden nach der allgemeinen Synthesevorschrift 1,1 mmol (0,500 g) 1-(4-Chlorphenyl)-3-tetradecylimidazoliumbromid in einer Mischung aus 30 mL Wasser und 34 mL Methanol gelöst und unter Erwärmen auf 35 °C 1,2 mmol (0,350 g) Lithiumbis(trifluormethylsulfon)imid zugegeben.
**Summenformel:** C₂₅H₃₆ClF₆N₃O₄S₂ (656,14 g/mol)
**Ausbeute:** 0,620 g (86,1%)
**Schmelzpunkt:** 36 °C
**¹H-NMR (300 MHz, d₆-DMSO, ppm):**
δ=0.90 (t, J=6,5 Hz, 3 H, CH₂C*H₃*), 1.25 (m, 22 H, Alkyl-CH₂), 1.85 (m, 2 H, NCH₂C*H₂*), 4.21 (t, J=7,2 Hz, 2 H, NC*H₂*), 7.80 (d, J=8,8 Hz, 2 H, arom. *C*H), 7.85 (d, J=8,9 Hz, 2 H, arom. *C*H), 8.02 (s, 1 H, NC*H*CHN), 8.34 (s, 1 H, NCHC*H*N), 9.84 (s, 1 H, NC*H*N)
**¹³C**-**NMR (75,5 MHz, d₆-DMSO, ppm):**
δ=13.9 (CH₂*C*H₃), 22.1 (Alkyl-*C*H₂), 25.5 (Alkyl-*C*H₂), 28.4 (Alkyl-*C*H₂), 28.7 (Alkyl-*C*H₂), 28.8 (Alkyl-*C*H₂), 28.9 (Alkyl-*C*H₂), 29.0 (Alkyl-*C*H₂), 29.0 (arom. *C*H₂), 31.3 (NCH₂*C*H₂), 49.4 (N*C*H₂), 121.2 (N*C*HCHN), 123.3 (NCH*C*HN), 123.7 (arom. *C*H), 130.1 (arom. CH), 133.7 (arom. C4), 134.2 (arom. *C1*), 135.5 (N*C*HN).
**¹⁹F-NMR (283 MHz, d₆-DMSO, ppm):**
δ=-78.7 ppm (C*F₃*).
**Elementaranalyse:** C₂₅H₃₆ClF₆N₃O₄S₂ ber.: C 45,76% H 5,73% N 6,40% S 9,77%, gef.: C 45,89% H 5,73%, N 6,41%, S 9,86%.

### Ausführungsbeispiel 54

### 1-(4-Ethylcarboxyphenyl)-3-propylimidazoliumbis(trifluormethylsulfon)imid

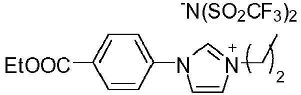

Es werden nach der allgemeinen Synthesevorschrift 1,47 mmol (0,500 g) 1-(4-Ethylcarboxyphenyl)-3-propylimidazoliumbromid in einer Mischung aus 20 mL Wasser und 4 mL Methanol gelöst und 1,62 mmol (0,470 g) Lithiumbis(trifluormethylsulfon)imid zugegeben.
**Summenformel:** C₁₇H₁₉F₆N₃O₆S₂ (539,47 g/mol)
**Ausbeute:** 0,730 g (92,1%)
**¹H-NMR (300 MHz, d₆-DMSO, ppm):**
δ=0.98 (t, J=7,3 Hz, 3 H, CH₂CH₂C*H₃*), 1.45 (t, J=7,3 Hz, 3 H, OCH₂C*H₃*), 1.98 (hept, J=7.3 Hz, 2 H, NCH₂C*H₂*), 4.23 (t, J=7,3 Hz, 2 H, NC*H₂*), 4.48 (q, J=7,1 Hz, 2 H, OC*H*₂), 8.00 (d, J=8,8 Hz, 2 H, arom. *C*H), 8.11 (s, 1 H, NC*H*CHN), 8.27 (d, J=8,8 Hz, 2 H, arom. *C*H), 8.48 (s, 1 H, NCH*C*HN), 9.99 (s, 1 H, NC*H*N).
**¹³C-NMR (75,5 MHz, d₆-DMSO, ppm):**
δ=10.5 (CH₂CH₂*C*H₃), 14.1 (OCH₂*C*H₃), 22.6 (Alkyl-*C*H₂), 51.0 (N*C*H₂), 61.3 (CH₂), 119.5 (q, J=322 Hz *C*F₃), 121.0 (N*C*HCHN), 121.6 (arom. *C*H), 123.5 (NCH*C*HN), 130.7 (arom. *C1*), 131.0 (arom. *C*H), 135.8 (N*C*HN), 138.1 (arom. C4), 164.6 (COO).
**¹⁹F-NMR (283 MHz, d₆-DMSO, ppm):**
δ=-78.7 ppm (C*F*₃).
**Elementaranalyse:** C₁₇H₁₉F₆N₃O₆S₂ ber.: C 37,85%, H 3,55%, N 7,79%, S 11,89%, gef.: C 38,07%, H 3,20%, N 7,92%, S 11,74%.

### Ausführungsbeispiel 55

### 1-(4-Ethylcarboxyphenyl)-3-heptylimidazoliumbis(trifluormethylsulfon)imid

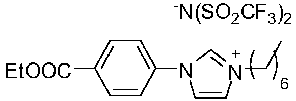

Es werden nach der allgemeinen Synthesevorschrift 1,47 mmol (0,500 g) 1-(4-Ethylcarboxyphenyl)-3-heptylimidazoliumbromid in einer Mischung aus 15 mL Wasser und 9 mL Methanol gelöst und 1,39 mmol (0,399 g) Lithiumbis(trifluormethylsulfon)imid zugegeben.
**Summenformel:** C₂₁H₂₇F₆N₃O₆S₂ (595,57 g/mol)
**Ausbeute:** 0,690 g (92,0%)
**¹H-NMR (300 MHz, d₆-DMSO, ppm):**
δ=0.87 (t, J=6,9 Hz, 3 H, CH₂CH₂C*H₃*), 1.35 (m, 11 H, Alkyl-C*H*₂ und OCH₂C*H₃*), 1.87 (m, 2 H, NCH₂C*H₂*), 4.25 (t, J=7,3 Hz, 2 H, NC*H₂*), 4.36 (q, J=7,2 Hz, 2 H, OC*H₂*), 7.97 (d, J=8,7 Hz, 2 H, arom. C*H*), 8.10 (s, 1 H, NC*H*CHN), 8.22 (d, J=8,7 Hz, 2 H, arom. CH), 8.41 (s, 1 H, NCHC*H*N), 9.93 (s, 1 H, NC*H*N).
**¹³C**-**NMR (75,5 MHz, d₆**-**DMSO**, **ppm):**
δ=13.8 (CH₂CH₂*C*H₃), 14.1 (OCH₂*C*H₃), 22.0 (Alkyl-*C*H₂), 25.5 (Alkyl-*C*H₂), 28.0 (Alkyl-*C*H₂), 29.1 (Alkyl-*C*H₂), 31.0 (NCH₂*C*H₂), 49.5 (N*C*H₂), 61.3 (O*C*H₂), 119.5 (q, J=322 Hz *C*F₃),120.9 (N*C*HCHN), 121.9 (arom. *C*H), 123.5 (NCH*C*HN), 130.7 (arom. *C1*), 131.0 (arom. *C*H), 135.8 (N*C*HN), 138.1 (arom. C4), 164.6 (*C*OO).
**¹⁹F-NMR (283 MHz, d₆-DMSO, ppm):**
δ=-78.7 ppm (C*F₃*).
**Elementaranalyse:** C₂₁H₂₇F₆N₃O₆S₂ ber.: C 42,35%, H 4,57%, N 7,06%,S 10,77%, gef.: C 42,52%, H 4,34%, N 7,17%, S 10,42%.

### Ausführungsbeispiel 56

### 1-(4-Ethylcarboxyphenyl)-3-tetradecylimidazoliumbis(trifluormethylsulfon)imid

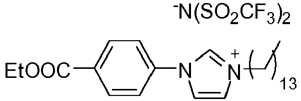

Es werden nach der allgemeinen Synthesevorschrift 1,01 mmol (0,500 g) 1-(4-Ethylcarboxyphenyl)-3-tetradecylimidazoliumbromid in einer Mischung aus 20 mL Wasser und 20 mL Methanol gelöst und 1,11 mmol (0,320 g) Lithiumbis(trifluormethylsulfon)imid zugegeben.
**Summenformel:** C₂₈H₄,F₆N₃O₆S₂ (693,76 g/mol)
**Ausbeute:** 0,56 g (80,0%)
**¹H-NMR (300 MHz, d₆-DMSO, ppm):**
δ=0.85 (t, J=6,9 Hz, 3 H, CH₂CH₂C*H₃*), 1.25 (bs, 22 H, Alkyl-C*H₂*),1.41 (t, J=7,0 Hz, 3 H, OCH₂C*H₃*), 1.92 (m, 2 H, NCH₂C*H₂*), 4.27 (t, J=7,2 Hz, 2 H, NC*H₂*), 4.41 (q, J=7,0 Hz, 2 H, OC*H₂*), 7.97 (d, J=8,8 Hz, 2 H, arom. *C*H), 8.10 (s, 1 H, NC*H*CHN), 8.23 (d, J=8,8 Hz, 2 H, arom. *C*H), 8.45 (s, 1 H, NCHC*H*N), 9.96 (s, 1 H, NC*H*N).
**¹³C**-**NMR (75,5 MHz, d₆-DMSO, ppm):**
δ=13.9 (CH₂CH₂*C*H₃), 14.1 (OCH₂*C*H₃), 22.1 (Alkyl-*C*H₂), 25.5 (Alkyl-*C*H₂), 28.4 (Alkyl-*C*H₂), 28.7 (Alkyl-*C*H₂), 28.8 (Alkyl-*C*H₂), 28.9 (Alkyl-*C*H₂), 29.0 (Alkyl-*C*H₂), 29.0 (Alkyl-*C*H₂), 31.3 (NCH₂*C*H₂), 49.5 (N*C*H₂), 61.3 (OCH₂), 119.5 (q, J=322 Hz *C*F₃), 120.9 (N*C*HCHN), 121.9 (arom. CH), 123.5 (NCH*C*HN), 130.7 (arom. *C1*), 131.0 (arom. *C*H), 135.8 (N*C*HN), 138.1 (arom. C4), 164.6 (*C*OO).
**¹⁹F-NMR (283 MHz, d₆-DMSO, ppm):**
δ=-78.7 ppm (C*F₃*).

### Ausführungsbeispiel 57

### 1-(4-Nitrophenyl)-3-propylimidazolium-bis(trifluormetylsulfon)imid

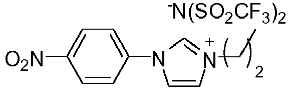

Es werden nach der allgemeinen Synthesevorschrift 1,24 mmol (0,400 g) 1-(4-Nitrophenyl)-3-propylimidazoliumbromid in einer Mischung aus 4 mL Wasser und 1 mL Methanol gelöst und 1,40 mmol (0,406 g) Lithiumbis(trifluormetylsulfon)imid zugegeben.
**Summenformel:** C₁₄H₁₄F₆N₄O₆S₂ (312,17 g/mol)
**Ausbeute:** 0,560 g (84,8%)
**Schmelzpunkt:** -43°C
**¹H-NMR (300 MHz, d₆-DMSO, ppm):**
δ=0.90 (t, J=7,4 Hz, 3 H, CH₂C*H₃*), 1.93 (qt, J=7,4 Hz, J=7,1 Hz, 2 H, NCH₂C*H*₂), 4.25 (t, J₂=7,1 Hz, 2 H, NC*H₂*), 8.08 (d, J=8,2 Hz, 2 H, arom. *C*H), 8.12 (s, 1 H, N*C*HCHN), 8.48 (s, 1 H, NCH*C*HN), 8.52 (d, J=8,2 Hz, 2 H, arom. CH), 9.97 (s, 1 H, NC*H*N)
**¹³C**-**NMR (75,5 MHz, d₆-DMSO, ppm):**
δ=10.5 (CH₂*C*H₃), 22.5 (NCH₂*C*H₂), 51.1 (N*C*H₂), 119.5 (q, J=322 Hz *C*F₃), 121.1 (NCHCHN), 122.9 (arom. *C*H), 123.6 (NCH*C*HN), 125.6 (arom. *C*H), 136.2 (N*C*HN), 139.3 (arom. *C1*), 147.6 (arom. *C*4)
**¹⁹F-NMR (283 MHz, d₆-DMSO, ppm):**
δ=-78.7 ppm (C*F₃*)

### Ausführungsbeispiel 58

### 1-(4-Nitrophenyl)-3-heptylimidazolium bis(trifluormetylsulfon)imid

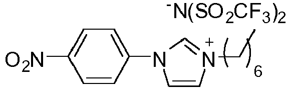

Es werden nach der allgemeinen Synthesevorschrift 1,4 mmol (0,500 g) 1-(4-Nitrophenyl)-3-heptylimidazoliumbromid in einer Mischung aus 20 mL Wasser und 20 mL Methanol gelöst und 1,50 mmol (0,430 g) Lithiumbis(trifluormetylsulfon)imid zugegeben.
**Summenformel:** C₁₈H₂₂F₆N₄O₆S₂ (368,27 g/mol)
**Ausbeute:** 0,630 g (81,8%)
**Schmelzpunkt:** -45 °C
**¹H-NMR (300 MHz, d₆-DMSO, ppm):**
δ=0.91 (t, J=6,7 Hz, 3 H, CH₂C*H₃*), 1.20-1.50 (m, 8 H, Alkyl-CH₂), 1.96 (m, 2 H, NCH₂C*H₂*), 4.31 (t, J=7,3 Hz, 2 H, NC*H₂*), 8.14 (d, J=8,7 Hz, 2 H, arom. *C*H), 8.15 (s, 1 H, NC*H*CHN), 8.49 (s, 1 H, NCHC*H*N), 8.62 (d, J=8,8 Hz, 2 H, arom. CH), 10.04 (s, 1 H, NC*H*N)
**¹³C**-**NMR (75,5 MHz, d₆-DMSO, ppm):**
δ=13.9 (CH₂*C*H₃), 22.0, 25.4, 28.1, 29.0 (Alkyl *C*H₂), 31.0 (NCH₂*C*H₂), 49.6 (N*C*H₂), 119.5 (q, J=322 Hz *C*F₃), 121.6 (N*C*HCHN), 122.9 (arom. CH), 123.6 (NCH*C*HN), 125.5 (arom. CH), 136.2 (N*C*HN), 139.3 (arom. *C*1), 147.5 (arom. *C4)*
**¹⁹F-NMR (283 MHz, d₆-DMSO, ppm):**
δ=-78.7 ppm (C*F*₃)

### Allgemeine Synthesevorschrift für Imidazoliumsalze mit Tetrafluoroborat-Anionen

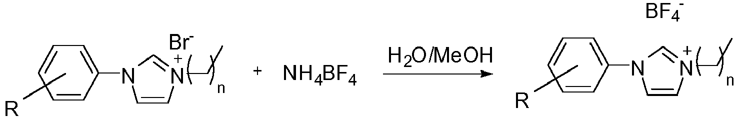

1,0 eq. des Imidazoliumbromid-Salzes wird in Wasser oder einer Wasser/Methanol Mischung vollständig gelöst. Unter ständigem Rühren wird NH₄BF₄ zugegeben. Dabei bilden sich in der Reaktionsmischung nach wenigen Minuten zwei Phasen aus. Zur Vervollständigung der Reaktion wird für weitere 15 Minuten gerührt. Anschließend werden zu der Reaktionsmischung 15 mL Dichlormethan gegeben und dann in einem Scheidetrichter die organische Phase von der wässrigen Phase abgetrennt. Die wässrige Phase wird zwei weitere Male mit je 10 mL Dichlormethan extrahiert. Die organischen Phasen werden vereinigt, über Magnesiumsulfat getrocknet und das Lösungsmittel im Vakuum entfernt.

### Ausführungsbeispiel 59

### 1-Butyl-3-mesitylimidazoliumtetrafluoroborat

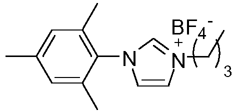

Es werden nach der allgemeinen Synthesevorschrift 0,93 mmol (0,300 g) 1-Butyl-3-mesitylimidazoliumbromid in 2 mL Wasser gelöst und 1,02 mmol (0,110 g, 1,1 eq.) Ammoniumtetrafluoroborat zugegeben.
**Summenformel:** C₁₆H₂₃BF₄N₂ (330,18 g/mol)
**Ausbeute:** 0,284 g (92,8%)
**Schmelzpunkt:** 92 °C
**¹H-NMR (500 MHz, d₆-DMSO, ppm):**
δ=0.94 (t, J=7,2 Hz, 3 H, CH₂C*H₃*), 1.28 (dq, J=7,2 Hz, J=7,4 Hz, 2 H, C*H₂*CH₃), 1.88 (p, J=7,4 Hz, 2 H, NCH₂CH₂), 2.02 (s, 6 H, arom. *o-*C*H₃*), 2.34 (s, 3 H, *p*-C*H₃*), 4.28 (t, J=7,1 Hz, 2 H, NC*H₂*CH₃), 7.16 (s, 2 H, arom. *C*H), 7.95 (s, 1 H, N*C*HCHN), 8.11 (s, 1 H, NCHC*H*N), 9.43 (s, 1 H, NC*H*N).
**¹³C**-**NMR (125,8 MHz, d₆-DMSO, ppm):**
δ=13.2 (CH₂*C*H₃), 16.8 (*o*-*C*H₃),18.7 (*C*H₂CH₃), 20.6 (*p-C*H₃), 31.0 (NCH₂*C*H₂), 49.1 (N*C*H₂CH₃), 123.2 (N*C*HCHN), 124.0 (NCH*C*HN), 129.2 (arom. *C*H), 131.2 (arom. *C1*), 134.3 (arom. *C*2 und *C*6), 137.2 (NCHN), 140.3 (arom. *C*4).
**¹⁹F-NMR (283 MHz, d₆-DMSO, ppm):**
δ=-148.3 ppm (B*F₄⁻*).
**Elementaranalyse:** C₁₆H₂₃BF₄N₂ ber.: C 58,20%, H 7,02%, N 8,48%, gef.: C 58, 31%, H 7,01%, N 8,43%.

### Ausführungsbeispiel 60

### 1-Mesityl-3-pentylimidazoliumtetrafluoroborat

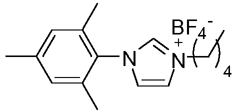

Es werden nach der allgemeinen Synthesevorschrift 1,48 mmol (0,500 g) 1-Mesityl-3-pentylimidazoliumbromid in 5 mL Wasser gelöst und 1,63 mmol (0,171 g, 1,1 eq.) Ammoniumtetrafluoroborat zugegeben.
**Summenformel:** C₁₇H₂₅BF₄N₂ (344,20 g/mol)
**Ausbeute:** 0,461 g (90,4%)
**Schmelzpunkt:** 100 °C
**¹H-NMR (300 MHz, d₆-DMSO, ppm):**
δ=0.89 (t, J=7,3 Hz, 3 H, CH₂C*H₃*), 1.20-1.42 (m, 4 H, C*H₂*CH₂), 1.93 (p, J=7,2 Hz, 2 H, NCH₂C*H*₂), 2.02 (s, 6 H, arom. *o-*C*H₃*), 2.34 (s, 3 H, *p*-C*H₃*), 4.27 (t, J=7,1 Hz, 2 H, NC*H₂*), 7.16 (s, 2 H, arom. *C*H), 7.95 (s, 1 H, NC*H*CHN), 8.11 (s, 1 H, NCHC*H*N), 9.43 (s, 1 H, NC*H*N).
**¹³C-NMR (75,5 MHz, d₆-DMSO, ppm):**
δ=13.8 (CH₂*C*H₃), 16.8 (*o*-*C*H₃), 20.6 (*p-C*H₃), 21.4 (Alkyl-*C*H₂), 27.6 (Alkyl-CH₂), 28.7 (NCH₂*C*H₂), 49.3 (N*C*H₂CH₃), 123.2 (N*C*HCHN), 124.0 (NCH*C*HN) 129.2 (arom. *C*H), 131.2 (arom. *C1*), 134.3 (arom. *C*2 und *C*6), 137.2 (N*C*HN), 140.3 (arom. *C*4).
**¹⁹F-NMR (283 MHz, d₆-DMSO, ppm):**
δ=-148.2 ppm (B*F₄⁻*).
**Elementaranalyse:** C₁₇H₂₅BF₄N₂ ber.: C 59,32%, H 7,32%, N 8,14%, gef.: C 59,34%, H 7,38%, N 8,15%.

### Ausführungsbeispiel 61

### 1-Hexyl-3-mesitylimidazoliumtetrafluoroborat

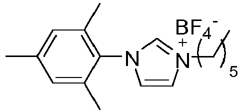

Es werden nach der allgemeinen Synthesevorschrift 1,37 mmol (0,500 g) 1-Hexyl-3-mesitylimidazoliumbromid und 1,57 mmol (0,164 g, 1,1 eq.) Ammoniumtetrafluoroborat in einer Mischung aus 7 mL Wasser und 2 mL Methanol gelöst und bei Raumtemperatur gerührt.
**Summenformel:** C₁₈H₂₇BF₄N₂ (358,23 g/mol)
**Ausbeute:** 0,477 g (93,5%)
**Schmelzpunkt:** 74 °C
**¹H-NMR (300 MHz, d₆-DMSO, ppm):**
δ=0.86 (t, J=6,8 Hz, 3 H, CH₂C*H₃*), 1.29-1.35 (m, 6 H, C*H₂*C*H₂*C*H₂*),1.89-1.92 (m, 2 H, NCH₂C*H*₂), 2.02 (s, 6 H, arom. *o-*C*H₃*), 2.34 (s, 3 H, *p*-C*H₃*), 4.28 (t, J=7,1 Hz, 2 H, NC*H₂*), 7.16 (s, 2 H, arom. *C*H), 7.95 (s, 1 H, NC*H*CHN), 8.11 (s, 1 H, NCHC*H*N), 9.43 (s, 1 H, NC*H*N).
**¹³C**-**NMR (75,5 MHz, d₆-DMSO, ppm):**
δ=13.7 (CH₂*C*H₃), 16.8 (*o-C*H₃), 20.6 (*p-C*H₃), 21.9 (Alkyl-*C*H₂), 25.0 (Alkyl-*C*H₂), 28.9 (Alkyl-*C*H₂), 30.4 (Alkyl-CH₂), 30.4 (NCH₂*C*H₂), 49.3 (N*C*H₂CH₃), 123.2 (N*C*HCHN), 124.0 (NCH*C*HN), 129.2 (arom. *C*H), 131.2 (arom. *C1*), 134.3 (arom. *C*2 und *C*6), 137.2 (N*C*HN), 140.3 (arom. *C*4).
**¹⁹F-NMR (283 MHz, d₆-DMSO, ppm):**
δ=-148.2 ppm (B*F₄⁻*)
**Elementaranalyse:** C₁₈H₂₇BF₄N₂ ber.: C 60,35%, H 7,60%, N 7,82%, gef.: C 60,52% ,H 7,80%, N 7,94%.

### Ausführungsbeispiel 62

### 3-Mesityl-1-octyl-imidazoliumtetrafluoroborat

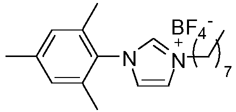

Es werden nach der allgemeinen Synthesevorschrift 1,31 mmol (0,500 g) 1-Mesityl-3-octylimidazoliumbromid in 8 mL Wasser und 4 mL Methanol gelöst und 3,96 mmol (0,415 g, 3 eq.) Ammoniumtetrafluororborat zugegeben.
**Summenformel:** C₂₀H₃₁BF₄N₂ (386,28 g/mol)
**Ausbeute:** 0,498 g (98,0%)
**Schmelzpunkt:** -18 °C
**¹H-NMR (300 MHz, d₆-DMSO, ppm):**
δ=0.88 (t, J=6,5 Hz, 3 H, CH₂C*H₃*), 1.28 (m, 10 H, Alkyl-C*H₂*),1.90 (m, 2 H, NCH₂C*H₂*), 2.03 (s, 6 H, *o*-C*H₃*), 2.34 (s, 3 H, *p*-C*H₃*), 4.28 (t, J=7,0 Hz, 2 H, NC*H₂*), 7.16 (s, 2 H, arom. C*H*), 7.95 (s, 1 H, NC*H*CHN), 8.10 (s, 1 H, NCHC*H*N), 9.44 (s, 1 H, NC*H*N).
**¹³C-NMR (75,5 MHz, d₆-DMSO, ppm):**
δ=13.9 (CH₂*C*H₃), 16.8 (*o*-*C*H₃), 20.6 (*p-C*H₃), 22.0 (Alkyl-*C*H₂), 25.4 (Alkyl-*C*H₂), 28.2 (Alkyl-*C*H₂), 28.5 (Alkyl-*C*H₂), 29.0, (Alkyl-CH₂), 31.1 (NCH₂*C*H₂), 49.3 (N*C*H₂CH₃), 123.2 (N*C*HCHN), 124.0 (NCH*C*HN) 129.2 (arom. *C*H), 131.2 (arom. *C1*), 134.3 (arom. *C*2 und *C*6), 137.2 (N*C*HN), 140.3 (arom. *C*4).
**¹⁹F-NMR (283 MHz, d₆-DMSO, ppm):**
δ=-148.3 ppm (B*F₄⁻*).
**Elementaranalyse:** C₂₀H₃₁BF₄N₂ ber.: C 62,19%, H 8,03%, N 7,25%, gef.: C62,24%, H 8,05%, N 7,28%.

### Ausführungsbeispiel 63

### 1-Mesityl-3-undecylimidazoliumtetrafluoroborat

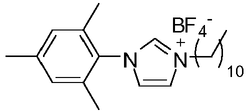

Es werden nach der allgemeinen Synthesevorschrift 1,19 mmol (0,500 g) 1-Mesityl-3-undecylimidazoliumbromid in 11 mL Wasser und 4 mL Methanol gelöst und 3,54 mmol (0,370 g, 3 eq.) Ammoniumtetrafluororborat zugegeben.
**Summenformel:** C₂₃H₃₇BF₄N₂ (428,36 g/mol)
**Ausbeute:** 0,472 g (92,9%)
**Schmelzpunkt:** -49 °C
**¹H-NMR (300 MHz, d₆-DMSO, ppm):**
δ=0.86 (t, J=6,4 Hz, 3 H, CH₂C*H₃*), 1.25 (bs, 16 H, Alkyl-C*H₂*), 1.96 (m, 2 H, NCH₂C*H₂*), 2.02 (s, 6 H, arom. *o-*C*H₃*), 2.34 (s, 3 H, *p*-C*H₃*), 4.33 (t, J=7,0 Hz, 2 H, NC*H₂*), 7.16 (s, 2 H, arom. CH), 7.95 (s, 1 H, NC*H*CHN), 8.11 (s, 1 H, NCHCHN), 9.43 (s, 1 H, N*C*HN).
**¹³C**-**NMR (75,5 MHz, d₆-DMSO, ppm):**
δ=13.9 (CH₂*C*H₃), 16.8 (*o-C*H₃), 20.6 (*p-C*H₃), 22.1 (C*H₂*CH₃), 25.4 (Alkyl-*C*H₂), 28.2 (Alkyl-*C*H₂), 28.7 (Alkyl-*C*H₂), 28.8 (Alkyl-*C*H₂), 28.8 (Alkyl-*C*H₂), 28.9 (Alkyl-*C*H₂), 29.0 (Alkyl-*C*H₂), 31.3 (NCH₂*C*H₂), 49.3 (N*C*H₂), 123.2 (N*C*HCHN), 124.0 (NCH*C*HN), 129.2 (arom. *C*H), 131.1 (arom. *C1*), 134.2 (arom. *C*2 und *C*6), 137.2 (N*C*HN), 140.3 (arom. *C*4).
**¹⁹F-NMR (283 MHz, d₆-DMSO, ppm):**
δ=-148.3 ppm (B*F₄⁻*).

### Ausführungsbeispiel 64

### 1-Mesityl-3-tetradecylimidazoliumtetrafluoroborat

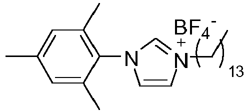

Es werden nach der allgemeinen Synthesevorschrift 1,08 mmol (0,500 g) 1-Mesityl-3-tetradecylimidazoliumbromid in 9 mL Wasser und 3 mL Methanol gelöst und 1,19 mmol (0,125 g) Ammoniumtetrafluoroborat zugegeben.
**Summenformel:** C₂₆H₄₃BF₄N₂ (470,44 g/mol)
**Ausbeute:** 0,456 g (89,9%)
**Schmelzpunkt:** 57 °C
**¹H-NMR (300 MHz, d₆-DMSO, ppm):**
δ=0.86 (t, J=7,1 Hz, 3 H, CH₂C*H₃*), 1.25 (bs, 22 H, Alkyl-C*H₂*), 1.89 (m, 2 H, NCH₂C*H₂*), 2.02 (s, 6 H, arom. *o-*C*H₃*), 2.34 (s, 3 H, *p*-C*H₃*), 4.27 (t, J=7,0 Hz, 2 H, NC*H₂*), 7.16 (s, 2 H, arom. *C*H), 7.95 (s, 1 H, NC*H*CHN), 8.10 (s, 1 H, NCHCHN), 9.43 (s, 1 H, NC*H*N).
**¹³C**-**NMR (75,5 MHz, d₆-DMSO, ppm):**
δ=13.9 (CH₂*C*H₃), 16.8 (*o-C*H₃), 20.6 (*p-C*H₃), 22.1 (C*H₂*CH₃), 25.4 (Alkyl-*C*H₂), 28.2 (Alkyl-*C*H₂), 28.7 (Alkyl-*C*H₂), 28.8 (Alkyl-*C*H₂), 28.8 (Alkyl-*C*H₂), 29.0 (Alkyl-*C*H₂), 29.0 (Alkyl-*C*H₂), 31.3 (NCH₂*C*H₂), 49.3 (N*C*H₂), 123.2 (N*C*HCHN), 124.0 (NCH*C*HN), 129.2 (arom. *C*H), 131.2 (arom. *C1*), 134.3 (arom. *C*2 und *C*6), 137.2 (N*C*HN), 140.3 (arom. *C*4).
**¹⁹F-NMR (283 MHz, d₆-DMSO, ppm):**
δ=-148.3 ppm (B*F₄⁻*).
**Elementaranalyse:** C₂₆H₄₃BF₄N₂ ber: C 66,38%, H 9,21%, N 5,95%, gef.: C 66,52%, H 9,34%, N 5,99%.

### Allgemeine Synthesevorschrift für Imidazoliumsalze mit Hexafluorophosphat-Anionen

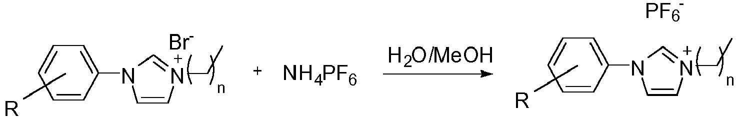

1,0 eq. des Imidazoliumbromid-Salzes wird in Wasser oder einer Wasser/Methanol Mischung vollständig gelöst. Unter ständigem Rühren werden 1,1 eq. NH₄PF₆ zugegeben. Dabei bilden sich in der Reaktionsmischung nach wenigen Minuten zwei Phasen aus. Zur Vervollständigung der Reaktion wird für weitere 15 Minuten gerührt. Anschließend werden zu der Reaktionsmischung 15 mL Dichlormethan gegeben und dann in einem Scheidetrichter die organische Phase von der wässrigen Phase abgetrennt. Die wässrige Phase wird zwei weitere Male mit je 10 mL Dichlormethan extrahiert. Die organischen Phasen werden vereinigt, über Magnesiumsulfat getrocknet und das Lösungsmittel im Vakuum entfernt.

### Ausführungsbeispiel 65

### 1-Butyl-3-mesitylimidazoliumhexafluorophosphat

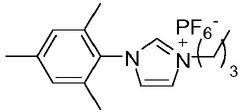

Es werden nach der allgemeinen Synthesevorschrift 1,55 mmol (0,500 g) 1-Butyl-3-mesitylimidazoliumbromid in 4 mL Wasser gelöst und 1,70 mmol (0,277 g) Ammoniumhexafluorophosphat zugegeben.
**Summenformel:** C₁₆H₂₃F₆N₂P (388,34 g/mol)
**Ausbeute:** 0,577 g (96,0%)
**Schmelzpunkt:** 127 °C
**¹H-NMR (500 MHz, d₆-DMSO, ppm):**
δ=0.94 (t, J=7,4 Hz, 3 H, CH₂C*H₃*), 1.28 (tq, J=7,4 Hz, 2 H, C*H₂*CH₃), 1.88 (tt, J=7,4 Hz, 2 H, NCH₂C*H₂*), 2.02 (s, 6 H, arom. *o-*C*H₃*), 2.34 (s, 3 H, *p*-C*H₃*), 4.28 (t, J=7,1 Hz, 2 H, NC*H₂*), 7.16 (s, 2 H, arom. C*H*), 7.95 (s, 1 H, NC*H*CHN), 8.11 (s, 1 H, NCHC*H*N), 9.43 (s, 1 H, NC*H*N).
**¹³C**-**NMR (125,8 MHz, d₆-DMSO, ppm):**
δ=13.2 (CH₂*C*H₃), 16.8 (*o-C*H₃),18.7 (*C*H₂CH₃), 20.6 (*p-C*H₃), 31.0 (NCH₂*C*H₂), 49.1 (N*C*H₂CH₃), 123.2 (N*C*HCHN), 124.0 (NCH*C*HN), 129.2 (arom. CH), 131.2 (arom. *C1*), 134.3 (arom. *C*2 und *C6*), 137.2 (N*C*HN), 140.3 (arom. *C4*).
**¹⁹F-NMR (283 MHz, d₆-DMSO, ppm):**
δ=-71.4, -68.9 (P*F₆⁻*).
**Elementaranalyse:** C₁₆H₂₃F₆N₂P ber.: C 49,49%, H 5,97%, N 7,21%, gef.: C 49, 43%, H 6,13%, N 7,17%.

### Ausführungsbeispiel 66

### 1-Mesityl-3-pentylimidazoliumhexafluorophosphat

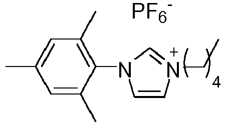

Es werden nach der allgemeinen Synthesevorschrift 1,48 mmol (0,500 g) 1-Mesityl-3-pentylimidazoliumbromid in 4 mL Wasser gelöst und 1,63 mmol (0,656 g) Ammoniumhexafluorophosphat zugegeben.
**Summenformel:** C₁₇H₂₅F₆N₂P (402,36 g/mol)
**Ausbeute:** 0,563 g (94,5%)
**Schmelzpunkt:** 90 °C
**¹H-NMR (300 MHz, d₆-DMSO, ppm):**
δ=0.89 (t, J=7,2 Hz, 3 H, CH₂C*H₃*), 1.20-1.40 (m, 4 H, C*H₂*C*H₂*), 1.90 (p, J=7,3 Hz, 2 H, NCH₂C*H*₂), 2.02 (s, 6 H, arom. *o-*C*H₃*), 2.34 (s, 3 H, *p*-C*H₃*), 4.27 (t, J=7,1 Hz, 2 H, NC*H₂*), 7.16 (s, 2 H, arom. C*H*), 7.95 (s, 1 H, NC*H*CHN), 8.11 (s, 1 H, NCHC*H*N), 9.43 (s, 1 H, NC*H*N).
**¹³C**-**NMR (75,5 MHz, d₆-DMSO, ppm):**
δ=13.8 (CH₂*C*H₃), 16.8 (*o-C*H₃), 20.6 (*p*-*C*H₃), 21.4 (Alkyl-*C*H₂), 27.6 (Alkyl-*C*H₂), 28.7 (NCH₂*C*H₂), 49.3 (N*C*H₂CH₃), 123.2 (NCH*C*HN), 124.0 (NCH*C*HN) 129.2 (arom. *C*H), 131.2 (arom. *C1*), 134.3 (arom. *C*2 und *C*6), 137.2 (N*C*HN), 140.3 (arom. *C*4).
**¹⁹F-NMR (283 MHz, d₆-DMSO, ppm):**
δ=-71.4, -68.9 (P*F₆⁻*).
**Elementaranalyse:** C₁₇H₂₅F₆N₂P ber.: C 50,75%, H 6,26%, N 6,96%, gef.: C 50,89%, H 6,36%, N 7,04%.

### Ausführungsbeispiel 67

### 1-Hexyl-3-mesitylimidazoliumhexafluorophosphat

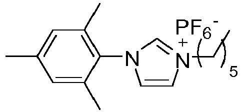

Es werden nach der allgemeinen Synthesevorschrift 1,42 mmol (0,500 g) 1-Hexyl-3-mesitylimidazoliumbromid und 1,57 mmol (0,260 g) Ammoniumhexafluorophosphat in einer Mischung aus 8 mL Wasser und 2 mL Methanol gelöst und bei Raumtemperatur gerührt.
**Summenformel:** C₁₈H₂₇F₆N₂P (416,39 g/mol)
**Ausbeute:** 0,577 g (97,5%)
**Schmelzpunkt:** -16 °C
**¹H-NMR (300 MHz, d₆-DMSO, ppm):**
δ=0.86 (t, J=6,8 Hz, 3 H, CH₂C*H₃*), 1.20-1.40 (m, 6 H, C*H₂*C*H₂*C*H₂*), 1.85-1.93 (m, 2 H, NCH₂C*H₂*), 2.02 (s, 6 H, arom. *o-*C*H₃*), 2.34 (s, 3 H, *p*-C*H₃*), 4.28 (t, J=7,1 Hz, 2 H, NC*H₂*), 7.16 (s, 2 H, arom. *C*H), 7.94 (s, 1 H, NC*H*CHN), 8.10 (s, 1 H, NCHC*H*N), 9.43 (s, 1 H, NC*H*N).
**¹³C**-**NMR (75,5 MHz, d₆-DMSO, ppm):**
δ=13.7 (CH₂*C*H₃), 16.8 (*o-C*H₃), 20.6 (*p-C*H₃), 21.9 (Alkyl-*C*H₂), 25.0 (Alkyl-*C*H₂), 28.9 (Alkyl-*C*H₂), 30.4 (Alkyl-CH₂), 30.4 (NCH₂*C*H₂), 49.3 (N*C*H₂CH₃), 123.2 (N*C*HCHN), 124.0 (NCHC*H*N), 129.2 (arom. CH), 131.2 (arom. *C1*), 134.3 (arom. *C*2 und *C*6), 137.2 (N*C*HN), 140.3 (arom. *C*4).
**¹⁹F-NMR (283 MHz, d₆-DMSO, ppm):**
δ=-71.4, -68.9 (P*F₆⁻*).
**Elementaranalyse:** C₁₈H₂₇F₆N₂P ber.: C 51,92%, H 6,54%, N 6,73%, gef.: C 51,92%, H 6,61%, N 6,65%.

### Ausführungsbeispiel 68

### 1-Mesityl-3-octyl-imidazoliumhexafluorophosphat

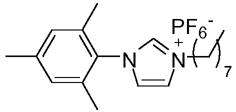

Es werden nach der allgemeinen Synthesevorschrift 0,66 mmol (0,252 g) 1-Mesityl-3-octylimidazoliumbromid in 6 mL Wasser und 3 mL Methanol gelöst und 0.73 mmol (0,120 g) Ammoniumhexafluorophosphat zugegeben.
**Summenformel:** C₂₀H₃₁F₆N₂P (444,33 g/mol)
**Ausbeute:** 0,262 g (89,1%)
**Schmelzpunkt:** -21 °C
**¹H-NMR (300 MHz, d₆-DMSO, ppm):**
δ=0.87 (t, J=6,5 Hz, 3 H, CH₂C*H₃*), 1.29 (m, 10 H, Alkyl-C*H₂*), 1.90 (m, 2 H, NCH₂C*H₂*), 2.03 (s, 6 H, *o*-C*H₃*), 2.34 (s, 3 H, *p*-C*H₃*), 4.28 (t, J=7,0 Hz, 2 H, NC*H₂*), 7.16 (s, 2 H, arom. CH), 7.95 (s, 1 H, NC*H*CHN), 8.10 (s, 1 H, NCH*C*HN), 9.44 (s, 1 H, NC*H*N).
**¹³C**-**NMR (75,5 MHz, d₆-DMSO, ppm):**
δ=13.9 (CH₂*C*H₃), 16.8 (*o*-*C*H₃), 20.6 (*p-C*H₃), 22.0 (Alkyl-*C*H₂), 25.4 (Alkyl-*C*H₂), 28.2 (Alkyl-*C*H₂), 28.5 (Alkyl-*C*H₂), 29.0 (Alkyl-C*H₂*),31.1 (NCH₂*C*H₂), 49.3 (N*C*H₂CH₃), 123.2 (N*C*HCHN), 124.0 (NCH*C*HN), 129.2 (arom. *C*H), 131.2 (arom. *C1*), 134.3 (arom. *C*2 und *C*6), 137.2 (N*C*HN), 140.3 (arom. *C*4).
**¹⁹F-NMR (283 MHz, d₆-DMSO, ppm):**
δ=-71.4, -68.9 (P*F₆⁻*).
**Elementaranalyse:** C₂₀H₃₁F₆N₂P ber.: C 54,05%, H 7,03%, N 6,30%, gef.: C54,12%, H 7,17%, N 6,20%.

### Ausführungsbeispiel 69

### 1-Mesityl-3-undecylimidazoliumhexafluorophosphat

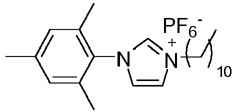

Es werden nach der allgemeinen Synthesevorschrift 1,19 mmol (0,500 g) 1-Mesityl-3-undecylimidazoliumbromid in 4 mL Methanol gelöst und 1,3 mmol (0,213 g) Ammoniumhexafluorophosphat zugegeben.
**Summenformel:** C₂₃H₃₇F₆N₂P (486,52 g/mol)
**Ausbeute:** 0,53 g (91,4%)
**Schmelzpunkt:** 0 °C
**¹H-NMR (300 MHz, d₆-DMSO, ppm):**
δ=0.86 (t, J=6,9 Hz, 3 H, CH₂C*H₃*), 1.25 (bs, 16 H, Alkyl-C*H₂*),1.88 (m, 2 H, NCH₂C*H₂*), 2.03 (s, 6 H, arom. *o-*C*H₃*), 2.32 (s, 3 H, *p-*C*H₃*), 4,27 (t, J=7,0 Hz, 2 H, NC*H₂*), 7.12 (s, 2 H, arom. *C*H), 7.89 (s, 1 H, NC*H*CHN), 8.09 (s, 1 H, NCHCHN), 9.38 (s, 1 H, NC*H*N).
**¹³C**-**NMR (75,5 MHz, d₆-DMSO, ppm):**
δ=13.9 (CH₂*C*H₃), 16.8 (*o*-*C*H₃), 20.6 (*p*-*C*H₃), 22.1 (C*H*₂CH₃), 25.4 (Alkyl-*C*H₂), 28.2 (Alkyl-*C*H₂), 28.7 (Alkyl-*C*H₂), 28.8 (Alkyl-*C*H₂), 28.8 (Alkyl-*C*H₂), 28.9 (Alkyl-*C*H₂), 29.0 (Alkyl-*C*H₂), 31.3 (NCH₂*C*H₂), 49.3 (N*C*H₂), 123.2 (N*C*HCHN), 124.0 (NCH*C*HN) 129.2 (arom. *C*H), 131.1 (arom. *C1*), 134.3 (arom. *C*2 und *C*6), 137.2 (N*C*HN), 140.3 (arom. *C*4).
**¹⁹F-NMR (283 MHz, d₆-DMSO, ppm):**
δ=-71.4, -68.9 (P*F₆⁻*).

### Ausführungsbeispiel 70

### 1-Mesityl-3-tetradecylimidazoliumtetrafluoroborat

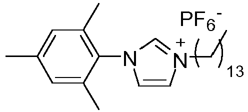

Es werden nach der allgemeinen Synthesevorschrift 1,08 mmol (0,500 g) 1-Mesityl-3-tetradecylimidazoliumbromid in 10 mL Wasser und 5 mL Methanol gelöst und 1,19 mmol (0,193 g) Ammoniumhexafluorophosphat zugegeben.
**Summenformel:** C₂₆H₄₃F₆N₂P (528,60 g/mol)
**Ausbeute:** 0,530 g (93,0%)
**Schmelzpunkt:** 57 °C
**¹H-NMR (300 MHz, d₆-DMSO, ppm):**
δ=0.86 (t, J=7,1 Hz, 3 H, CH₂C*H₃*), 1.25 (bs, 22 H, Alkyl-C*H₂*),1.87 (m, 2 H, NCH₂C*H₂*), 2.02 (s, 6 H, arom. *o-*C*H₃*), 2.34 (s, 3 H, *p*-C*H₃*), 4.27 (t, J=7,0 Hz, 2 H, NC*H₂*), 7.16 (s, 2 H, arom. C*H*), 7.95 (s, 1 H, NC*H*CHN), 8.10 (s, 1 H, NCHC*H*N), 9.43 (s, 1 H, NC*H*N).
**¹³C**-**NMR (75,5 MHz, d₆-DMSO, ppm):**
δ=14.0 (CH₂*C*H₃), 16.9 (*o*-*C*H₃), 20.6 (*p*-*C*H₃), 22.1 (C*H₂*CH₃), 25.4, 28.3, 28.8, 28.9, 28.9, 29.1, 29.1 (Alkyl-*C*H₂), 31.3 (NCH₂*C*H₂), 49.3 (N*C*H₂), 123.2 (N*C*HCHN), 124.0 (NCH*C*HN), 129.3 (arom. *C*H), 131.2 (arom. *C1*), 134.3 (arom. *C*2 und *C*6), 137.3 (NCHN), 140.3 (arom. *C*4).
**¹⁹F-NMR (283 MHz, d₆-DMSO, ppm):**
δ=-71.4, -68.9 (P*F₆⁻*).
**Elementaranalyse:** C₂₆H₄₃F₄N₂P ber: C 59,08%, H 8,20%, N 5,30%, gef.: C 59,09%, H 8,27%, N 5,31%.

### Synthesevorschrift für 1-Phenyltriazol

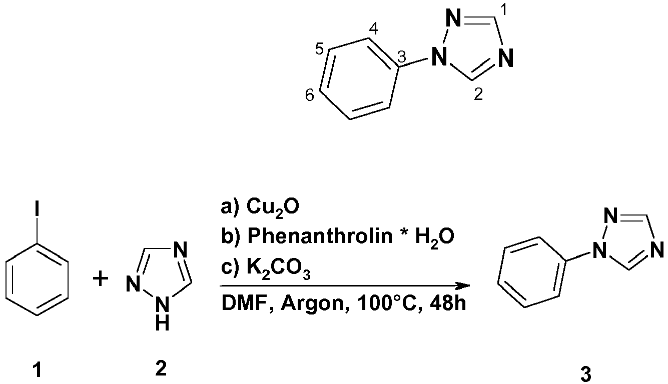

1 g (0,0145 mol) Triazol 2, 0,21 g (0,00145 mol) Kupfer(I)oxid, 0,29 g (0,00145 mol) Phenanthrolin Monohydrat und 6,01 g (0,044 mol) Kaliumcarbonat werden in ein Schlenkgefäß eingewogen. Nach mehrmaligem Evakuieren und Spülen mit Argon werden 10 ml trockenes DMF zugesetzt. Es wird mehrmals evakuiert und mit Argon gespült. Anschließend werden 2,42 ml (4,43 g, 0,022 mol) Iodbenzol zugegeben. Es wird 48 h bei 100 °C unter Argon gerührt. Nach Abkühlung werden 20 ml DCM zugegeben und abfiltriert. Das Lösungsmittel wird im Vakuum entfernt und das Produkt nach Säulenchromatographischer Reinigung (KG 60, Gradient Petrolether/ EtOAc 8:2 zu EtOAc) als gelblich-weißer Feststoff gewonnen.

Mm 145,17 C₈H₇N₃

**Ausbeute:** 1,362 g (65 %)
**¹H-NMR DM-94 (300MHz/DMSO) :**
δ (ppm)=7,41 (t, 1H, 6-H); 7,58 (t, 2H, 5/5'-H); 7,87 (d, 2H, 4-H); 8,25 (s, 1H, 1-H); 9,31 (s, 1H, 2-H)
**¹³C-NMR DM-94 (75,475MHz/DMSO) :**
δ (ppm)=119,37 (5/5'-C); 127,78 (6-C); 129,77 (4/4'-C); 136,74 (3-C); 142,27 (2-C); 152,39 (1-C)

### Ausführungsbeispiel 71

### 1-Phenyl-4-(prop-1-yl)triazoliumbromid

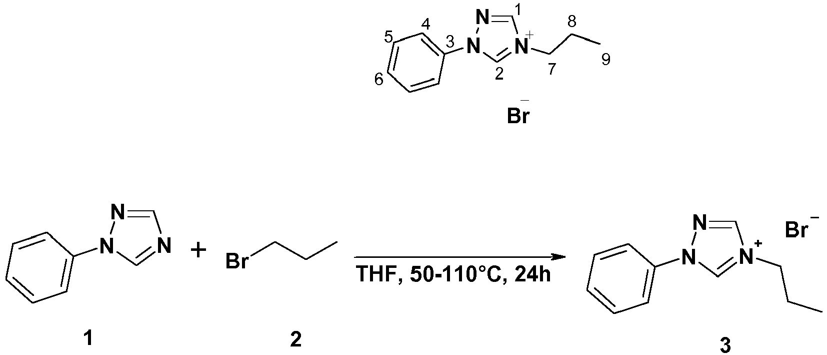

| Substanz | **1** | **2** |
|---|---|---|
| m/V [g/ml] | 0,3 g | 0,762/0,564 |
| M [g*mol⁻¹] | 145,17 | 122,99 |
| v[eq] | 1 | 3 |
| n [mol] | 0,002 | 0,006 |

0,300 g (0,002 mol) 1-Phenyltriazol **1** und 0,188 ml (0,254 g, 0,002 mol) 1-Brompropan **2** wurden in einem Druckrohr in 5 ml THF gelöst. Es wurde 1h bei 50°C gerührt, da sich aber kein Niederschlag bildete wurde die Temperatur zunächst auf 80 (2 h) und dann auf 110°C erhöht. Nach 21 h bei 110°C wurde das Reaktionsgemisch abgekühlt. Eine DC-Kontrolle zeigte noch erhebliche Mengen des Edukts **1** an, sodass weitere 0,376 ml (0,508 g, 0,004 mol) des Edukts **2** zugesetzt wurden und erneut 24h bei 110°C gerührt wurde. Anschließend wurde auf Raumtemperatur abgekühlt und das gleiche Volumen Petrolether zugesetzt. Der ausgefallene Feststoff wird abfiltriert, mit Petrolether gewaschen und im HV getrocknet.

Mm 268,16 C₁₁H₁₄N₃Br

**Ausbeute:** 0,0374 g (7%)
**¹H**-**NMR DM-102.w (300MHz**/**DMSO)** :
δ (ppm)=0,98 (t, 3H, 9-H); 3,95 (q, 2H, 8-H); 4,31 (t, 2H, 7-H); 7,70 (m, 3H, 5/5'/6-H); 7,96 (d, 2H, 4/4'-H); 9,51 (s, 1H, 1-H); 11,00 (s, 1H, 2-H)
**¹³C-NMR DM-102 (74,475MHz/DMSO) :**
δ (ppm)=10,51 (9-C); 22,18 (8-C); 49,45 (7-C); 120,64 (Phenyl-C); 130,17 (Phenyl-C); 130,47 (Phenyl-C); 135,04 (3-Cᵢₚₛₒ); 141,54 (Triazol-C); 145,05 (Triazol-C)
**Schmelzpunkt**
189°C
**Elementaranalyse**
ber.: C 49,27%; H 5,26%; N 15,67%
gef.: C 49,00%; H 5,44%; N 15,50%

### Ausführungsbeispiel 72

### 1-Phenyl-4-(hex-1-yl)triazoliumbromid

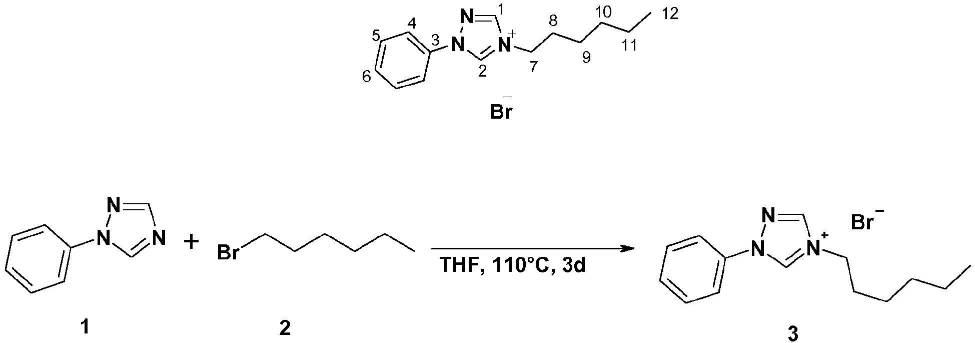

| Substanz | **1** | **2** |
|---|---|---|
| m/V [g/ml] | 0,3 g | 0,990/ 0,842 |
| M [g*mol⁻¹] | 145,17 | 165,07 |
| v[eq] | 1 | 3 |
| n [mol] | 0,002 | 0,006 |

0,300 g (0,002 mol) 1-Phenyltriazol **1** und 0,842 ml (0,990 g, 0,006 mol) 1-Bromhexan **2** wurden in einem Druckrohr in 5 ml THF gelöst. Es wurde 3 d bei 110°C gerührt. Aufgrund einer kaputten Dichtung war nach der Zeit das Lösungsmittel vedampft. Es wurde erneut 1-Bromhexan **2** und THF zugesetzt und weiter gerührt. Das Reaktionsgemisch abgekühlt und das gleiche Volumen Petrolether zugesetzt. Der ausgefallene Feststoff wird abfiltriert, mit Petrolether gewaschen und im HV getrocknet.

Während das Lösungsmittel bereits verdampft war schien die Reaktion besser zu laufen, da bei diesem Versuch erheblich höhere Ausbeuten erzielt wurden als bei allen THF-Versuchen.

Mm 310,24 C₁₄H₂₀N₃Br

**Ausbeute:** 0,4601 g (74 %)
**¹H-NMR DM-107 (300MHz/DMSO) :**
δ (ppm)=0,88 (t, 3H, 12-H); 1,33 (m, 6H, 9/10/11-H); 1,94 (q, 2H, 8-H); 4,31 (t, 2H, 7-H); 7,70 (m, 3H, 5/5'/6-H); 7,96 (d, 2H, 4/4'-H); 9,51 (s, 1H, 1-H); 11,00 (s, 1H, 2-H)
**¹³C-NMR DM-107.w (300MHz/DMSO) :**
δ (ppm)=13,84 (12-C); 21,82 (11-C); 25,13 (10-C); 28,58 (9-C); 30,57 (8-C); 47,93 (7-C); 120,60 (Phenyl-C); 130,13 (Phenyl-C); 130,41 (Phenyl-C); 135,04 (3-Cᵢₚₛₒ); 141,54 (Triazol-C); 145,00 (Triazol-C)
**Schmelzpunkt**
133°C
**Elementaranalyse**
ber.: C 54,20%; H 6,50%; N 13,54%
gef.: C 53,86%; H 6,54%; N 13,60%

### Ausführungsbeispiel 73

### 1-Phenyl-4-(tetradec-1-yl)triazoliumbromid

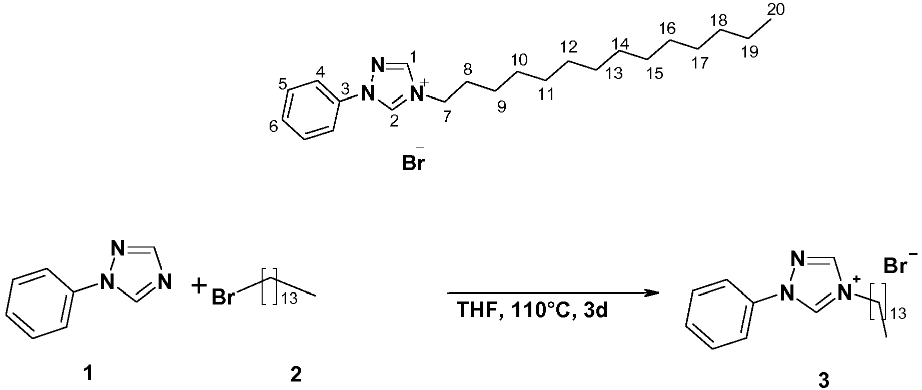

| Substanz | **1** | **2** |
|---|---|---|
| m/V [g/ml] | 0,3 g | 1,57 |
| M [g*mol⁻¹] | 145,17 | 263,23 |
| v[eq] | 1 | 3 |
| n [mol] | 0,002 | 0,006 |

0,300 g (0,002 mol) 1-Phenyltriazol **1** und 1,500 g (0,006 mol) 1-Bromtetradecan **2** wurden in einem Druckrohr in 7 ml THF gelöst. Es wurde 4 d bei 110°C gerührt. Durch die parallel erhaltenen positiven Erfahrungen der lösungsmittelfreien Synthese wurde THF entfernt und weitere 4h bei 110°C gerührt. Zu dem entstandenen Feststoff wurden 10 ml Petrolether gegeben. Das Produkt wird abfiltriert, mit Petrolether gewaschen und im Hochvakuum getrocknet.

Mm 422,53 C₂₂H₃₆N₃Br

**Ausbeute:** 0,4632 g (59 %)
**¹H-NMR DM-108 (300MHz/DMSO) :**
δ (ppm)=0,86 (t, 3H, 20-H); 1,25 (m, 24H, 9-19-H); 1,94 (q, 2H, 8-H); 4,32 (t, 2H, 7-H); 7,70 (m, 3H, 5/5'/6-H); 7,96 (d, 2H, 4/4'-H); 9,50 (s, 1H, 1-H); 10,97 (s, 1H, 2-H)
**¹³C-NMR DM-108 (75,475MHz/DMSO) :**
δ (ppm)=13,93 (20-C); 22,06 (19-C); 25,46 (18-C); 28,40 (17-C); 28,62 (16-C); 28,68 (15-C); 28,76 (13/14-C); 28,83 (12-C); 28,93 (11-C); 28,98 (9/10-C); 31,26 (8-C); 47,95 (7-C); 120,59 (5/5'-C); 130,15 (4/4'-C); 130,44 (6-C); 135,04 (3-C); 141,53 (2-C); 145,02 (1-C)
**Schmelzpunkt**
156 °C
**Elementaranalyse**
ber.: C 60,91%; H 8,59%; N 9,95%
gef.: C 62,04%; H 8,92%; N 9,00%

### Ausführungsbeispiel 74

### 1-Phenyl-4-(hept-1-yl)triazoliumbromid

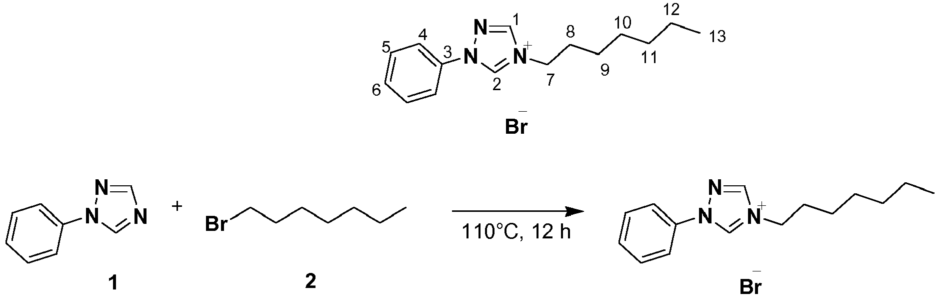

| Substanz | **1** | **2** |
|---|---|---|
| m/V [g/ml] | 0,15 g | 1,11/0,97 |
| M [g*mol⁻¹] | 145,17 | 165,07 |
| v[eq] | 1 | 6 |
| n [mol] | 0,001 | 0,006 |

0,150 g (0,002 mol) 1-Phenyltriazol **1** (eigentlich 0,3 g, es waren jedoch noch 2 eq. DMF im Edukt enthalten) und 0,97 ml (1,110 g, 0,006 mol) 1-Bromheptan **2** werden in einem Druckrohr vereinigt. Es wirde 12 h bei 110°C gerührt. Das Reaktionsgemisch wird abgekühlt und das gleiche Volumen Petrolether zugesetzt. Der ausgefallene Feststoff wird abfiltriert, mit Petrolether gewaschen und im HV getrocknet.

Mm 324,26 C₁₅H₂₂N₃Br

**Ausbeute:** 0,225 g (69 %)
**¹H-NMR DM-170 (300MHz/DMSO) :**
δ (ppm)=0,87 (t, 3H, 13-H); 1,30 (m, 8H, 9/10/11/12-H); 1,95 (qui, 2H, 8-H); 4,34 (t, 2H, 7-H); 7,68 (m, 3H, 5/5'/6-H); 7,95 (d, 2H, 4/4'-H); 9,55 (s, 1H, 1-H); 11,10 (s, 1H, 2-H)
**¹³C-NMR DM-170 (300MHz/DMSO) :**
δ (ppm)=13,90 (13-C); 21,97 (12-C); 25,43 (11-C); 28,05 (10-C); 28,61 (9-C); 30,96 (8-C); 47,93 (7-C); 120,53 (Phenyl-C); 130,11 (Phenyl-C); 130,38 (Phenyl-C); 135,04 (3-Cᵢₚₛₒ); 141,54 (Triazol-C); 144,99 (Triazol-C)
**Schmelzpunkt**
123°C

### Ausführungsbeispiel 75

### 1-Phenyl-4-(pent-1-yl)triazoliumbromid

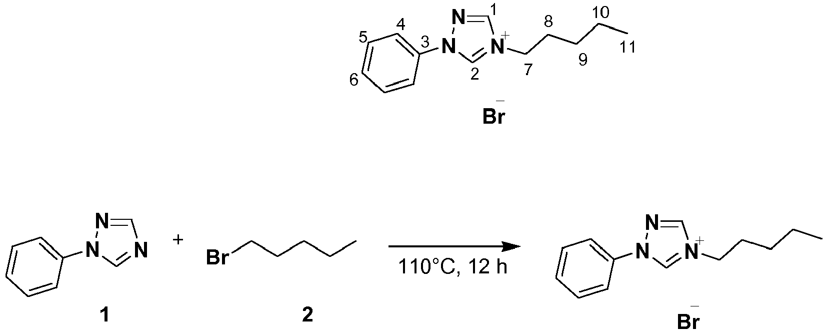

| Substanz | **1** | **2** |
|---|---|---|
| m/V [g/ml] | 0,15 g | 0,91/0,74 |
| M [g*mol⁻¹] | 145,17 | 165,07 |
| v[eq] | 1 | 6 |
| n [mol] | 0,001 | 0,006 |

0,150 g (0,001 mol) 1-Phenyltriazol **1** (eigentlich 0,3 g, es waren jedoch noch 2 eq. DMF im Edukt enthalten) und 0,74 ml (0,906 g, 0,006 mol) 1-Bromheptan **2** werden in einem Druckrohr vereinigt. Es wird 12 h bei 110°C gerührt. Das Reaktionsgemisch wird abgekühlt und das gleiche Volumen Petrolether zugesetzt. Der ausgefallene Feststoff wird abfiltriert, mit Petrolether gewaschen und im HV getrocknet.

Mm 296,21 C₁₃H₁₈N₃Br

**Ausbeute:** 0,236 g (80 %)
**¹H-NMR DM-170 (300MHz/DMSO) :**
δ (ppm)=0,90 (t, 3H, 11-H); 1,35 (m, 4H, 9/10-H); 1,95 (qui, 2H, 8-H); 4,33 (t, 2H, 7-H); 7,67 (m, 3H, 5/5'/6-H); 7,95 (d, 2H, 4/4'-H); 9,52 (m, 1H, 1-H); 11,04 (m, 1H, 2-H)
**¹³C-NMR DM-170 (300MHz/DMSO) :**
δ (ppm)=13,71(11-C); 21,53 (10-C); 27,57 (9-C); 28,31 (8-C); 47,91 (7-C); 120,60 (Phenyl-C); 130,13 (Phenyl-C); 130,41 (Phenyl-C); 135,04 (3-Cᵢₚₛₒ); 141,55 (Triazol-C); 145,01 (Triazol-C)
**Schmelzpunkt**
116°C

### Ausführungsbeispiel 76

### 1-Phenyl-4-(undec-1-yl)triazoliumbromid

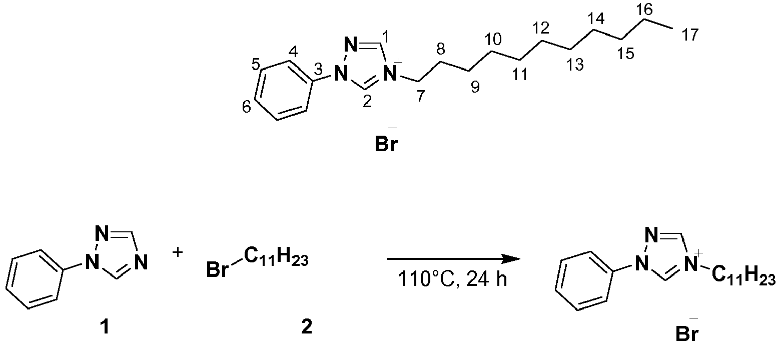

| Substanz | **1** | **2** |
|---|---|---|
| m/V [g/ml] | 0,30 g | 1,41 |
| M [g*mol⁻¹] | 145,17 | 235,20 |
| v[eq] | 1 | 3 |
| n [mol] | 0,002 | 0,006 |

0,300 g (0,002 mol) 1-Phenyltriazol **1** und 1,41 g (0,006 mol) 1-Bromundecan **2** werden in einem Druckrohr vereinigt. Es wird 24 h bei 110°C gerührt. Das Reaktionsgemisch abgekühlt und das gleiche Volumen Petrolether zugesetzt. Der ausgefallene Feststoff wird abfiltriert, mit Petrolether/THF (1:1) gewaschen und im HV getrocknet.

Mm 380,37 C₁₉H₃₀N₃Br

Ausbeute: 0,281 g (37 %)
**¹H-NMR DM-187 (300MHz/DMSO) :**
δ (ppm)=0,86 (t, 3H, 17-H); 1,26 (m, 16H, 9/10/11/12/13/14/15/16-H); 1,96 (qui, 2H, 8-H); 4,34 (t, 2H, 7-H); 7,67 (m, 3H, 5/5'/6-H); 7,95 (d, 2H, 4/4'-H); 9,55 (m, 1H, 1-H); 11,08 (m, 1H, 2-H)
**¹³C-NMR DM-187 (300MHz/DMSO) :**
δ (ppm)=13,91 (17-C); 22,05 (16-C); 25,46 (15-C); 28,40 (14-C); 28,61 (13-C); 28,67 (12-C); 28,76 (11-C); 28,93 (10-C); 28,95 (9-C); 31,26 (8-C); 47,93 (7-C); 120,57 (5/5'-C); 130,11 (4/4'-C); 130,39 (6-C); 135,03 (3-C); 141,53 (2-C); 145,00 (1-C)
**Schmelzpunkt**
160,1 °C
**Elementaranalyse**
ber.: C 60,00%; H 7,95%; N 11,05%
gef.: C 59,86%; H 7,79%; N 11,04%

### Ausführungsbeispiel 77

### 1-Phenyl-4-(hex-1-yl)triazoliumbis(trifluormethylsulfon)amid

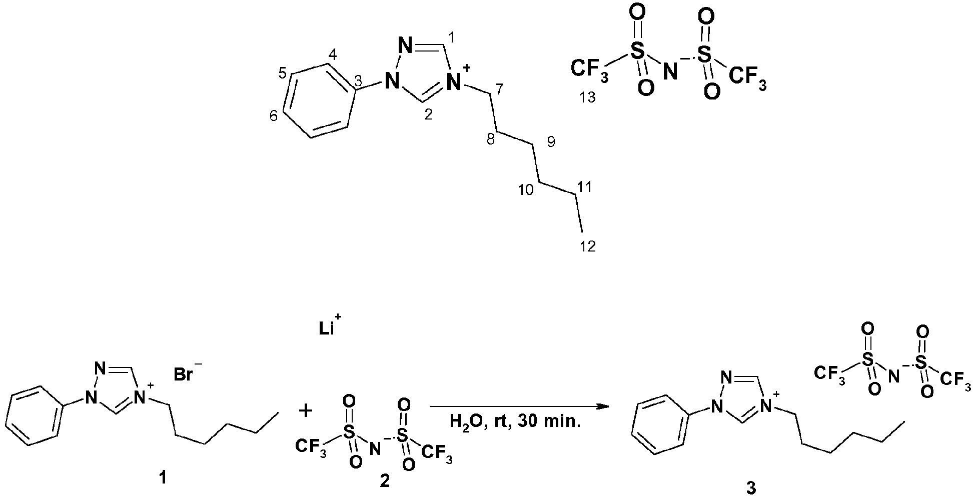

| Substanz | **1** | **2** |
|---|---|---|
| m/V [g/ml] | 0,15 g | 0,158 |
| M [g*mol⁻¹] | 310,24 | 287,08 |
| v[eq] | 1 | 1,1 |
| n [mol] | 0,0005 | 0,00055 |

0,150 g (0,0005 mol) 1-Phenyl-4-(hex-1-yl)triazoliumbromid **1** werden in wenig Wasser gelöst. Anschließend werden 0,158 g (0,00055 mol) Lithiumbis(trifluormethylsulfon)amid zugegeben und es wird 30 min. bei Raumtemperatur gerührt. Die sich bildende zweite Phase wird durch Zugabe von Dichlormethan vergrößert. Die Phasen werden separiert und die wässrige zweimal mit Dichlormethan gewaschen. Die vereinigten organischen Phasen werden über Natriumsulfat getrocknet. Das Lösungsmittel wird im Vakuum entfernt und das Produkt als bräunliches Öl gewonnen.

Mm 510,48 C₁₆H₂₀F₆N₄O₄S₂

**¹H-NMR DM-135 (300MHz/DMSO) :**
δ (ppm)=0,89 (t, 3H, 12-H); 1,33 (m, 6H, 9/10/11-H); 1,93 (q, 2H, 8-H); 4,30 (t, 2H, 7-H); 7,69 (m, 3H, 5/5'/6-H); 7,92 (d, 2H, 4/4'-H); 9,46 (s, 1H, 1-H); 10,88 (s, 1H, 2-H)
**¹⁹F-NMR DM-135 (282,4MHz/DMSO) :**
δ (ppm)=-78,71(s, 13-F);

### Ausführungsbeispiel 78

### 1-Phenyl-4-(prop-1-yl)triazoliumbis(trifluormethylsulfon)amid

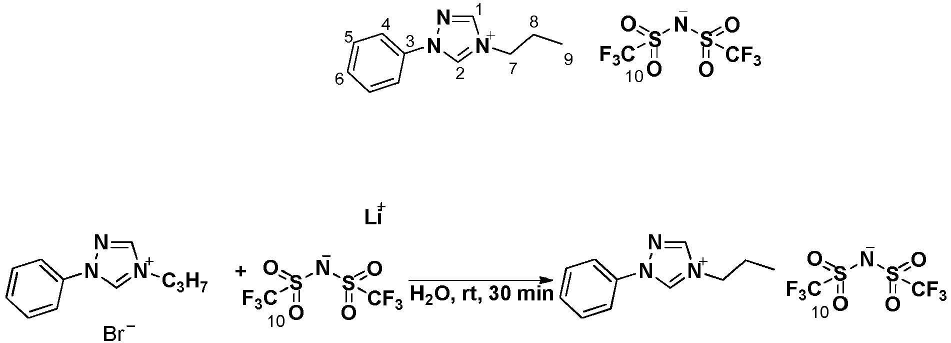

| Substanz | **1** | **2** |
|---|---|---|
| m/V [g/ml] | 0,020 g | 0,030 |
| M [g*mol⁻¹] | 268,16 | 287,08 |
| v[eq] | 1 | 1,4 |
| n [mol] | 0,00007 | 0,0001 |

0,020 g (0,00007 mol) 1-Phenyl-4-(prop-1-yl)triazoliumbromid **1** werden in wenig Wasser gelöst. Anschließend werden 0,030 g (0,00010 mol) Lithiumbis(trifluormethylsulfon)amid zugegeben und es wird 30 min. bei Raumtemperatur gerührt. Die sich bildende zweite Phase wird durch Zugabe von Dichlormethan vergrößert. Die Phasen werden separiert und die wässrige zweimal mit Dichlormethan gewaschen. Die vereinigten organischen Phasen werden über Natriumsulfat getrocknet. Das Lösungsmittel wird im Vakuum entfernt und das Produkt als bräunlich, weißer Feststoff gewonnen.

Mm 468,4 C₁₃H₁₄ F₆N₄O₄S₂

**Ausbeute:** 0,029 g (88%)
**¹H-NMR DM-215 (300MHz/DMSO) :**
δ (ppm)=0,98 (t, 3H, 9-H); 1,95 (dt, 2H, 8-H); 4,28 (t, 2H, 7-H); 7,71 (m, 3H, 5/5'/6-H); 7,92 (d, 2H, 4/4'-H); 9,46 (s, 1H, 1-H); 10,88 (s, 1H, 2-H)
**¹³C-NMR DM-215 (75,453MHz/DMSO) :**
δ (ppm)=10,49 (9-C); 22,18 (8-C); 49,46 (7-C); 117,33 (10/10'-C); 120,64 (4/4'-C); 130,16 (5/5'-C); 130,47 (6-C); 135,04 (3-C); 141,53 (2-C); 145,05 (1-C)
**¹⁹F-NMR DM-215 (282,4MHz/DMSO) :**
δ (ppm)=-78,72 (s, 10-F);
**Schmelzpunkt:**
99°C
**Elementaranalyse**
ber.: C 33,33%; H 3,01%; N 11,96% S 13,69%
gef.: C 33,71%; H 2,37%; N 11,94% S 13,76%

### Ausführungsbeispiel 79

### 1-Phenyl-4-(pent-1-yl)triazoliumbis(trifluormethyl)sulfonamid

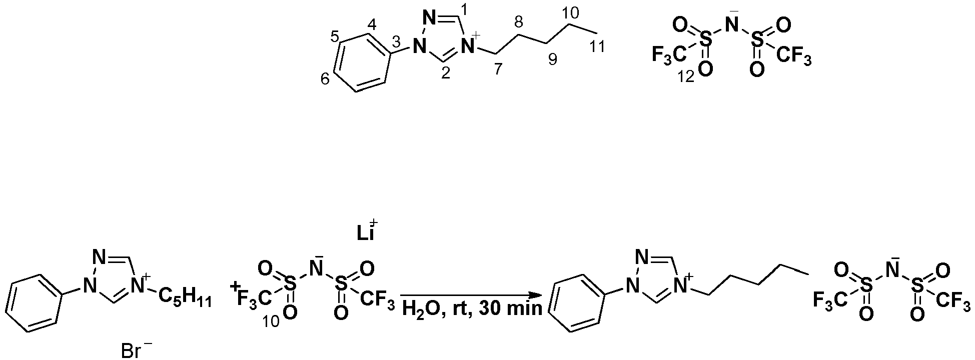

| Substanz | **1** | **2** |
|---|---|---|
| m/V [g/ml] | 0,116 g | 0,124 |
| M [g*mol⁻¹] | 296,26 | 287,08 |
| v[eq] | 1 | 1,1 |
| n [mol] | 0,00039 | 0,00043 |

0,116 g (0,00039 mol) 1-Phenyl-4-(pent-1-yl)triazoliumbromid **1** werden in wenig Wasser gelöst. Anschließend werden 0,124 g (0,00043 mol) Lithiumbis(trifluormethylsulfon)amid zugegeben und es wird 30 min. bei Raumtemperatur gerührt. Die sich bildende zweite Phase wird durch Zugabe von Dichlormethan vergrößert. Die Phasen werden separiert und die wässrige zweimal mit Dichlormethan gewaschen. Die vereinigten organischen Phasen werden über Natriumsulfat getrocknet. Das Lösungsmittel wird im Vakuum entfernt und das Produkt als bräunliches Öl gewonnen.

Mm 496,45 C₁₅H₁₅F₆N₄O₄S₂

**Ausbeute:** 0,174 g (90%)
**¹H-NMR DM-216 (300MHz/DMSO) :**
δ (ppm)=0,92 (t, 3H, 11-H); 1,36 (m, 4H, 9/10-H); 1,95 (q, 2H, 8-H); 4,31 (t, 2H, 7-H); 7,71 (m, 3H, 5/5'/6-H); 7,93 (d, 2H, 4/4'-H); 9,47 (s, 1H, 1-H); 10,89 (s, 1H, 2-H)
**¹³C-NMR DM-216 (75,453MHz/DMSO) :**
δ (ppm)=13,69 (11-C); 21,54 (10-C); 27,58 (9-C); 28,32 (8-C); 47,59 (7-C); 117,33 (12/12'-C); 120,63 (4/4'-C); 130,16 (5/5'-C); 130,47 (6-C); 135,04 (3-C); 141,52 (2-C); 145,03 (1-C)
**¹⁹F-NMR DM-216 (282,4MHz/DMSO) :**
δ (ppm)=-78,71 (s, 12-F);
**Schmelzpunkt**
flüssig

### Ausführungsbeispiel 80

### 1-Phenyl-4-(hept-1-yl)triazoliumbis(trifluormethyl)sulfonamid

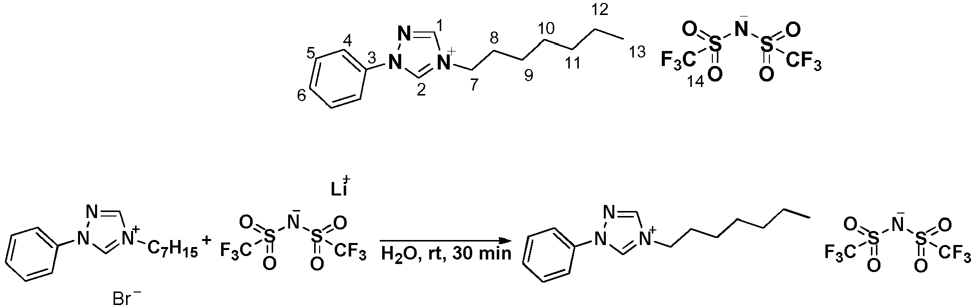

| Substanz | **1** | **2** |
|---|---|---|
| m/V [g/ml] | 0,127 g | 0,124 |
| M [g*mol⁻¹] | 324,26 | 287,08 |
| v[eq] | 1 | 1,1 |
| n [mol] | 0,00039 | 0,00043 |

0,127 g (0,00039 mol) 1-Phenyl-4-(hept-1-yl)triazoliumbromid **1** werden in wenig Wasser gelöst. Anschließend werden 0,124 g (0,00043 mol) Lithiumbis(trifluormethylsulfon)amid zugegeben und es wird 30 min. bei Raumtemperatur gerührt. Die sich bildende zweite Phase wird durch Zugabe von Dichlormethan vergrößert. Die Phasen werden separiert und die wässrige zweimal mit Dichlormethan gewaschen. Die vereinigten organischen Phasen werden über Natriumsulfat getrocknet. Das Lösungsmittel wird im Vakuum entfernt und das Produkt als bräunliches Öl gewonnen.

Mm 524,5 C₁₇H₂₂F₆N₄O₄S₂

**Ausbeute:** 0,186 g (91%)
**¹H-NMR DM-217 (300MHz/DMSO) :**
δ (ppm)=0,88 (t, 3H, 13-H); 1,33 (m, 8H, 9/10/11/12-H); 1,94 (q, 2H, 8-H); 4,31 (t, 2H, 7-H); 7,71 (m, 3H, 5/5'/6-H); 7,92 (d, 2H, 4/4'-H); 9,46 (s, 1H, 1-H); 10,88 (s, 1H, 2-H)
**¹³C-NMR DM-217 (75,453MHz/DMSO) :**
δ (ppm)=13,91 (13-C); 21,99 (12-C); 25,43 (11-C); 28,07 (10-C); 28,64 (9-C); 30,98 (8-C); 47,98 (7-C); 117,33 (14/14'-C); 120,63 (4/4'-C); 130,17 (5/5'-C); 130,47 (6-C); 135,05 (3-C); 141,51 (2-C); 145,03 (1-C)
**¹⁹F-NMR DM-217 (282,4MHz/DMSO) :**
δ (ppm)=-78,71 (s, 14-F);
**Schmelzpunkt** flüssig

### Ausführungsbeispiel 81

### 1-Phenyl-4-(undec-1-yl)triazoliumbis(trifluormethylsulfon)amid

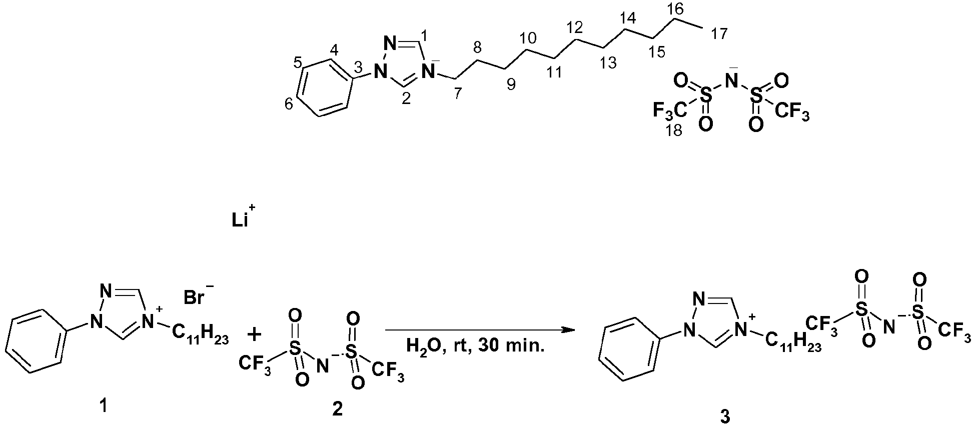

| Substanz | **1** | **2** |
|---|---|---|
| m/V [g/ml] | 0,15 g | 0,125 |
| M [g*mol⁻¹] | 380,37 | 287,08 |
| v[eq] | 1 | 1,1 |
| n [mol] | 0,0004 | 0,0004 |

0,150 g (0,0004 mol) 1-Phenyl-4-(undec-1-yl)triazoliumbromid 1 werden in wenig Wasser und Methanol gelöst. Anschließend werden 0,125 g (0,0004 mol) Lithiumbis-(trifluormethylsulfon)amid zugegeben und es wird 30 min. bei Raumtemperatur gerührt. Die sich bildende zweite Phase wird durch Zugabe von Dichlormethan vergrößert. Die Phasen werden separiert und die wässrige zweimal mit Dichlormethan gewaschen. Die vereinigten organischen Phasen werden über Natriumsulfat getrocknet. Das Lösungsmittel wird im Vakuum entfernt und das Produkt als farbloser Feststoff gewonnen.

Mm 580,61 C₂₁H₃₀F₆N₄O₄S₂

**Ausbeute:** 0,194 g (84%)
**¹H-NMR DM-218 (300MHz/DMSO) :**
δ (ppm)=0,87 (t, 3H, 17-H); 1,26 (m, 16H, 9/10/11/12/13/14/15/16-H); 1,94 (q, 2H, 8-H); 4,31 (t, 2H, 7-H); 7,72 (m, 3H, 5/5'/6-H); 7,94 (d, 2H, 4/4'-H); 9,47 (s, 1H, 1-H); 10,89 (s, 1H, 2-H)
**¹³C-NMR DM-218 (75,475MHz/DMSO) :**
δ (ppm)=13,95 (17-C); 22,10 (16-C); 25,48 (15-C); 28,43 (14-C); 28,65 (13-C); 28,71 (12-C); 28,78 (11-C); 28,97 (10-C); 28,99 (9-C); 31,30 (8-C); 47,98 (7-C); 118,19 (18/18'-C); 120,62 (4/4'-C); 130,18 (5/5'-C); 130,47 (6-C); 135,06 (3-C); 141,55 (2-C); 145,05 (1-C)
**¹⁹F-NMR DM-218 (282,4MHz/DMSO) :** δ (ppm)=-78,72(s, 18-F);
**Schmelzpunkt:** 40°C
**Elementaranalyse**
ber.: C 43,44%; H 5,21%; N 9,65% S 11,05%
gef.: C 43,31%; H 3,60%; N 9,26% S 11,62%

### Ausführungsbeispiel 82

### 1-Phenyl-4-(tetradec-1-yl)triazoliumbis(trifluormethylsulfon)amid

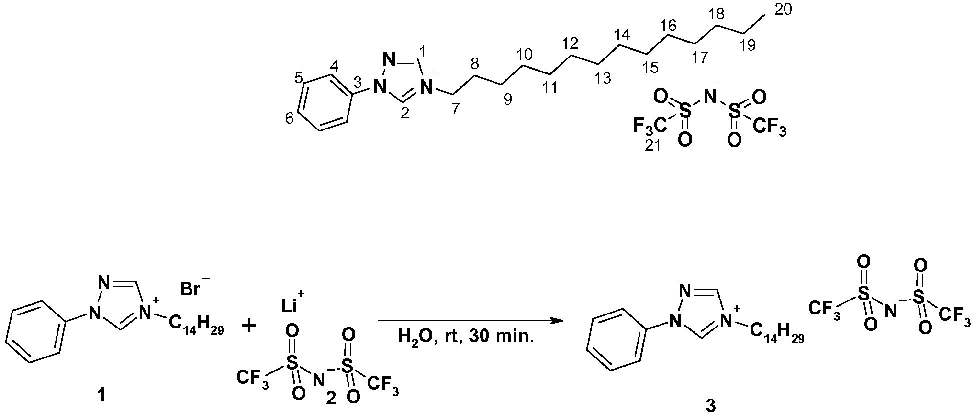

| Substanz | **1** | **2** |
|---|---|---|
| m/V [g/ml] | 0,165 g | 0,118 |
| M [g*mol⁻¹] | 442,45 | 287,08 |
| v[eq] | 1 | 1,1 |
| n [mol] | 0,0004 | 0,0004 |

0,165 g (0,0004 mol) 1-Phenyl-4-(tetradec-1-yl)triazoliumbromid 1 werden in wenig Wasser und Methanol gelöst. Anschließend werden 0,118 g (0,0004 mol) Lithiumbis(trifluormethylsulfon)amid zugegeben und es wird 30 min. bei Raumtemperatur gerührt. Die sich bildende zweite Phase wird durch Zugabe von Dichlormethan vergrößert. Die Phasen werden separiert und die wässrige zweimal mit Dichlormethan gewaschen. Die vereinigten organischen Phasen werden über Natriumsulfat getrocknet. Das Lösungsmittel wird im Vakuum entfernt und das Produkt als farbloser Feststoff gewonnen.

Mm 622,69 C₂₄H₃₆F₆N₄O₄S₂

**Ausbeute:** 0,216 g (87%)
**¹H-NMR DM-219 (300MHz/DMSO) :**
δ (ppm)=0,86 (t, 3H, 20-H); 1,25 (m, 22H, 9/10/11/12/13/14/15/16/17/18/19-H); 1,94 (q, 2H, 8-H); 4,31 (t, 2H, 7-H); 7,72 (m, 3H, 5/5'/6-H); 7,91 (d, 2H, 4/4'-H); 9,47 (s, 1H, 1-H); 10,89 (s, 1H, 2-H)
**¹³C-NMR DM-219 (75,475MHz/DMSO) :**
δ (ppm)=13,95 (21-C); 22,11 (20-C); 25,49 (19-C); 28,44 (18-C); 28,66 (17-C); 28,73 (16-C); 28,80 (15-C); 28,94 (14-C); 28,97 (12/13-C); 29,03 (10/11-C); 29,05 (9-C); 31,31 (8-C); 47,99 (7-C); 118,21 (18/18'-C); 120,62 (4/4'-C); 130,18 (5/5'-C); 130,48 (6-C); 135,07 (3-C); 141,56 (2-C); 145,06 (1-C)
**¹⁹F-NMR DM-219 (282,4MHz/DMSO) :** δ (ppm)=-78,72(s, 21-F);
**Schmelzpunkt** 38°C
**Elementaranalyse**
ber.: C 47,03%; H 5,83%; N 10,28% S 10,30%
gef.: C 47,07%; H 5,53%; N 8,76% S 9,09%

### Synthesevorschrift für 4-Phenyltriazol

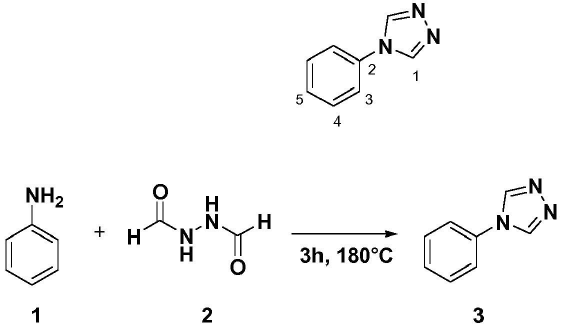

| Substanz | **1** | **2** |
|---|---|---|
| m/V [g/ml] | 2,64/ 2,6 | 2,5 |
| M [g*mol⁻¹] | 93,1 | 88,07 |
| v[eq] | 1 | 1 |
| n [mol] | 0,028 | 0,028 |

2,6 ml (2,64 g, 0,028 mol) Anilin 1 und 2,5 g (0,028 mol) Diformylhydrazin in einem Schlenkkolben vereinigt. Der Kolben wird mit einem Plastikdeckel locker abgedeckt und es wird 3 h bei 180°C gerührt. Gegen Ende der Reaktionszeit wird der Deckel entfernt, um entstandenes Reaktionswasser zu verdampfen. Nach Abkühlen werden 20 ml Chloroform zugegeben. Das übrige Diformylhydrazin wird abfiltriert und mit Chloroform gewaschen. Die vereinigten Chloroformphasen werden über Natriumsulfat getrocknet. Es werden 100 ml Diethylether zugegeben und 3 Tage im Kühlschrank zum Kristallisieren stehen gelassen. Der ausgefallene Niederschlag wird abfiltriert, mit Diehtylether gewaschen und im Vakuum getrocknet.

Mm 217,25 C₁₂H₁₃N₂O₂

**Ausbeute:** 1,0054 g (25 %)
**¹H-NMR DM-209 (300MHz/CDCl₃):**
δ (ppm)=7,40 (m, 5H, 3,3',4,4',5-H); 8,5 (s, 2H, 1,1'-H)
**¹³C-NMR DM-209 (75,475MHz/CDCl₃):**
δ (ppm)=122,13 (4,4'-C); 129,00 (5-C); 130,22 (3,3'-C); 141,35 (1,1'-C), Ipso-C?

### Ausführungsbeispiel 83

### 4-Phenyl-1-(prop-1-yl)triazoliumbromid

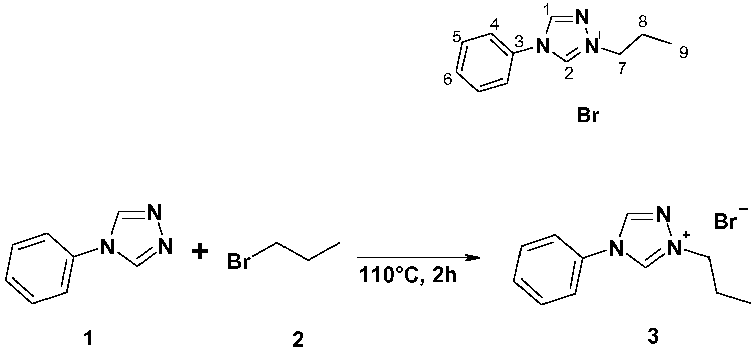

| Substanz | **1** | **2** |
|---|---|---|
| m/V [g/ml] | 0,15 g | 0,369/0,273 |
| M [g*mol⁻¹] | 145,17 | 122,99 |
| v[eq] | 1 | 3 |
| n [mol] | 0,001 | 0,003 |

0,15 g (0,001 mol) 4-Phenyltriazol **1** und 0,27 ml (0,37 g, 0,003 mol) 1-Brompropan 2 werden in einem Druckrohr vereinigt. Es wird 12h bei 110°C gerührt. Anschließend wird auf Raumtemperatur abgekühlt. Der ausgefallene Feststoff wird in einer 1:1-Mischung aus THF und Petrolether aufgenommen, abfiltriert und mit Diethylether gewaschen.

Mm 268,15 C₁₁H₁₄N₃Br

**Ausbeute:** 0,259 (97 %)
**¹H-NMR DM-228 (300MHz/DMSO) :**
δ (ppm)=0,99 (t, 3H, 9-H); 1,96 (q, 2H, 8-H); 4,43 (t, 2H, 7-H); 7,71 (m, 3H, 5/5'/6-H); 7,88 (d, 2H, 4/4'-H); 9,84 (d, 1H, 2-H); 10,94 (d, 1H, 1-H)
**¹³C-NMR DM-228 (75,475MHz/DMSO) :**
δ (ppm)=10,54 (9-C); 21,49 (8-C); 53,43 (7-C); 122,43 (5/5'-C); 130,14 (4/4'-C); 130,39 (6-C); 132,16 (3-C); 141,49 (2-C); 142,82 (1-C)
**Schmelzpunkt**
137,3°C

### Ausführungsbeispiel 84

### 4-Phenyl-1-(pent-1-yl)triazoliumbromid

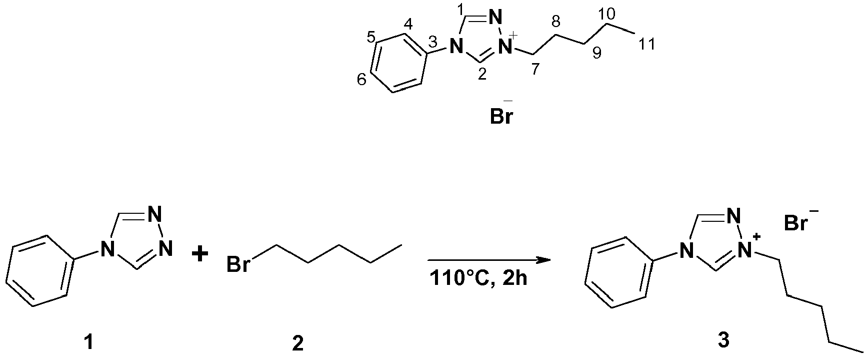

| Substanz | **1** | **2** |
|---|---|---|
| m/V [g/ml] | 0,15 g | 0,453/0,372 |
| M [g*mol⁻¹] | 145,17 | 151,04 |
| v[eq] | 1 | 3 |
| n [mol] | 0,001 | 0,003 |

0,15 g (0,001 mol) 4-Phenyltriazol **1** und 0,37 ml (0,45 g, 0,003 mol) 1-Brompentan **2** werden in einem Druckrohr vereinigt. Es wird 12h bei 110°C gerührt. Anschließend wird auf Raumtemperatur abgekühlt. Der ausgefallene Feststoff wird in einer 1:1-Mischung aus THF und Petrolether aufgenommen, abfiltriert und mit Diethylether gewaschen.

Mm 296,21 C₁₃H₁₈N₃Br

**Ausbeute:** 0,231 g (78 %)
**¹H-NMR DM-229 (300MHz/DMSO) :**
δ (ppm)=0,88 (t, 3H, 11-H); 1,37 (m, 4H, 9/10-H); 1,96 (q, 2H, 8-H); 4,44 (t, 2H, 7-H); 7,69 (m, 3H, 5/5'/6-H); 7,85 (d, 2H, 4/4'-H); 9,81 (s, 1H, 2-H); 10,86 (s, 1H, 1-H)
**¹³C-NMR DM-229 (75,475MHz/DMSO) :**
δ (ppm)=13,72 (11-C); 21,53 (10-C); 27,55 (9-C); 27,63 (8-C); 51,93 (7-C); 122,45 (5/5'-C); 130,17 (4/4'-C); 130,43 (6-C); 132,18 (3-C); 141,47 (2-C); 142,84 (1-C)
**Schmelzpunkt**
118°C

### Ausführungsbeispiel 85

### 4-Phenyl-1-(hept-1-yl)triazoliumbromid

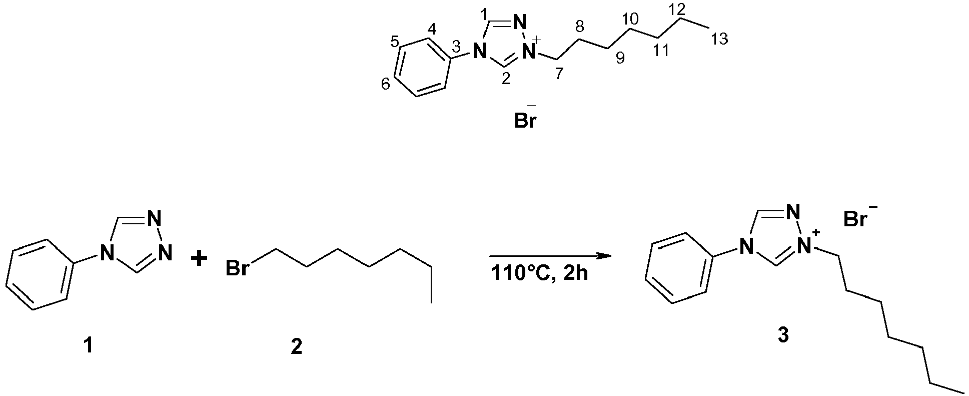

| Substanz | **1** | **2** |
|---|---|---|
| m/V [g/ml] | 0,15 g | 0,537/ 0,47 |
| M [g*mol⁻¹] | 145,17 | 179,1 |
| v[eq] | 1 | 3 |
| n [mol] | 0,001 | 0,003 |

0,15 g (0,001 mol) 4-Phenyltriazol **1** und 0,47 ml (0535 g, 0,003 mol) 1-Bromheptan **2** werden in einem Druckrohr vereinigt. Es wird 12h bei 110°C gerührt. Anschließend wird auf Raumtemperatur abgekühlt. Der ausgefallene Feststoff wird in einer 1:1-Mischung aus THF und Petrolether aufgenommen, abfiltriert und mit Diethylether gewaschen.

Mm 324,26 C₁₅H₂₂N₃Br

**Ausbeute:** 0,306 (94 %)
**¹H-NMR DM-230 (300MHz/DMSO) :**
δ (ppm)=0,88 (t, 3H, 13-H); 1,37 (m, 8H, 9/10/11/12-H); 1,96 (q, 2H, 8-H); 4,44 (t, 2H, 7-H); 7,69 (m, 3H, 5/5'/6-H); 7,85 (d, 2H, 4/4'-H); 9,81 (s, 1H, 2-H); 10,85 (s, 1H, 1-H)
**¹³C-NMR DM-230 (75,475MHz/DMSO) :**
δ(ppm)=13,92 (13-C); 21,98 (12-C); 25,39 (11-C); 27,94 (10-C); 28,06 (9-C); 31,00 (8-C); 51,96 (7-C); 122,45 (5/5'-C); 130,17 (4/4'-C); 130,43 (6-C); 132,18 (3-C); 141,47 (2-C); 142,84 (1-C)
**Schmelzpunkt**
127,2°C

### Ausführungsbeispiel 86

### 4-Phenyl-1-(hex-1-yl)triazoliumbromid

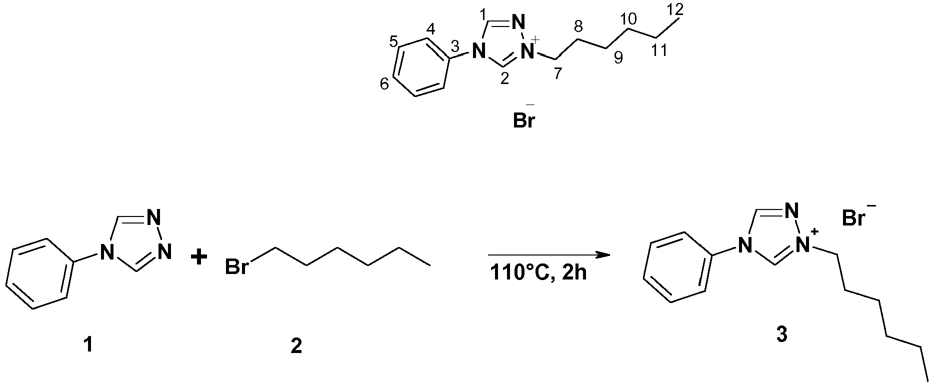

| Substanz | **1** | **2** |
|---|---|---|
| m/V [g/ml] | 0,15 g | 0,495/0,421 |
| M [g*mol⁻¹] | 145,17 | 165,1 |
| v[eq] | 1 | 3 |
| n [mol] | 0,001 | 0,003 |

0,15 g (0,001 mol) 4-Phenyltriazol **1** und 0,42 ml (0,495 g, 0,003 mol) 1-Bromhexan **2** werden in einem Druckrohr vereinigt. Es wird 12h bei 110°C gerührt. Anschließend wird auf Raumtemperatur abgekühlt. Der ausgefallene Feststoff wird in einer 1:1-Mischung aus THF und Petrolether aufgenommen, abfiltriert und mit Diethylether gewaschen.
Mm 310,23 C₁₄H₂₀N₃Br
**Ausbeute:** 0,254 g (82 %)
**¹H-NMR DM-231 (300MHz/DMSO) :**
δ (ppm)=0,89 (t, 3H, 12-H); 1,33 (m, 6H, 9/10/11-H); 1,96 (q, 2H, 8-H); 4,45 (t, 2H, 7-H); 7,69 (m, 3H, 5/5'/6-H); 7,86 (d, 2H, 4/4'-H); 9,81 (s, 1H, 2-H); 10,88 (s, 1H, 1-H)
**¹³C-NMR DM-231 (75,475MHz/DMSO) :**
δ (ppm)=13,88 (12-C); 21,88 (11-C); 25,13 (10-C); 27,91 (9-C); 30,60 (8-C); 51,97 (7-C); 122,46 (5/5'-C); 130,20 (4/4'-C); 130,45 (6-C); 132,19 (3-C); 141,47 (2-C); 142,84 (1-C)
**Schmelzpunkt**
120,9°C

### Ausführungsbeispiel 87

### 4-Phenyl-1-(undec-1-yl)triazoliumbromid

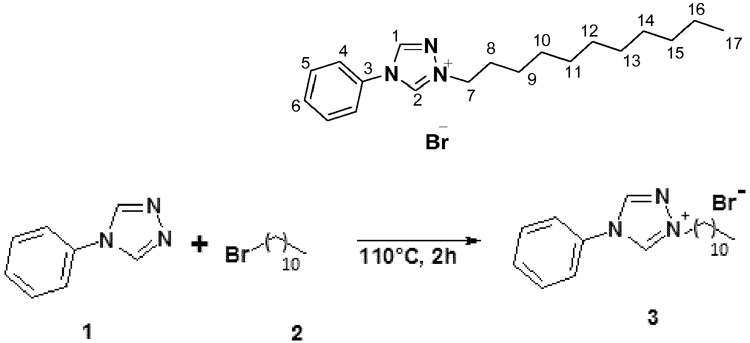

| Substanz | **1** | **2** |
|---|---|---|
| m/V [g/ml] | 0,15 g | 0,486 |
| M [g*mol⁻¹] | 145,17 | 235,20 |
| v[eq] | 1 | 3 |
| n [mol] | 0,001 | 0,003 |

0,15 g (0,001 mol) 4-Phenyltriazol **1** und 0,486 g (0,003 mol) 1-Bromundecan **2** werden in einem Druckrohr vereinigt. Es wird 12h bei 110°C gerührt. Anschließend wird auf Raumtemperatur abgekühlt. Der ausgefallene Feststoff wird in einer 1:1-Mischung aus THF und Petrolether aufgenommen, abfiltriert und mit Diethylether gewaschen.
Mm 380,37 C₁₉H₃₀N₃Br
**Ausbeute:** 0,242 g (64 %)
**Schmelzpunkt**
150,7°C

### Ausführungsbeispiel 88

### 4-Phenyl-1-(tetradec-1-yl)-(1,2,4)-triazoliumbromid

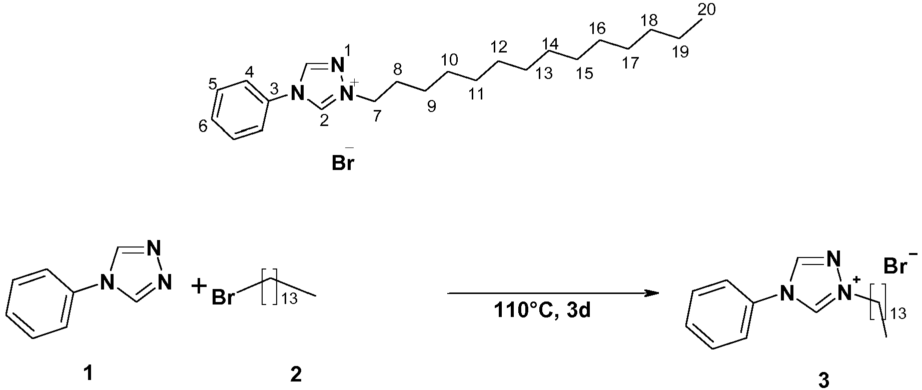

| Substanz | **1** | **2** |
|---|---|---|
| m/V [g/ml] | 0,1 g | 0,572 |
| M [g*mol⁻¹] | 145,17 | 277 |
| v[eq] | 1 | 3 |
| n [mol] | 0,0007 | 0,0021 |

0,100 g (0,0007 mol) 4-Phenyl-(1,2,4)-triazol **1** und 0,572 g (0,0021 mol) 1-Bromtetradecan **2** werden in einem Druckrohr vereinigt. Es wird 3d bei 110°C gerührt. Zu dem entstandenen Feststoff wurden 10 ml Petrolether gegeben. Das Produkt wird abfiltriert, mit Petrolether/THF 1:1 und Diethylether gewaschen und im Hochvakuum getrocknet.

Mm 422,53 C₂₂H₃₆N₃Br

**Ausbeute:** 0,242 g (74%)
**¹H-NMR DM-233 (300MHz/DMSO) :**
δ (ppm)=0,85 (t, 3H, 20-H); 1,24 (m, 24H, 9-19-H); 1,94 (q, 2H, 8-H); 4,42 (t, 2H, 7-H); 7,71 (m, 3H, 5/5'/6-H); 7,82 (d, 2H, 4/4'-H); 9,78 (s, 1H, 1-H); 10,79 (s, 1H, 2-H)
**¹³C-NMR DM-233 (75,475MHz/DMSO) :**
δ (ppm)=13,94 (20-C); 22,07 (19-C); 25,43 (18-C); 28,40 u. 28,68 u. 28,79 u. 28,94 u. 28,99 u. 29,03 (9-17-C); 31,27 (8-C); 51,96 (7-C); 122,45 (5/5'-C); 130,18 (4/4'-C); 130,45 (6-C); 132,17 (3-C); 141,45 (2-C); 142,85 (1-C)
**Schmelzpunkt**
153,7 °C

### Ausführungsbeispiel 89

### 4-Phenyl-1-(prop-1-yl)triazoliumbis(trifluormethylsulfon)amid

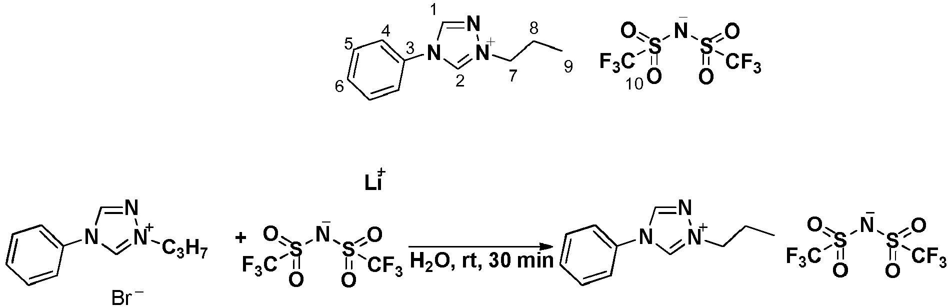

| Substanz | **1** | **2** |
|---|---|---|
| m/V [g/ml] | 0,106 g | 0,113 |
| M [g*mol⁻¹] | 268,16 | 287,08 |
| v[eq] | 1 | 1 |
| n [mol] | 0,00039 | 0,00039 |

0,106 g (0,00039 mol) 4-Phenyl-1-(prop-1-yl)triazoliumbromid **1** werden in wenig Wasser gelöst. Anschließend werden 0,113 g (0,00039 mol) Lithiumbis(trifluormethylsulfon)amid zugegeben und es wird 30 min. bei Raumtemperatur gerührt. Die sich bildende zweite Phase wird durch Zugabe von Dichlormethan vergrößert. Die Phasen werden separiert und die wässrige zweimal mit Dichlormethan gewaschen. Die vereinigten organischen Phasen werden über Natriumsulfat getrocknet. Das Lösungsmittel wird im Vakuum entfernt und das Produkt als beiger Feststoff gewonnen.

Mm 468,4 C₁₃H₁₄ F₆N₄O₄S₂

**Ausbeute:** 0,164 g (90%)
**¹H-NMR DM-272 (300MHz/DMSO) :**
δ (ppm)=0,98 (t, 3H, 9-H); 1,97 (dt, 2H, 8-H); 4,40 (t, 2H, 7-H); 7,71 (m, 3H, 5/5'/6-H); 7,81 (d, 2H, 4/4'-H); 9,77 (s, 1H, 1-H); 10,74 (s, 1H, 2-H)
**¹⁹F-NMR DM-215 (282,4MHz/DMSO) :**
δ (ppm)=-78,71 (s, 10-F);
**Schmelzpunkt:**
72,6°C

### Ausführungsbeispiel 90

### 1-Phenyl-4-(pent-1-yl)triazoliumbis(trifluormethyl)sulfonamid

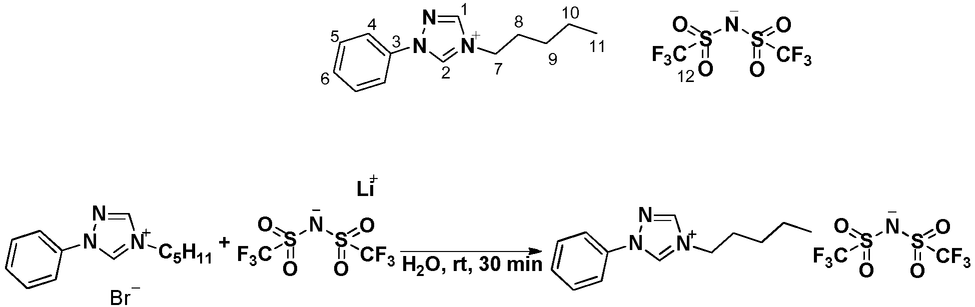

| Substanz | **1** | **2** |
|---|---|---|
| m/V [g/ml] | 0,116 g | 0,113 |
| M [g*mol⁻¹] | 296,26 | 287,08 |
| v[eq] | 1 | 1, |
| n [mol] | 0,00039 | 0,00039 |

0,116 g (0,00039 mol) 1-Phenyl-4-(pent-1-yl)triazoliumbromid **1** werden in wenig Wasser gelöst. Anschließend werden 0,113 g (0,00039 mol) Lithiumbis(trifluormethylsulfon)amid zugegeben und es wird 30 min. bei Raumtemperatur gerührt. Die sich bildende zweite Phase wird durch Zugabe von Dichlormethan vergrößert. Die Phasen werden separiert und die wässrige zweimal mit Dichlormethan gewaschen. Die vereinigten organischen Phasen werden über Natriumsulfat getrocknet. Das Lösungsmittel wird im Vakuum entfernt und das beiger Feststoff gewonnen.

Mm 496,45 C₁₅H₁gF₆N₄O₄S₂

**Ausbeute:** quant.
**¹H-NMR DM-273 (300MHz/DMSO) :**
δ (ppm)=0,92 (t, 3H, 11-H); 1,36 (m, 4H, 9/10-H); 1,95 (q, 2H, 8-H); 4,31 (t, 2H, 7-H); 7,71 (m, 3H, 5/5'/6-H); 7,93 (d, 2H, 4/4'-H); 9,47 (s, 1H, 1-H); 10,89 (s, 1H, 2-H)
**¹⁹F-NMR DM-273 (282,4MHz/DMSO) :**
δ (ppm)=-78,72 (s, 12-F);
**Schmelzpunkt**
45,4 °C

### Ausführungsbeispiel 91

### 4-Phenyl-1-(hex-1-yl)triazoliumbis(trifluormethyl)sulfonamid

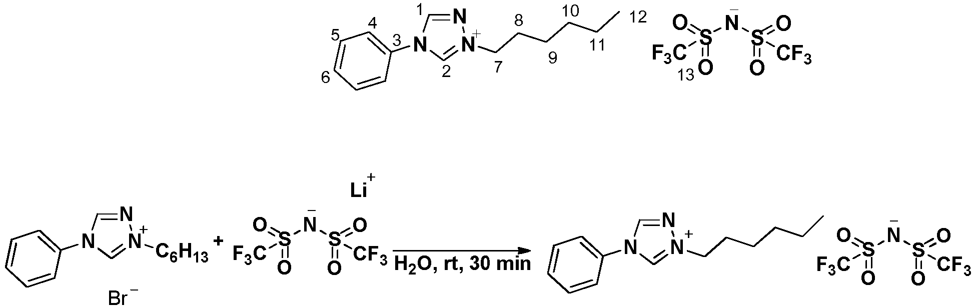

| Substanz | **1** | **2** |
|---|---|---|
| m/V [g/ml] | 0,121 g | 0,113 |
| M [g*mol⁻¹] | 310,24 | 287,08 |
| v[eq] | 1 | 1, |
| n [mol] | 0,00039 | 0,00039 |

0,121 g (0,00039 mol) 1-Phenyl-4-(hex-1-yl)triazoliumbromid **1** werden in wenig Wasser gelöst. Anschließend werden 0,113 g (0,00039 mol) Lithiumbis(trifluormethylsulfon)amid zugegeben und es wird 30 min. bei Raumtemperatur gerührt. Die sich bildende zweite Phase wird durch Zugabe von Dichlormethan vergrößert. Die Phasen werden separiert und die wässrige zweimal mit Dichlormethan gewaschen. Die vereinigten organischen Phasen werden über Natriumsulfat getrocknet. Das Lösungsmittel wird im Vakuum entfernt und das Produkt als beiger Feststoff gewonnen.

Mm 510,47 C₁₆H_{2O}F₆N₄O₄S₂

**Ausbeute:** 0,165 g (83%) ¹H-NMR DM-274 (300MHz/DMSO) :
δ (ppm)=0,88 (t, 3H, 12-H); 1,33 (m, 6H, 9/10/11-H); 1,94 (q, 2H, 8-H); 4,42 (t, 2H, 7-H); 7,71 (m, 3H, 5/5'/6-H); 7,81 (d, 2H, 4/4'-H); 9,76 (s, 1H, 1-H); 10,73 (s, 1H, 2-H)
¹⁹F-NMR DM-274 (282,4MHz/DMSO) :
δ (ppm)=-78,71 (s, 13-F);
Schmelzpunkt
36 °C

### Ausführungsbeispiel 92

### 4-Phenyl-1-(hept-1-yl)triazoliumbis(trifluormethyl)sulfonamid

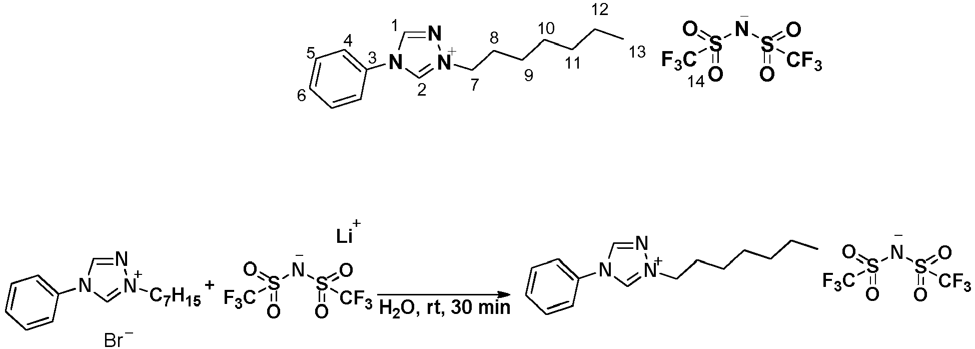

| Substanz | **1** | **2** |
|---|---|---|
| m/V [g/ml] | 0,127 g | 0,113 |
| M [g*mol⁻¹] | 324,26 | 287,08 |
| v[eq] | 1 | 1 |
| n [mol] | 0,00039 | 0,00039 |

0,127 g (0,00039 mol) 1-Phenyl-4-(hept-1-yl)triazoliumbromid **1** werden in wenig Wasser und einigen Tropfen Methanol gelöst. Anschließend werden 0,113 g (0,00039 mol) Lithiumbis(trifluormethylsulfon)amid zugegeben und es wird 30 min. bei Raumtemperatur gerührt. Die sich bildende zweite Phase wird durch Zugabe von Dichlormethan vergrößert. Die Phasen werden separiert und die wässrige zweimal mit Dichlormethan gewaschen. Die vereinigten organischen Phasen werden über Natriumsulfat getrocknet. Das Lösungsmittel wird im Vakuum entfernt und das Produkt als bräunlicher Feststoff gewonnen.

Mm 524,5 C₁₇H₂₂F₆N₄O₄S₂

**Ausbeute:** quant.
**¹H-NMR DM-275 (300MHz/DMSO) :**
δ (ppm)=0,87 (t, 3H, 13-H); 1,30 (m, 8H, 9/10/11/12-H); 1,94 (q, 2H, 8-H); 4,42 (t, 2H, 7-H); 7,71 (m, 3H, 5/5'/6-H); 7,81 (d, 2H, 4/4'-H); 9,76 (s, 1H, 1-H); 10,73 (s, 1H, 2-H)
**¹⁹F-NMR DM-275 (282,4MHz/DMSO) :**
δ (ppm)=-78,72 (s, 14-F);
**Schmelzpunkt**
44,4 °C

### Ausführungsbeispiel 93

### 4-Phenyl-1-(undec-1-yl)triazoliumbis(trifluormethylsulfon)amid

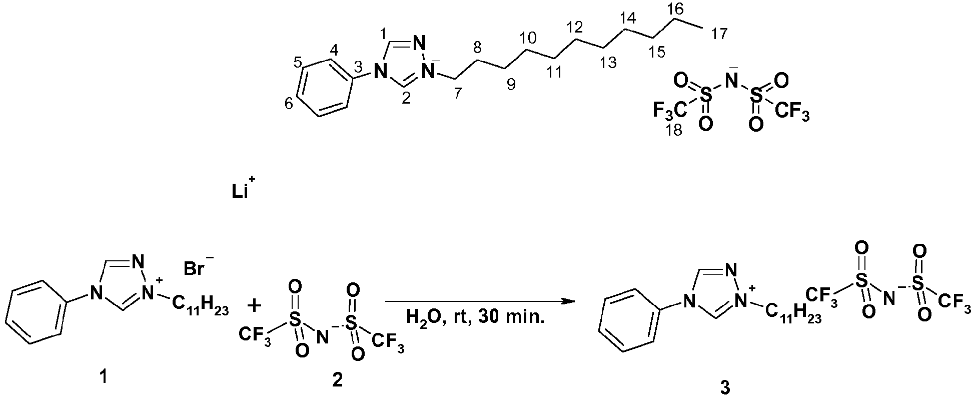

| Substanz | **1** | **2** |
|---|---|---|
| m/V [g/ml] | 0,15 g | 0,113 |
| M [g*mol⁻¹] | 380,37 | 287,08 |
| v[eq] | 1 | 1 |
| n [mol] | 0,00039 | 0,00039 |

0,150 g (0,00039 mol) 4-Phenyl-1-(undec-1-yl)triazoliumbromid 1 werden in wenig Wasser und Methanol gelöst. Anschließend werden 0,113 g (0,00039 mol) Lithiumbis(trifluormethylsulfon)amid zugegeben und es wird 30 min. bei Raumtemperatur gerührt. Die sich bildende zweite Phase wird durch Zugabe von Dichlormethan vergrößert. Die Phasen werden separiert und die wässrige zweimal mit Dichlormethan gewaschen. Die vereinigten organischen Phasen werden über Natriumsulfat getrocknet. Das Lösungsmittel wird im Vakuum entfernt und das Produkt als farbloser Feststoff gewonnen.

Mm 580,61 C₂₁H₃₀F₆N₄O₄S₂

**Ausbeute:** 0,188 g (83%)
**¹H-NMR DM-276 (300MHz/DMSO) :**
δ (ppm)=0,85 (t, 3H, 17-H); 1,27 (m, 16H, 9/10/11/12/13/14/15/16-H); 1,94 (q, 2H, 8-H); 4,42 (t, 2H, 7-H); 7,71 (m, 3H, 5/5'/6-H); 7,81 (d, 2H, 4/4'-H); 9,76 (s, 1H, 1-H); 10,73 (s, 1H, 2-H)
**¹⁹F-NMR DM-276 (282,4MHz/DMSO) :**
δ (ppm)=-78,71(s, 18-F);
**Schmelzpunkt**
50,7 °C

### Ausführungsbeispiel 94

### 4-Phenyl-1-(tetradec-1-yl)triazoliumbis(trifluormethylsulfon)amid

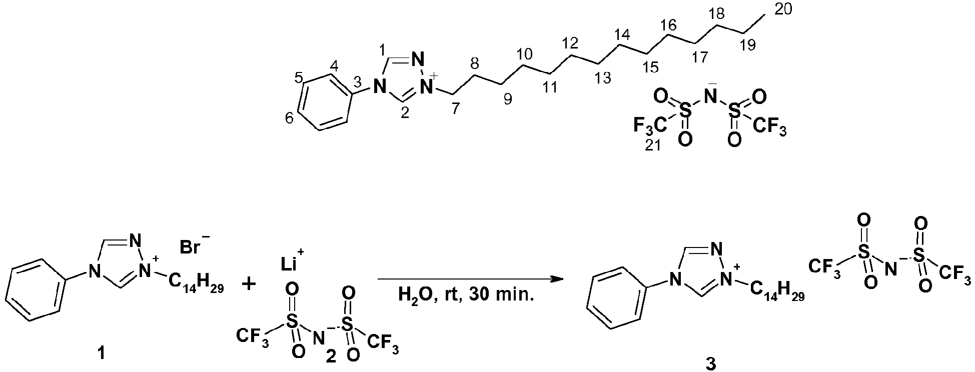

| Substanz | **1** | **2** |
|---|---|---|
| m/V [g/ml] | 0,166 g | 0,113 |
| M [g*mol⁻¹] | 442,45 | 287,08 |
| v[eq] | 1 | 1 |
| n [mol] | 0,00039 | 0,00039 |

0,166 g (0,00039 mol) 4-Phenyl-1-(tetradec-1-yl)triazoliumbromid 1 werden in wenig Wasser und Methanol gelöst. Anschließend werden 0,113 g (0,00039 mol) Lithiumbis(trifluormethylsulfon)amid zugegeben und es wird 30 min. bei Raumtemperatur gerührt. Die sich bildende zweite Phase wird durch Zugabe von Dichlormethan vergrößert. Die Phasen werden separiert und die wässrige zweimal mit Dichlormethan gewaschen. Die vereinigten organischen Phasen werden über Natriumsulfat getrocknet. Das Lösungsmittel wird im Vakuum entfernt und das Produkt als bräunlicher Feststoff gewonnen.

Mm 622,69 C₂₄H₃₆F₆N₄O₄S₂

**Ausbeute:** 0,196 g (81%)
**¹H-NMR DM-277 (300MHz/DMSO) :**
δ (ppm)=0,85 (t, 3H, 20-H); 1,26 (m, 22H, 9/10/11/12/13/14/15/16/17/18/19-H); 1,94 (q, 2H, 8-H); 4,42 (t, 2H, 7-H); 7,71 (m, 3H, 5/5'/6-H); 7,80 (d, 2H, 4/4'-H); 9,76 (s, 1H, 1-H); 10,73 (s, 1H, 2-H)
**¹⁹F-NMR DM-277 (282,4MHz/DMSO) :**
δ (ppm)=-78,72(s, 21-F);
**Schmelzpunkt**
67,7°C

### Ausführungsbeispiel 95

### Wasserstoffspeicherung

Es wird für 10 Minuten Wasserstoff durch 5 g der oben genannten ionischen Flüssigkeit 3-Mesityl-1-octyl-imidazoliumtetrafluoroborat (Ausführungsbeispiel 62) geleitet. Danach wird unter reduziertem Druck der gespeicherte Wasserstoff freigesetzt.

Es werden 0,511 g Wasserstoff freigesetzt. Das entspricht einer Wasserstoffspeicherkapazität der ionischen Flüssigkeit 3-Mesityl-1-octyl-imidazoliumtetrafluoroborat von 10,22 Gewichts-Prozent.

### Ausführungsbeispiel 96

### Löslichkeit in Ether bzw. THF

Zu 80 mg 1-Mesityl-3-tetradecylimidazoliumtetrafluoroborat (Ausführungsbeispiel 64) werden 4 ml Diethylether bzw. 2 ml Tetrahydrofuran gegeben.

1-Mesityl-3-tetradecylimidazoliumtetrafluoroborat löst sich dabei nicht in Diethylether, aber hervorragend in Tetrahydrofuran.

### Ausführungsbeispiele 97 - 123

Weitere synthetisierte Verbindungen sind in Tab. 1 und 2 aufgeführt. Die hergestellten Verbindungen wurden durch NMR-Spektroskopie und/oder Bestimmung des Schmelzpunkts charakterisiert.

**Tab. 1 Halogenide**

| **Name/Molekulargewicht/ Ausführungsbeispiel** | **Struktur** | **1H-NMR** | **13C-NMR** | **Smp [°C]** |
|---|---|---|---|---|
| 1-(4-Brom-phenyl)-3-butyl-imidazoliumbromid | 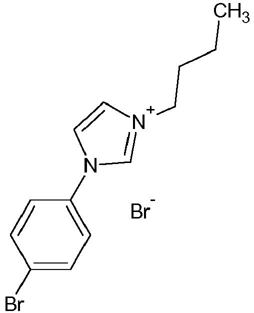 | 9.93 ppm (1H, s, NCHN), 8.38 ppm (1H, s, NCCH), 8.09 ppm (1H, s, NCCH), 7.9 ppm (2H, m, CH aromat.), 7.8 ppm (2H, m, CH aromat.), 4.36 ppm (2H, t, N-CH₂), 1.86 ppm (2H, m, CH₂), 1.35 ppm (2H, m, CH₂), 0.95 ppm (3H, t, CH₃) | | 161,3 |
| 360.088 g/mol | | | | |
| 97 | | | | |
| 1-(4-Methyl-phenyl)-3-hexylimidazoliumbromid | 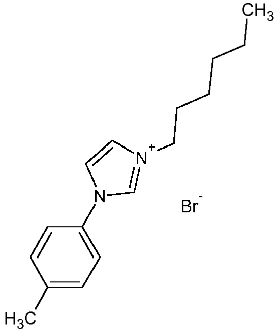 | 10.05 ppm (1H, s, NCHN); 8.36 ppm (1H, m, NCHC); 8.12 ppm (1H, m, NCHC); 7.72 ppm (2H, d, J=9 Hz, Ar-H); 7.43 ppm (2H, d, H=9 Hz, Ar-H); 4.28 ppm (2H, t, J=7 Hz, N-CH2); 2.38 ppm (3H, s, Ar-CH3); 1.89 ppm (2H, m, CH2); 1.29 ppm (6H, m, 3xCH2); 0.85 ppm (3H, t, J=7 Hz, C-CH3); in DMSO | - | |
| 323.270 g/mol | | | | |
| 98 | | | | |
| 1-(2-Methylphenyl)-3-propylimidazoliumbromid | 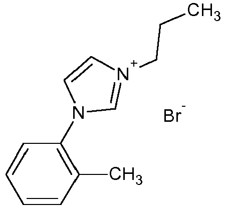 | - | - | 101,8 |
| 281.192 g/mol | | | | |
| 99 | | | | |
| 1-(2-Methylphenyl)-3-hexylimidazoliumbromid | 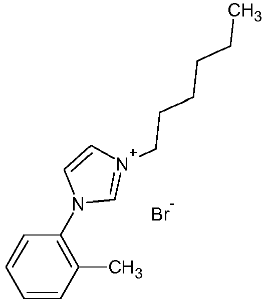 | 9.69 ppm (1H, s, NCHN); 8.16 ppm (1H, s, NCHC); 8.156 ppm (1H, s, NCHC); 7.61 ppm (3H, m, Ar-H); 7.55 ppm (2H, m, Ar-H); 4.35 ppm (2H, t, J=7 Hz, N-CH2); 2.29 ppm (3H, s, Ar-CH3); 1.96 ppm (2H, m, CH2); 1.37 ppm (6H, m, 3xCH2); 0.94 ppm (3H, m, CH3); in DMSO | - | |
| 323.271 g/mol | | | | |
| 100 | | | | |
| 1-(4-Methyl-phenyl)-3-hexylimidazoliumiodid | 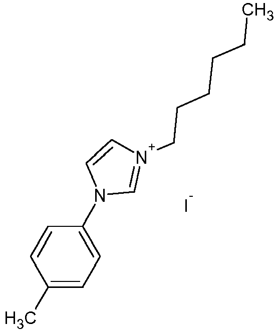 | 9.87 ppm (1H, s, NCHN); 8.36 ppm (1H, s, NCHC); 8.12 ppm (1H, s, NCHC); 7.74 ppm (2H, d, J=8 Hz, Ar-H); 7.52 ppm (2H, d, J=8 Hz, Ar-H); 4.31 ppm (2H, t, J=7 Hz, N-CH2); 2.46 ppm (3H, s, Ar-CH3); 1.95 ppm (2H, m, CH2); 1.37 ppm (6H, m, 3xCH2); 0.93 ppm (3H, m, CH3); in DMSO | - | |
| 370,272 g/mol | | | | |
| 101 | | | | |
| 1-(2-Methoxy-phenyl)-3-hexylimidazoliumiodid | 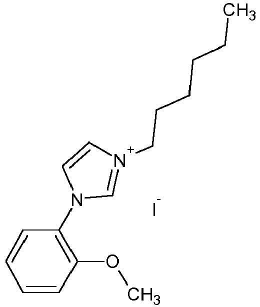 | 9.65 ppm (1H, s, NCHN); 8.13 ppm (1H, s, NCHC); 8.09 ppm (1H, s, NCHC); 7.68 ppm (2H, m, Ar-H); 7.44 ppm (1H, m, Ar-H); 7.26 ppm (1H, m, Ar-H); 4.33 ppm (2H, t, J=7 Hz, N-CH2); 3.95 ppm (3H, s, OCH3); 1.94 ppm (2H, m, CH2); 1.36 ppm (6H, m, 3xCH2); 0.94 ppm (3H, m, CH3); in DMSO | - | 72,5 |
| 386,271 g/mol | | | | |
| 102 | | | | |
| 1-(4-Brom-phenyl)-3-hexylimidazoliumiodid | 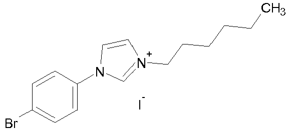 | 9.89 ppm (1H, s, NCHN); 8.39 ppm (1H, s, NCHC); 8.12 ppm (1H, s, NCHC); 7.97 ppm (2H, d, J=9 Hz, Ar-H); 7.83 ppm (2H, d, J=9 Hz, Ar-H); 4.30 ppm (2H, t, J=7.5 Hz, N-CH2); 1.95 ppm (2H, m, CH2); 1.38 ppm (6H, m, 3xCH2); 0.94 ppm (3H, m, CH3); in DMSO | - | 95,2 |
| 435.141 g/mol | | | | |
| 103 | | | | |
| 1-(4-Brom-phenyl)-3-butylimidazoliumiodid | 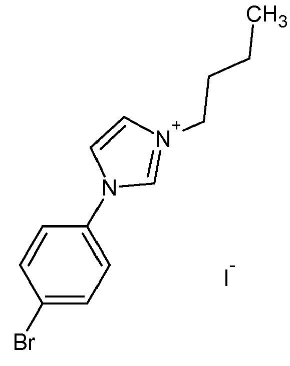 | 9.84 ppm (1H, s, NCHN); 8.33 ppm (1H, s, NCHC); 8.06 ppm (1H, s, NCHC); 7.90 ppm (2H, d, J=9 Hz, Ar-H); 7.76 ppm (2H, d, J=9 Hz, Ar-H); 4.25 ppm (2H, t, J=7 Hz, N-CH2); 1.88 ppm (2H, m, CH2); 1.34 ppm (2H, m, CH2); 0.94 ppm (3H, t, J=7 Hz, CH3); in DMSO | - | 118,6 |
| 407.088 g/mol | | | | |
| 104 | | | | |
| 1-(4-Methoxy-phenyl)-3-butyl-imidazoliumiodid | 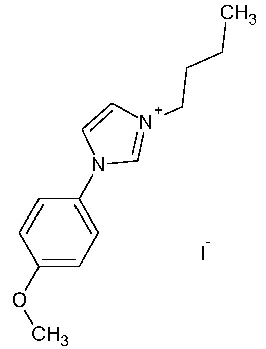 | - | - | |
| 358.218 g/mol | | | | |
| 105 | | | | |
| 1-(4-Chlor-phenyl)-3-butyl-imidazoliumiodid | 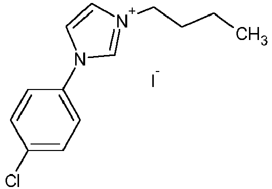 | 9,85 ppm (1H, s, NCHN), 8,34 ppm (1H, s, NCHC), 8,07 ppm (1H, s, NCHC), 7,85 ppm (2H, d, J=9 Hz, CH) 7,78 ppm (2H, d, J=9 Hz, CH) 4,26 ppm (2H, t, J=7,5 Hz, N-CH2), 1,88 ppm (2H, m, CH2), 1,34 ppm (2H, m, CH2), 0,94 ppm (3H, t, J=7,4 Hz, CH3); in DMSO | 135,5 ppm (NCHN), 134,1 ppm (C-Aromat), 133,6 ppm (C-Aromat), 130 ppm (2CH), 123,8 ppm (2CH), 123,3 ppm (NCHC), 121,2 ppm (NCHC), 49,2 ppm (N-CH2), 31,0 ppm (CH2), 18,8 ppm (CH2), 13,3 ppm (CH3); in DMSO | 112,2 |
| 362.637 g/mol | | | | |
| 106 | | | | |
| 1-(2-Methylphenyl)-3-butyl-imidazoliumiodid | 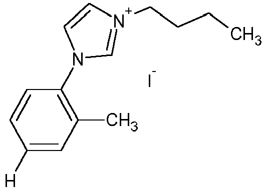 | 9.56 ppm (1H, s, NCHN); 8.09 ppm (2H, m, NCHC); 7.54 ppm (4H, m, Ar-H); 4.29 ppm (2H, t, J=7 Hz, N-CH2); 1.89 ppm (2H, m, CH2); 1.34 ppm (2H, m, CH2); 0.95 ppm (3H, t, J=7 Hz, CH3); in DMSO | 136,9 ppm (NCHN), 124,2 ppm (C-Aromat), 133,3 ppm (C-Aromat), 131,5 ppm (CH), 130,6 ppm (CH), 127,3 ppm (CH), 126,5 ppm (CH), 123,8 ppm (CH), 122,7 ppm (CH), 48,9 ppm (NCH2), 31,1 ppm (CH2), 18,8 ppm (CH2), 17,0 ppm (Ar-CH3), 13,3 ppm (CH3); in DMSO | 70 |
| 342.219 g/mol | | | | |
| 107 | | | | |
| 1-(4-Ethylphenyl)-3-butylimidazoliumiodid | 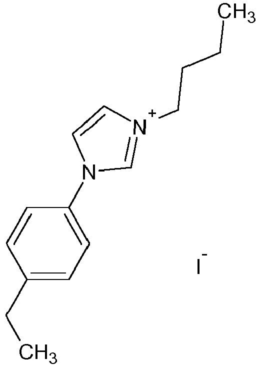 | 9.78 ppm (1H, s, Ar-H); 8.30 ppm (1H, m, Ar-H); 8.05 ppm (1H, m, Ar-H); 7.71 ppm (2H, d, J=8 Hz, Ar-H); 7.51 ppm (2H, d, J=8 Hz, Ar-H); 4.25 ppm (2H, t, J=7 Hz, N-CH2); 2.71 ppm (2H, q, J=7 Hz, Ar-CH2); 1.88 ppm (2H, m, CH2); 1.35 ppm (2H, m, CH2); 1.26 ppm (3H, t, J=7 Hz, Ar-C-CH3); 0.94 ppm (3H, t, J=7 Hz, CH3); in DMSO | 145,8 ppm (C-Aromat); 135,1 ppm (NCHN); 132,6 ppm (C-Aromat); 129,3 ppm (2CH); 123,2 ppm (CH); 121,8 ppm (2CH); 121,2 ppm (CH); 49,1 ppm (CH2); 31,1 ppm (CH2); 31,1 ppm (CH2); 27,6 ppm (CH2); 15,5 ppm (CH3); 13,3 ppm (CH3); DMSO | 75,6 |
| 3356.245 g/mol | | | | |
| 108 | | | | |
| 1-(4-Ethylphenyl)-3-hexylimidazoliumiodid | 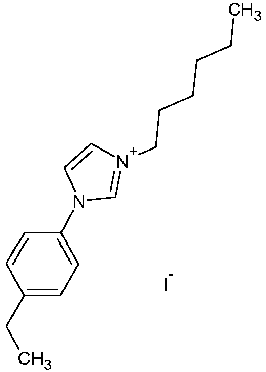 | 9.77 ppm (1H, s, NCHN); 8.29 ppm (1H, m, NCHC); 8.04 ppm (1H, m, NCHC); 7.70 ppm (2H, d, J=9 Hz, Ar-H); 7.51 ppm (2H, d, J=9 Hz, Ar-H); 4.23 ppm (2H, t, J=7 Hz, N-CH2); 2.70 ppm (2H, q, J=7.6 Hz, Ar-CH2), 1.88 ppm (2H, t, J=6.5 ppm, CH2), 1.31 ppm (6H, m, 3xCH2), 1.22 ppm (3H, t, J=7.6 Hz, Ar-C-CH3), 0.87 ppm (3H, t, J=6.5 Hz, CH3), in DMSO | 145,8 ppm (C-Aromat); 135,1 ppm (NCHN); 132,6 ppm (C-Aromat); 129,4 ppm (2CH); 123,2 ppm (CH); 121,8 ppm (2CH); 121,1 ppm (CH); 49,3 ppm (CH2); 30,6 ppm (CH2); 29,1 ppm (CH2); 27,6 ppm (CH2); 21,8 ppm (CH2); 15,5 ppm (CH2); 13,8 ppm (CH3); DMSO | 76,1 |
| 384,298 g/mol | | | | |
| 109 | | | | |
| 1-(2-Ethylphenyl)-3-butylimidazoliumiodid | 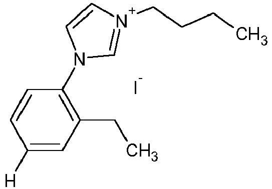 | 9,56 ppm (1H, s, NCHN); 8,08 ppm (2H, m, NCHC); 7,65-7,45 ppm (4H, m, CH-Aromat); 4,28 (2H, t, J=7 Hz, N-CH2); 2,48 ppm (2H, m, Ar-CH2); 1,89 ppm (2H, m, CH2); 1,33 ppm (2H, m, CH2); 1,08 ppm (3H, t, J=7 Hz, Ar-C-CH3); 0,95 ppm (3H, t, J=7,2 Hz, CH3); DMSO | 139,2 ppm (1C-Aromat); 137,1 ppm (NCHN); 133,6 ppm (1C-Aromat); 131,0 ppm (CH); 129,0 ppm (CH); 127,3 ppm (CH); 127,0 ppm (CH); 124,3 ppm (CH); 122,8 ppm (CH); 49,0 ppm (N-CH2); 31,1 ppm (CH2); 23,1 ppm (CH2); 18,8 ppm (CH2); 14,4 ppm (CH3); 13,3 ppm (CH3); DMSO | 83,5 |
| 356,245 g/mol | | | | |
| 110 | | | | |
| 1-(2-Ethylphenyl)-3-hexyl-imidazoliumiodid | 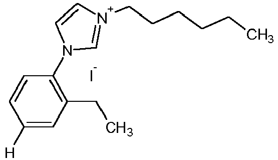 | 9,57 ppm (1H, s, NCHN); 8,08 ppm (2H, s, NCHC); 7,65-7,45 ppm (4H, m, CH-Aromat); 4,27 ppm (2H, t, J=7 Hz, N-CH2), 2,46 ppm (2H, q, J=7,5 Hz, Ar-CH2); 1,89 ppm (2H, m, CH2), 1,30 ppm (6H, m, 3 CH2); 1,08 ppm (3H, t, J=7,5 Hz, Ar-C-CH3); 0,88 ppm (3H, t, J=6,5 Hz); DMSO | 139,2 ppm (1C-Aromat); 137,1 ppm (NCHN); 133,6 ppm (1C-Aromat); 131,0 ppm (1CH); 129,8 ppm (1CH); 127,3 ppm ((CH); 126,9 ppm (1CH); 124,3 ppm (1CH); 122,8 ppm (1CH);49,3 ppm (1CH2); 30,5 ppm (1CH2); 29,0 ppm (1CH2); 25,9 ppm (1CH2); 23,2 (1CH2); 21,9 (1CH2); 14,4 (1CH3); 13,8 ppm (1CH3); DMSO | 57,1 |
| 384,298 g/mol | | | | |
| 111 | | | | |
| 1-(2-Ethylphenyl)-3-butyl-imidazoliumbromid | 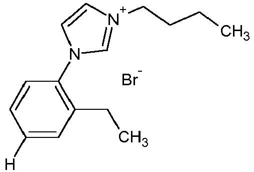 | 9,64 ppm (1H, s, NCHN); 8,11 ppm (2H, m, NCHC); 7,56 ppm (3H, m, CH-Aromat); 7,48 (1H, m, CH-Aromat); 4,31 ppm (2H, t, J= 7Hz, N-CH2); 2,48 ppm (2H, q, J=7,5 Hz, Ar-CH2); 1,89 ppm (2H, m, CH2); 1,33 ppm (2H, m, CH2); 1,08 ppm (3H, t, J=7,5 Hz, Ar-C-CH3); 0,94 ppm (3H, t, J=7,3 Hz, CH3); DMSO | 139,2 ppm (C-Aromat); 137,1 (1CH); 133,6 (C-Aromat); 130,9 ppm (1CH); 129,8 ppm (1CH); 127,3 ppm (1CH); 126,9 ppm (1CH); 124,2 ppm (1CH); 122,8 ppm (1CH); 48,9 ppm (N-CH2); 31,1 ppm (CH2); 23,2 ppm (2CH2); 18,7 ppm (2CH2); 14,3 ppm (Ar-C-CH3); 13,3 ppm (CH3); DMSO | 64,5 |
| 309,245 g/mol | | | | |
| 112 | | | | |
| 1-(2-Ethylphenyl)-3-hexyl-imidazoliumbromid | 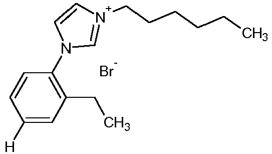 | 9,62 ppm (1H, m, NCHN); 8,10 (2H, s, NCHC); 7,60 ppm (3H, m, CH-Aromat); 7,48 (1H, m, CH-Aromat); 4,29 ppm (2H, t, J=7,2 Hz); 2,48 ppm (2H, q, J=7,4 Hz, CH2); 1,90 ppm (2H, m, CH2); 1,31 ppm (6H, m, 3 CH2); 1,08 ppm (3H, t, J=7,6 Hz, Ar-C-CH3); 0,88 ppm (3H, t, J=6,4 Hz, CH3); DMSO | 139,2 ppm (C-Aromat); 137,1 ppm (NCHN); 133,6 (C-Aromat); 130,9 ppm (CH); 129,8 ppm (CH); 127,3 (CH); 126,9 ppm (CH); 124,2 ppm (CH); 122,8 ppm (CH); 49,2 ppm (N-CH2); 30,5 ppm (CH2); 29,0 ppm (CH2); 25,1 ppm (CH2); 23,1 ppm (CH2); 21,9 ppm (CH2); 14,3 ppm (CH3); 13,8 ppm (CH3); DMSO | 87,8 |
| 337,297 g/mol | | | | |
| 113 | | | | |
| 1-(2-Ethylphenyl)-3-propyl-imidazoliumbromid | 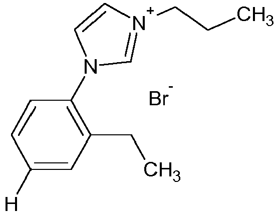 | 9,61 ppm (1H, s, NCHN); 8,09 ppm (2H, s, NCHC); 7,58 ppm (3H, m, CH-Aromat); 7,48 ppm (1H, m, CH-Aromat); 4,26 ppm (2H, t, J= 7,2 Hz, N-CH2); 2,48 ppm (2H, q, J=7,5 ppm, Ar-CH2); 1,92 ppm (2H, m, CH2); 1,08 ppm (3H, t, J=7,6 Hz, Ar-C-CH3); 0,92 ppm (3H, t, J=7,4 Hz, CH3); DMSO | 139,2 ppm (C-Aromat); 137,1 (NCHC); 133,6 ppm (CH); 130.9 ppm (CH); 129,8 ppm (CH); 127,3 ppm (CH); 127,0 ppm (CH); 124,2 ppm (CH); 122,8 ppm (CH); 50,7 ppm (N-CH2); 23,2 ppm (CH2); 22,6 ppm (CH2); 14,3 ppm (CH3); 10,4 ppm (CH3); DMSO | 114,1 |
| 295,218 g/mol | | | | |
| 114 | | | | |
| 1-(4-Ethylphenyl)-3-butylimidazoliumbromid | 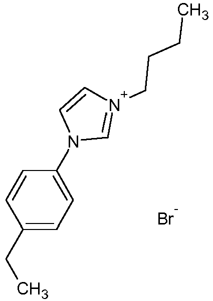 | | | |
| 309,245 g/mol | | | | |
| 115 | | | | |
| 1-(4-Ethylphenyl)-3-hexyl-imidazoliumbromid | 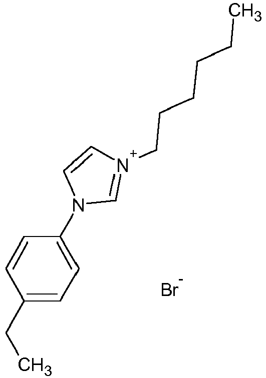 | 9,86 ppm (1H, s, NCHN); 8,32 ppm (1H, s, NCHC); 8,07 ppm (1H, s, NCHC); 7,72 ppm (2H, d, J=7,5 Hz; Ar-H); 7,50 ppm (2H, d, J=7,5 Hz; Ar-H); 4,25 ppm (2H, t, J=7,4 Hz, N-CH2); 2,71 ppm (2H, q, J=7,5 ppm, Ar-CH2); 1,89 ppm (2H, m, CH2); 1,32 ppm (6H, m, 3*CH2); 1,22 ppm (3H, t, J=7,5 Hz, Ar-C-CH3); 0,88 ppm (3H, t, J=6,8 ppm, CH3); DMSO | - | 40-50 |
| 337,298 g/mol | | | | |
| 116 | | | | |
| 1-(2-Ethylphenyl)-3-undecylimidazoliumiodi d | 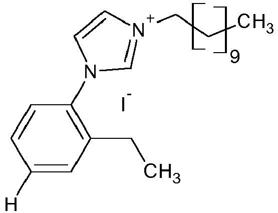 | 9,57 ppm (1H, s, NCHN); 8,09 ppm (2H, m, NCHC); 7,6 (3H, m, Ar-H); 7,48 ppm (1H, m, Ar-H); 4,78 ppm (2H, t, J=7,2 ppm, N-CH2); 2,48 ppm (2H, q, J=7,6 Hz, Ar-CH2); 1,89 ppm (2H, m, CH2); 1,25 ppm (16H, m, 8*CH2); 1,08 ppm (3H, t, J=7,6 Hz; Ar-C-CH3); 0,86 ppm (3H, t, J=6,8 Hz, CH3); DMSO | - | 61,7 |
| 454,431 g/mol | | | | |
| 117 | | | | |
| 1-(4-Ethoxy-phenyl)-3-butyl-imidazoliumiodi d | 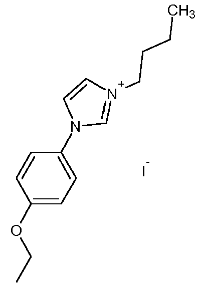 | - | - | 94,9 |
| 372,245 g/mol | | | | |
| 118 | | | | |
| 1-(4-Ethoxy-phenyl)-3-hexylimidazoliumiodi d | 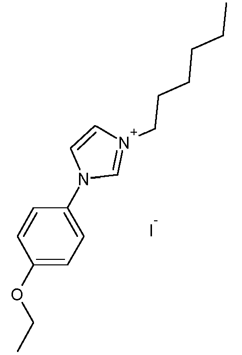 | - | - | 63,3 |
| 400,298 g/mol | | | | |
| 119 | | | | |
| 1-(4-Ethoxyphenyl)-3-undecylimidazoliumiodid 470,431 g/mol | 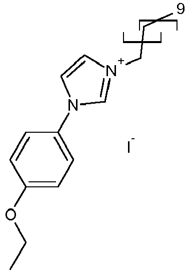 | - | - | 76,5 |
| 120 | | | | |
| 1-(4-Ethoxyphenyl)-3-butylimidazoliu m-bromid 325,244 g/mol | 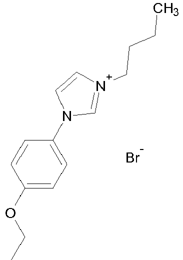 | | | 94,4 |
| 121 | | | | |

**Tab. 2 BTSA-Salze**

| **Name/Molekulargewicht/ Ausführungsbeispiel** | **Struktur** | **1H-NMR** | **13C-NMR** | **Smp [°C]** |
|---|---|---|---|---|
| 1-(4-Bromphenyl)-3-butylimidazoliu mBTSA | 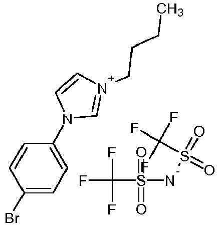 | 9,83 ppm (1H, s, NCHN); 8,33 ppm (1H, s, NCHC); 8,05 ppm (1H, s, NCHC); 7,90 ppm (2H, d, J=8,6 Hz, Ar-H); 4,25 ppm (2H, t, J=7,4 ppm, N-CH2); 1,88 ppm (2H, m, CH2); 1,34 ppm (2H, m, CH2); 0,94 ppm (3H, t, J=7,4 Hz, CH3); DMSO | - | - |
| 560.329 g/mol | | | | |
| 122 | | | | |
| 1-(4-Methylphenyl)-3-propylimidazoliumBT SA | 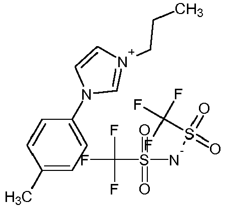 | 9,77 ppm (1H, s, NCHN); 8.29 ppm (1H, s, NCHC); 8.03 ppm (1H, s, NCHC); 7.68 ppm (2H, d, J=8 Hz, Ar-H); 7.47 ppm (2H, d, J=8 Hz, Ar-H); 4.22 ppm (2H, t, J=8 Hz, N-CH2); 2.41 ppm (3H, s, Ar-CH3); 1.93 ppm (2H, m, C-CH2-C); 0.94 ppm (3H, t, J=7 Hz, C-CH3); DMSO | - | 42 |
| 481.434 g/mol | | | | |
| 123 | | | | |

## Patentansprüche

1. Salze der allgemeinen Formel **(I),** 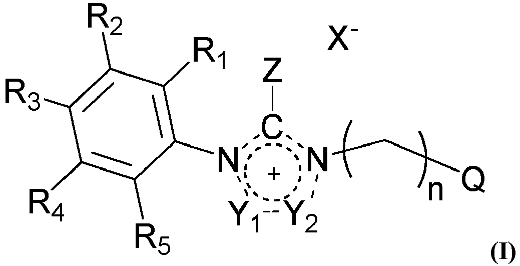 wobei
X⁻ ein Anion ist,
Y₁ und Y₂ CH sind oder Y₁ CH und Y₂ N ist oder Y₁ N und Y₂ CH ist,
n eine Zahl von 2 bis einschließlich 14 ist,
Q ausgewählt ist aus -CH₃, -OH, -ORₓ, -SO₃H, -SO₃Rₓ, -COOH, -COORₓ, -CORₓ, NH₂, - NHRₓ, N(Rₓ)₂, und -CH(Rₓ)₂,
Z H oder Rₓ ist,
R₁, R₂, R₃, R₄ und R₅ unabhängig voneinander -H, - -Cl, -Br, -I, -NO₂, -N(Rₓ)₂, -Rₓ, -COORₓ oder -ORₓ sind,
wobei Rₓ ein gegebenenfalls substituierter und / oder verzweigter C₁ bisC₁₈-Alkylrest ist,
ausgenommen Verbindungen der allgemeinen Formel **(I),** bei denen Y₁ und Y₂ CH sind und R₁, R₂, R₃, R₄ und R₅ H sind,
ausgenommen Verbindungen der allgemeinen Formel **(I),** bei denen Y₁ und Y₂ CH sind, R₁= R₃=R₅=CH₃ sind, n= 2 oder 6 ist und Q=CH₃ ist,
ausgenommen Verbindungen der allgemeinen Formel **(I),** bei denen Y₁ und Y₂ CH sind, R₁, R₂, R₄, R₅=H sind, R₃= ORₓ ist und Rₓ ein Kohlenwasserstoff mit 3 oder 12 Kohlenstoffatomen ist,
wobei das Anion X⁻ ausgewählt ist aus F⁻, Cl⁻, Br⁻, I⁻, CH₃COO⁻, CF₃COO⁻, CF_{3C}O₄⁻, SO₄²⁻, HSO₄⁻, CH₃OSO₃⁻, C₂H₅OSO₃⁻, SO₃²⁻, HSO₃⁻, AlCl₄⁻, Al₂Cl₇⁻, Al₃Cl₁₀⁻, AlBr₄⁻, NO₂⁻, NO3⁻, CuCl₂⁻, PO₄³⁻, HPO₄^{2-,} H₂PO₄⁻, CO₃²⁻, HCO₃⁻, PF₆⁻, BF₄⁻, (F₃CSO₂)₂N⁻ , P-CH₃C₆H₄SO₃⁻, OCN⁻, NCO⁻, SCN⁻, NCS⁻, B(CN)₄⁻, B(OR_{y})₄⁻, wobei R_{y} gegebenenfalls unabhängig voneinander ausgewählte substituierte und / oder verzweigte C₁ bis C₁₈-Alkylreste, C₁ bis C₁₈ Alkenylreste, C₁ bis C₁₈ Alkinylreste, C₆ bis C₁₂ Arylreste und / oder C₇ bis C₃₀ Aralkylreste, die gegebenenfalls ein oder mehrere, bevorzugt zwischen 1 und 4, Sauerstoff- und/oder Stickstoffatome als Heteroatome enthalten, sind, Borate der Grundstruktur B(O-A-O)₂⁻ gemäß den Formeln a) bis c); Borate der Grundstruktur BX₂(OR)⁻ oder BX₂(O-A-O)⁻ gemäß den Formeln d) bis i), 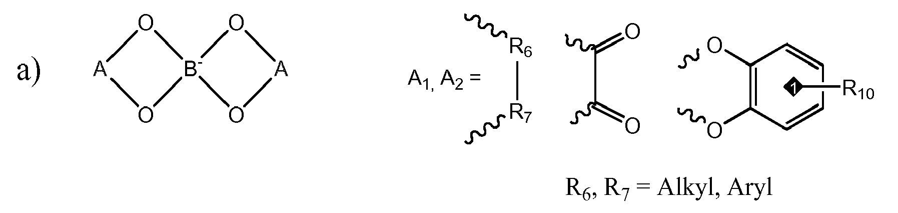 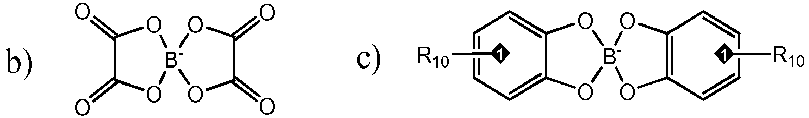 R₁₀ = Alkyl, Aryl, H, Halogen, -NO₂, -NH₂, -NHR₆, -N(R₆)₂, -COOR₆ oder -OR₆ 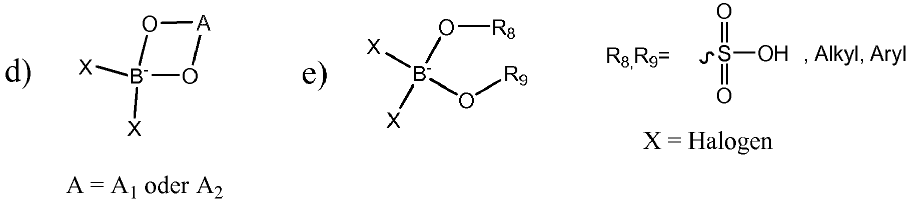 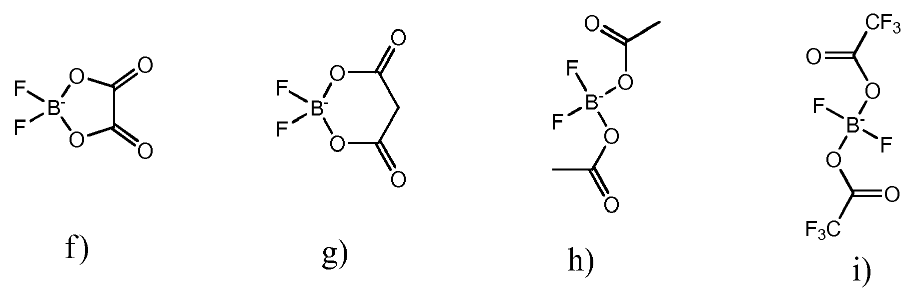 N(CF₃)₂⁻, N(CN)₂⁻ oder N(SO₂C_{z}F_{2z+1})₂⁻ wobei z eine natürliche Zahl zwischen 1 und 20 ist.

2. Salze nach Anspruch 1, **dadurch gekennzeichnet, dass** das Anion X⁻ ausgewählt ist aus Br⁻, I⁻ PF₆⁻, BF₄⁻ und (F₃CSO₂)₂N⁻.

3. Salz nach Anspruch 1 oder 2, **dadurch gekennzeichnet, dass** Y₁ und Y₂ CH sind, R₁, R₂, R₄ und R₅ H sind und R₃ ausgewählt ist aus -NO₂, -Cl, -Br, -COOEt, -CH₃, -OEt und -OMe.

4. Salz nach einem der Ansprüche 1 bis 3, **dadurch gekennzeichnet, dass** Y₁ N ist, Y₂ CH ist und R₁, R₂, R₃, R₄ und R₅ H sind.

5. Salz nach einem der Ansprüche 1 bis 3, **dadurch gekennzeichnet, dass** Y₁ CH ist, Y₂ N ist und R₁, R₂, R₃, R₄ und R₅ H sind.

6. Salz nach einem der Ansprüche 1 bis 5, **dadurch gekennzeichnet, dass** Z H oder -CH₃ ist.

7. Salz nach einem der Ansprüche 1 bis 6, **dadurch gekennzeichnet, dass** Q ausgewählt ist aus - CH₃, -OH, -OCH₃, -SO₃H, -SO₃Rₓ, -COOH, -COOCH₃, -COCH₃, NH₂, -NHCH₃, N(CH₃)₂, und -CH(CH₃)₂.

8. Verwendung eines Salzes nach einem der Ansprüche 1 bis 7, welches ein geeignetes Anion aufweist, als ionische Flüssigkeit.

9. Verwendung eines Salzes nach einem der Ansprüche 1 bis 7 in einer Batterie, einem Doppelschichtkondensator oder einer Brennstoffzelle.

10. Verwendung eines Salzes nach einem der Ansprüche 1 bis 7 als Lösungsmittel oder Lösungsmittelzusatz.

11. Verwendung eines Salzes nach einem der Ansprüche 1 bis 7 zur Speicherung von Wasserstoff.

12. Verfahren zur chemischen Umwandlung, umfassend ein Salz nach einem der Ansprüche 1 bis 7 als Lösungsmittel oder Lösungsmittelzusatz.

13. Verfahren zur Stofftrennung, umfassend ein Salz nach einem der Ansprüche 1 bis 7 als Lösungsmittel oder Lösungsmittelzusatz.

## Claims

1. Salts with the general formula (I), 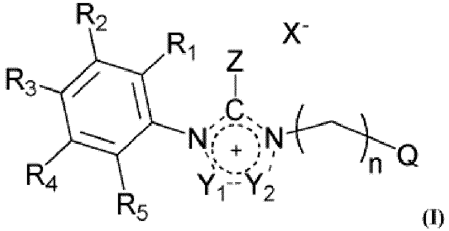 in which
X⁻ is an anion,
Y₁ and Y₂ are CH, or Y₁ is CH and Y₂ is N, or Y₁ is N and Y₂ is CH,
n is a number from 2 to 14 inclusive,
Q is selected from -CH₃, -OH, -ORₓ, -SO3H, -SO₃Rₓ, -COOH, -COORₓ, -CORₓ, NH₂, -NHR_{X}, N(R_{X})₂ and -CH(Rₓ₎ 2,
Z is H or Rₓ,
R₁, R₂, R₃, R₄, and R₅, independently of each other, are -H, -Cl, -Br, -I, -NO₂,-N(R_{X})₂, -Rₓ, -COORₓ or -ORₓ,
wherein Rₓ is an optionally substituted and/or branched C₁ to C₁₈ alkyl residue,
excluding compounds with the general formula (I) in which Y₁ and Y₂ are CH and R₁, R₂, R₃, R₄ and R₅ are H, excluding compounds with the general formula (I) in which Y₁ and Y₂ are CH, R_{1 =} R_{3 =} R_{5 =} CH₃, n = 2 or 6 and Q = CH₃,
excluding compounds with the general formula (I) in which Y₁ and Y₂ are CH, R₂, R₄, R_{5 =} H, R₃=ORₓ, and Rₓ is a hydrocarbon containing 3 or 12 carbon atoms,
in which the anion X⁻ is selected from F⁻, Cl⁻, Br⁻, I⁻, CH₃COO⁻, CF₃COO⁻, CF₃SO₃⁻, SO₄²⁻, HSO₄⁻, CH₃OSO₃⁻, C₂H₅OSO₃⁻, SO₃²⁻, HSO₃⁻, AlCl₄⁻, Al₂Cl₇⁻, Al₃Cl₁₀⁻, AlBr₄⁻, NO₂⁻, NO₃⁻, CuCl₂⁻, PO₄³⁻, HPO₄²⁻, H₂PO₄⁻, CO₃²⁻, HCO₃⁻ ,PF₆⁻, BF₄⁻, (F₃CSO₂)₂N⁻, p-CH₃C₆H₄SO₃⁻ , OCN⁻, NCO⁻ , SCN⁻, NCS⁻, B(CN)₄⁻, B(ORy)₄⁻, in which R_{y}, optionally selected independently of each other, are substituted and/or branched C₁ to C₁₈ alkyl residues, C₁ to C₁₈ alkenyl residues, C₁ to C₁₈ alkinyl residues, C₆ to C₁₂ aryl residues and/or C₇ to C₃₀ aralkyl residues which optionally contain one or more, preferably between 1 and 4, oxygen and/or nitrogen atoms as heteroatoms; borates with the basic structure B(O-A-O)₂⁻ in accordance with formulae a) to c); borates with the basic structure BX₂(OR)⁻ or BX₂(O-A-O)⁻ in accordance with formulae d) to i), 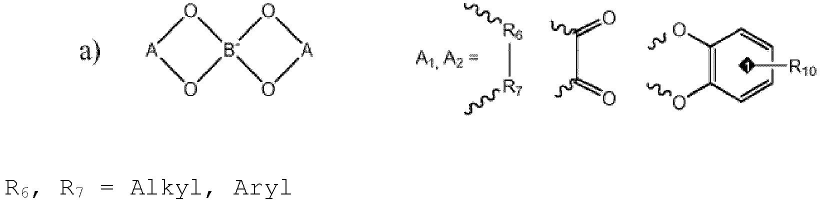 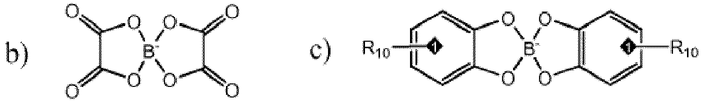 R₁₀ = Alkyl, Aryl, H, Halogen, -NO₂, -NH₂, -NHR₆, -N(R₆)₂, -COOR₆ or -OR₆ 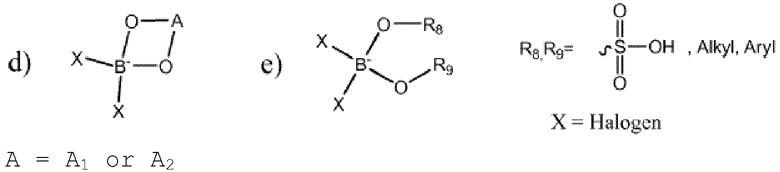 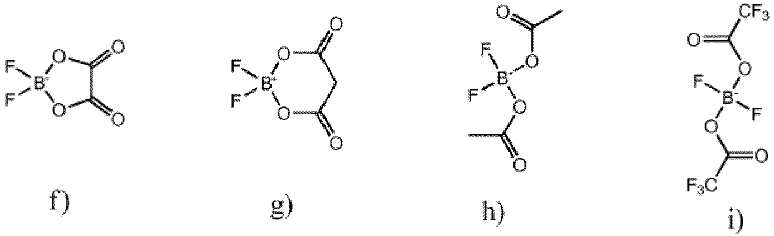 (N(CF₃)₂⁻, N(CN)₂ or N(SO₂C_{z}F_{2z+1})₂⁻ _{,} in which z is a natural number between 1 and 20.

2. Salts according to claim 1, **characterized in that** the anion X⁻ is selected from Br⁻, I⁻, PF₆⁻, BF₄⁻ and (F₃CSO₂)₂N⁻.

3. The salt according to claim 1 or claim 2, **characterized in that** Y₁ and Y₂ are CH, R_{1,} R₂, R₄, R₅ are H and R₃ is selected from -NO₂, -Cl, -Br, -COOEt, -CH₃, -OEt and -OMe.

4. The salt according to any one of claims 1 to 3, **characterized in that** Y₁ is N, Y₂ is CH and R₁, R₂, R₃, R₄, and R₅ are H.

5. The salt according to any one of claims 1 to 3, **characterized in that** Y₁ is CH, Y₂ is N and R₁, R₂, R₃, R₄, and R₅ are H.

6. The salt according to any one of claims 1 to 5, **characterized in that** Z is H or -CH₃.

7. The salt according to any one of claims 1 to 6, **characterized in that** Q is selected from -CH₃, -OH, - OCH₃, -SO₃H, -SO₃Rₓ, -COOH, -COOCH₃, -COCH₃, NH₂, - NHCH₃, N(CH₃)₂ and -CH(CH₃)₂.

8. Use of a salt according to any one of claims 1 to 7, which comprises a suitable anion, as an ionic liquid.

9. Use of a salt according to any one of claims 1 to 7 in a battery, a double layer capacitor or a fuel cell.

10. Use of a salt according to any one of claims 1 to 7, as a solvent or solvent additive.

11. Use of a salt according to any one of claims 1 to 7, for storing hydrogen.

12. A chemical reaction method involving a salt according to any one of claims 1 to 7, as a solvent or solvent additive.

13. A substance separation method involving a salt according to any one of claims 1 to 7, as a solvent or solvent additive.

## Revendications

1. Sels de la formule générale (I), 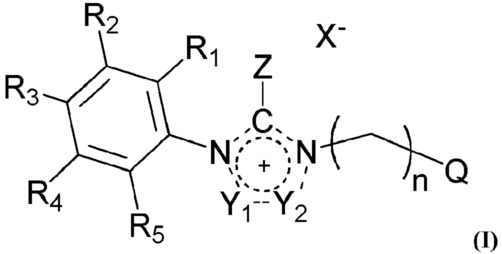 dans laquelle
X⁻ est un anion,
Y₁ et Y₂ sont CH ou Y₁ est CH et Y₂ est N ou Y₁ est N et Y₂ est CH,
n est un nombre compris entre 2 et 14 inclus,
Q est choisi parmi -CH₃, -OH, -ORₓ, -SO₃H, -SO3Rₓ, - COOH, -COORₓ, -CORₓ, NH₂, -NHRₓ, N(R_{X})₂ et -CH(Rₓ)2,
Z est H ou du Rx,
R₁, R₂, R₃, R₄ et R₅ indépendamment l' un de l' autre sont -H, - -Cl, -Br, -I, -NO₂, N(Rₓ₎₂, -Rₓ, -COORₓ ou - ORₓ,
Rₓ étant un radical alkyle en C₁ à C₁₈ substitué et/ou ramifié le cas échéant,
à l'exception des composés de la formule générale (I), dans lesquels Y₁ et Y₂ sont CH et R₁, R₂, R₃, R₄ et R₅ sont H,
à l'exception des composés de la formule générale (I), dans lesquels Y₁ et Y₂ sont CH et R₁ = R₃ = R₅ = CH₃, n = 2 ou 6 et Q = CH₃,
à l'exception des composés de la formule générale (I), dans lesquels Y₁ et Y₂ sont CH, R₁, R₂, R₄, R₅ = H, R₃ = ORₓ et Rₓ est un hydrocarbure avec 3 ou 12 atomes de carbone,
l'anion X⁻ étant choisi parmi F⁻, Cl⁻, Br⁻, I⁻ CH₃COO⁻, CF_{3C}OO⁻, CF₃SO₃, SO₄²⁻, HSO₄⁻, CH₃OSO₃⁻, C₂H₅OSO₃⁻, SO₃²⁻, HSO₃⁻, AlCl₄⁻, Al₂Cl₇⁻, Al₃Cl₁₀⁻, AlBr₄⁻, NO₂⁻, NO₃⁻, CuCl₂⁻, PO₄³⁻, HPO₄²⁻, H₂PO₄⁻, CO₃²⁻, HCO₃⁻, PF6⁻, BF₄⁻, (F₃CSO₂)₂N⁻ p-CH₃C₆H₄SO³⁻, OCN⁻, NCO⁻, SCN⁻, NCS⁻, (BCN)₄⁻, B(OR_{y})4⁻, R_{y} étant des radicaux alkyle en C₁ à C₁₈, de radicaux alkényle en C₁ à C₁₈, des radicaux alkinyle en C₁ à C₁₈, des radicaux aryle en C₆ à C₁₂ et/ou des radicaux aralkyle en C₇ à C₃₀, substitués et/ou ramifiés, choisis indépendamment les uns des autres, le cas échéant qui contiennent le cas échéant un ou plusieurs, de préférence entre 1 et 4 atomes d'oxygène et/ou d'azote en tant qu'hétéroatomes, des borates de la structure fondamentale B(O-A-O)2⁻, selon les formules a) à c), des borates de la structure fondamentale BX₂(OR) ⁻ ou BX₂(O-A-O)- selon les formules d) à i), 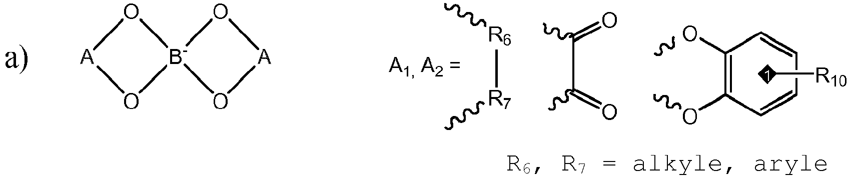 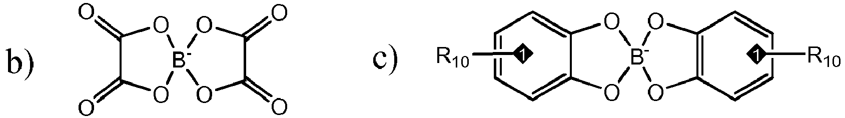 R₁₀ = alkyle, aryle, H, halogène, -NO₂, -NH₂, NHR₆, - N(R₆)₂, -COOR₆ ou -OR₆ 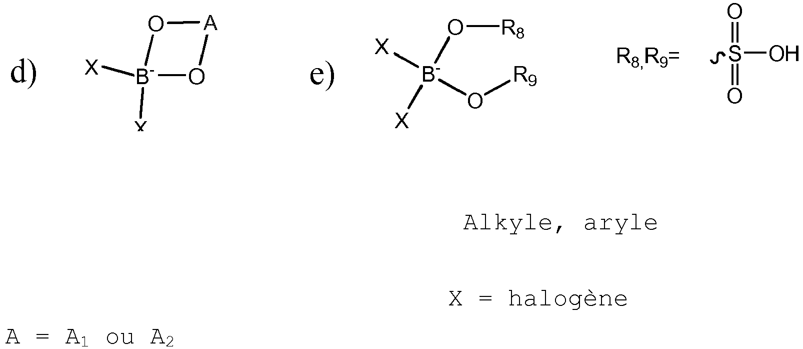 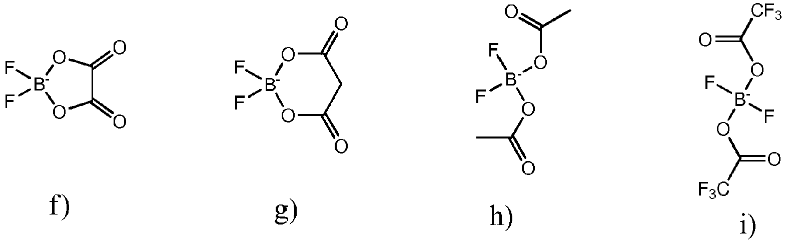 N(CF₃)2⁻, N(CN₎2⁻ ou N(SO₂C_{z}F_{2z+1})₂-, z étant un entier naturel compris entre 1 et 20.

2. Sels selon la revendication 1, **caractérisés en ce que** l'anion X⁻ est choisi parmi Br⁻, I⁻, PF₆⁻, BF₄⁻ et (F₃CSO₂)₂N⁻.

3. Sel selon la revendication 1 ou 2, **caractérisé en ce que** Y₁ et Y₂ sont CH et R₁, R₂, R₄ et R₅ sont H et R₃ est choisi parmi -NO₂, -Cl -Br, -COOEt, -CH₃, -OEt et-OMe.

4. Sel selon l'une quelconque des revendications 1 à 3, **caractérisé en ce que** Y₁ est N, Y₂ est CH et R₁, R₂, R₃, R₄ et R₅ sont H.

5. Sel selon l'une quelconque des revendications 1 à 3, **caractérisé en ce que** Y₁ est CH, Y₂ est N et R₁, R₂, R₃, R₄ et R₅ sont H.

6. Sel selon l'une quelconque des revendications 1 à 5, **caractérisé en ce que** Z est H ou CH₃.

7. Sel selon l'une quelconque des revendications 1 à 6, **caractérisé en ce que** Q est choisi parmi -CH₃, -OH, OCH₃, SO₃H, -SO₃Rₓ, -COOH, -COOCH₃, COCH₃, NH₂, -NHCH₃, N(CH₃)₂, et -CH(CH₃)₂.

8. Utilisation d'un sel selon l'une quelconque des revendications 1 à 7, lequel comporte un anion adapté, en tant que liquide ionique.

9. Utilisation d'un sel selon l'une quelconque des revendications 1 à 7 dans une batterie, un condensateur double-couche ou une cellule de combustible.

10. Utilisation d'un sel selon l'une quelconque des revendications 1 à 7 en tant que solvant ou qu'additif de solvant.

11. Utilisation d'un sel selon l'une quelconque des revendications 1 à 7, pour accumuler de l'hydrogène.

12. Procédé destiné à la transformation chimique, comprenant un sel selon l'une quelconque des revendications 1 à 7 en tant que solvant ou qu'additif de solvant.

13. Procédé destiné à la séparation de matières, comprenant un sel selon l'une quelconque des revendications 1 à 7 en tant que solvant ou qu'additif de solvant.
